(19) Europäisches Patentamt / European Patent Office / Office européen des brevets

(11) **EP 3 417 068 B1**

(12) **FASCICULE DE BREVET EUROPEEN**

(45) Date de publication et mention de la délivrance du brevet:
**18.03.2020 Bulletin 2020/12**

(21) Numéro de dépôt: **17708868.9**

(22) Date de dépôt: **17.02.2017**

(51) Int Cl.:
*C12Q 1/56* *(2006.01)*      *G01N 33/86* *(2006.01)*

(86) Numéro de dépôt international:
**PCT/FR2017/050354**

(87) Numéro de publication internationale:
**WO 2017/140989 (24.08.2017 Gazette 2017/34)**

(54) **MÉTHODE DE DOSAGE DES D-DIMÈRES SPÉCIFIQUES DE LA MALADIE THROMBOEMBOLIQUE VEINEUSE ET SON UTILISATION POUR LE DIAGNOSTIC DE L'EMBOLIE PULMONAIRE ET DE LA THROMBOSE VEINEUSE PROFONDE**

VERFAHREN ZUM TESTEN VON SPEZIFISCHEN D-DIMEREN FÜR VENÖSE THROMBOEMBOLIE UND VERWENDUNG ZUR DIAGNOSE VON LUNGENEMBOLIE UND TIEFER VENENTHROMBOSE

METHOD FOR ASSAYING D-DIMERS SPECIFIC TO VENOUS THROMBOEMBOLISM AND USE THEREOF FOR DIAGNOSING PULMONARY EMBOLISM AND DEEP VENOUS THROMBOSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.02.2016 FR 1651348**

(43) Date de publication de la demande:
**26.12.2018 Bulletin 2018/52**

(73) Titulaire: **Diagnostica Stago**
**92600 Asnières-sur- Seine (FR)**

(72) Inventeurs:
• **CONTANT, Geneviève**
**92400 Courbevoie (FR)**
• **BEAUPERE, Françoise**
**95600 Eaubonne (FR)**

(74) Mandataire: **Plasseraud IP**
**66, rue de la Chaussée d'Antin**
**75440 Paris Cedex 09 (FR)**

(56) Documents cités:
WO-A1-2016/012729      WO-A2-2011/022054
US-A1- 2004 029 286      US-A1- 2012 270 243

• Swaroopa Pulivarthi: "Effectiveness of D-Dimer as a Screening Test for Venous Thromboembolism: An Update", , 31 octobre 2014 (2014-10-31), XP055282367, Extrait de l'Internet: URL:http://www.ncbi.nlm.nih.gov/pmc/articl es/PMC4215485/ [extrait le 2016-06-21]
• M. RIGHINI ET AL: "D-Dimer for venous thromboembolism diagnosis: 20 years later", JOURNAL OF THROMBOSIS AND HAEMOSTASIS, vol. 6, no. 7, 1 juillet 2008 (2008-07-01) , pages 1059-1071, XP055093339, ISSN: 1538-7933, DOI: 10.1111/j.1538-7836.2008.02981.x
• RODGER MARC A ET AL: "Clinical Decision Rules and D-dimer in Venous Thromboembolism: Current controversies and future research priorities", THROMBOSIS RESEARCH, TARRYTOWN, NY, US, vol. 134, no. 4, 4 août 2014 (2014-08-04), pages 763-768, XP029060134, ISSN: 0049-3848, DOI: 10.1016/J.THROMRES.2014.07.031
• HALABY RIM ET AL: "d-Dimer elevation and adverse outcomes", JOURNAL OF THROMBOSIS AND THROMBOLYSIS, KLUWER ACADEMIC PUBLISHERS, DORDRECHT, NL, vol. 39, no. 1, 9 juillet 2014 (2014-07-09), pages 55-59, XP035431589, ISSN: 0929-5305, DOI: 10.1007/S11239-014-1101-6 [extrait le 2014-07-09]

## Description

### Domaine de l'Invention

La présente invention concerne une méthode de dosage des D-dimères spécifiques de la maladie thromboembolique veineuse dans un échantillon sanguin, comprenant le dosage habituel des D-dimères de l'échantillon d'une part, ainsi que la mesure dynamique de la formation de fibrine de cet échantillon d'autre part. La méthode est destinée notamment au diagnostic *in vitro* de l'embolie pulmonaire et de la thrombose veineuse profonde dans un échantillon sanguin.

### Contexte de l'Invention

La maladie thromboembolique veineuse (MTEV) est un problème majeur de santé publique. Elle regroupe la notion de thrombose veineuse profonde (TVP) et son risque vital immédiat, l'embolie pulmonaire (EP). Les principes de base de la pathogénèse de la MTEV ont été décrits par Virchow qui a défini l'origine de la thrombose dans l'association de trois facteurs favorisant la thrombose, qui sont la stase veineuse; le dysfonctionnement ou l'altération de l'endothélium; et l'hypercoagulabilité due à l'activation des facteurs de la coagulation, à l'hyperviscosité, à une déficience en antithrombine, à une thrombophilie, à un syndrome néphrotique, à un changement après de sévères traumatismes physiques ou brûlures, un cancer disséminé, une grossesse tardive, l'ethnie, l'âge, le tabac ou l'obésité (Lopez, Thromb. Res., 2009, 123(Suppl. 4): S30-34).

L'embolie pulmonaire (EP) est une maladie redoutée par tous les urgentistes parce que son diagnostic est difficile, peut-être un des plus difficiles, tant elle se manifeste avec des symptômes très différents, voire est totalement silencieuse. L'embolie pulmonaire est une affection si fréquente que toute anomalie respiratoire survenant de façon brutale doit suggérer ce diagnostic. Elle provoque une douleur thoracique aiguë et angoissante. Elle doit être différenciée de l'infarctus du myocarde par l'électrocardiogramme, le dosage des enzymes cardiaques (transaminases, troponine...) et la scintigraphie pulmonaire. Elle doit être différenciée de la péricardite aiguë et de la dissection aortique, qui contre-indiquent l'utilisation d'anticoagulants.

Plusieurs types d'EP existent:

- l'embolie pulmonaire avec cœur pulmonaire aigu peut être mortelle et apparaît surtout chez un patient alité dans les suites d'une intervention chirurgicale. L'aggravation est possible au cours des premières heures imposant le recours à un acte chirurgical ou embolectomie ou au traitement thrombolytique. L'évolution fatale reste très fréquente.
- les embolies pulmonaires foudroyantes entraînent une mort subite ou une mort en quelques minutes par collapsus;
- les embolies pulmonaires frustes sont très fréquentes, avec des symptômes souvent trompeurs. Le diagnostic est souvent porté quelques jours plus tard; et
- les embolies pulmonaires chez les patients souffrant d'insuffisance cardiaque et respiratoire sont fréquentes et leur gravité impose un traitement anticoagulant préventif.

Les signes cliniques de la thrombose veineuse profonde (TVP) sont souvent frustes voire inexistants, ou similaires à ceux d'une thrombose veineuse superficielle, beaucoup moins grave car sans évolution vers l'EP. Il est nécessaire de différencier la TVP :

- d'un hématome profond, car le traitement anticoagulant va favoriser le saignement,
- d'une maladie infectieuse sous-cutanée de la jambe,
- d'une maladie post-phlébitique, et
- de la rupture d'un kyste poplité qui donne un tableau clinique assez similaire.

Il existe trois types de complications, dont: l'EP, qui, dans près de la moitié des cas, est totalement asymptomatique; l'extension du thrombus dans le système veineux; et la maladie post-phlébitique, complication fréquente, chez près d'un tiers des patients.

Des scores permettent d'évaluer la probabilité clinique d'une EP en présence d'une dyspnée ou d'une douleur thoracique. Ils sont utilisés dans l'arbre de décision diagnostic pour optimiser la prise en charge des patients, en particulier pour décider de l'approche thérapeutique. L'algorithme diagnostic actuel repose sur une démarche qui comporte :

- une première étape d'évaluation de la probabilité clinique: score de Wells dans la TVP (Wells, J. Thromb. Haemost., 2007, 5(Suppl. 1): 41-50), score de Wells ou score de Genève et leurs versions simplifiées dans l'EP (Ceriani, J. Thromb. Haemost., 2010, 8(5): 957-970; Klok, Arch. Intern. Med., 2008, 168(19): 2131-2136; Le Gal, Ann. Intern. Med., 2006, 144(3): 165-171), regroupant plusieurs facteurs de risque, des symptômes et des signes cliniques,

- suivie dans la majorité des cas par un dosage de D-dimères, examen très sensible mais peu spécifique, qui, quand il est positif, impose la réalisation d'un angioscanner thoracique spiralé avec injection de produit de contraste iodé ou d'une scintigraphie pulmonaire dans l'EP, d'un doppler dans la TVP. Une faible probabilité clinique, combinée à un taux de D-dimères inférieur au seuil de 0,5 μg/mL, permet d'exclure la présence d'une EP ou d'une TVP avec une excellente valeur prédictive négative.

L'utilisation du dosage des D-dimères comme test d'exclusion entraîne la nécessité d'avoir recours aux tests d'imagerie coûteux, pour visualiser le thrombus et faire le diagnostic de gravité avec le nombre et le type d'artères pulmonaires atteintes en cas d'EP. En outre, de tels examens prolongent le séjour des malades aux urgences, sont sources d'irradiation et à l'origine d'un coût non négligeable. L'angioscanner peut être à l'origine de réactions allergiques au produit de contraste iodé, d'insuffisance rénale et d'œdème pulmonaire.

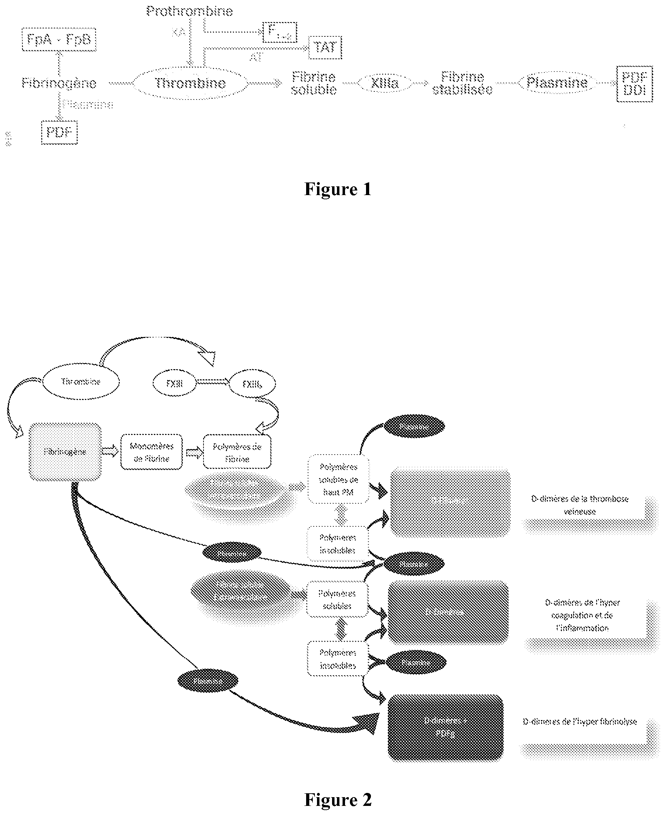

L'antigène D-dimère est un marqueur unique de la dégradation de la fibrine formée par l'action séquentielle de la thrombine, du facteur de coagulation FXIIIa et de la plasmine, comme le montre la Figure 1. En effet, la thrombine clive le fibrinogène en monomères de fibrine, qui polymérisent et servent de substrat au FXIIIa et à la formation de plasmine. Puis, la thrombine reste associée à la fibrine et active le FXIII lié aux polymères de fibrine pour produire le FXIIIa, qui catalyse la formation de liens covalents entre les domaines D de la fibrine polymérisée. Enfin, la plasmine dégrade la fibrine polymérisée pour libérer les produits de dégradation de la fibrine (PDFn) de différents poids moléculaires, incluant les produits terminaux contenant le complexe D-dimère-fragment E et expose l'antigène D-dimère, qui reste indétectable jusqu'à sa libération par la plasmine. L'antigène D-dimère peut être présent soit sur les PDFn provenant de la fibrine avant son incorporation dans le caillot de fibrine, soit des complexes de haut poids moléculaire libérés après dégradation du caillot par la plasmine (Adam, Blood, 2009, 113(13): 2878-2887).

Le D-dimère est un marqueur de la formation du caillot de fibrine et de la fibrinolyse. La fibrinolyse joue un rôle important sur la stabilité du caillot et le risque thrombotique si elle commence après la formation du caillot. Si elle est initiée en même temps, il y a compétition entre la liaison thrombine-fibrine et la liaison plasmine-fibrine, donc entre formation et lyse de la fibrine.

Les D-dimères proviennent de la dégradation des polymères de fibrine soluble, qui sont des marqueurs d'activation de la coagulation intravasculaire (Mirshahi. PLoS ONE, 2014, 9(3): e92379, doi:10.1371/journal.pone.0092379). Dans les états d'hyper coagulation et dans les états inflammatoires aigus, les D-dimères proviennent également de la dégradation de la fibrine extravasculaire par les enzymes présentes localement au niveau des sites d'inflammation, comme le montre la Figure 2. Cette notion est supportée par les taux souvent élevés de D-dimères chez les patients atteints de cancer ou d'états inflammatoires aigus (Dirix, Br. J. Cancer, 2002, 86(3): 389-395).

Lorsque la fibrinolyse s'étend et se généralise ("hyper fibrinolyse"), la dégradation du fibrinogène par la plasmine générée en large excès devient alors possible. Il apparaît dans la circulation, à la fois, des produits de dégradation de la fibrine dont les D-dimères et des produits de dégradation du fibrinogène (PDFg), comme le montre la Figure 2. Ainsi, des taux élevés de D-dimères sont générés dans le cancer et dans les états d'activation importante de la coagulation, en association avec des taux importants de PDFg.

Les dosages de D-dimères utilisés actuellement ne sont pas identiques, car l'antigène D-dimère est présent sur les PDFn de différente taille et les anticorps monoclonaux reconnaissent différents épitopes (Dempfle, FACT study group, Thromb. Haemost., 2001, 85(4): 671-678). Les méthodes les plus fréquemment utilisées sont les méthodes immuno-turbidimétriques automatisées, basées sur le changement de turbidité d'une suspension de microparticules de latex coatées par des anticorps monoclonaux anti-antigène D-dimère. En présence de D-dimères dans l'échantillon, les microparticules de latex agglutinent, induisant une augmentation de la turbidité du milieu, qui se traduit par une augmentation de l'absorbance en photométrie à 540 nm. Ces dosages en plasma présentent une meilleure sensibilité que les tests d'agglutination en sang total (de Groot, Thromb. Haemost., 1999, 82(6): 1588-1592), permettant ainsi l'exclusion de la MTEV, mais ils sont très peu spécifiques, et ne peuvent être utilisés pour le diagnostic de la MTEV. Les documents US 2012/27, WO 2011/022054 et US 2004/029286 révèlent tous une méthode de diagnostic de la MTVE reposant sur le dosage des D-dimères.

Cependant, aucune méthode de l'art antérieur n'a relié la mesure dynamique de la formation de fibrine d'un échantillon, aux D-dimères et aux produits de dégradation du fibrinogène (PDFg) générés par l'hyper coagulation, l'inflammation et l'hyper fibrinolyse, pour déterminer les D-dimères résultant de la lyse de la fibrine intravasculaire. Ainsi, aucune méthode actuelle n'est capable de mesurer les D-dimères spécifiques de la thrombose veineuse dans un échantillon sanguin.

La mise à disposition d'une méthode de diagnostic in vitro, aussi sensible mais plus spécifique que le dosage habituel des D-dimères, pourrait ainsi diminuer le recours aux examens d'imagerie chez les malades avec suspicion d'EP et de TVP, tels qu'un angioscanner thoracique spiralé avec injection de produit de contraste iodé ou une scintigraphie pulmonaire. Une telle méthode permettrait de limiter les coûts, d'assurer une prise en charge plus rapide des patients, en particulier les personnes âgées et les patients atteints de cancer, à haut risque de thrombose, et permettre une décision rapide de traitement.

Il existe donc un besoin de disposer d'une méthode de détermination des D-dimères spécifiques de la thrombose veineuse dans un échantillon de patient, ladite méthode étant capable de différencier les D-dimères dudit patient générés par l'hyper coagulation, l'inflammation et l'hyper fibrinolyse, de ceux résultant de la lyse de la fibrine intravasculaire et spécifiques de la thrombose, comme cela est montré en Figure 2. Une telle méthode doit, en outre, être automatisée pour être réalisée en urgence au laboratoire ou réalisable au lit du patient. De plus, elle doit être fiable, reproductible, simple et aider à la décision de traitement.

**Résumé de l'Invention**

De façon surprenante, les présents Inventeurs ont mis au point une méthode capable de distinguer les D-dimères provenant de l'activation de la coagulation, de l'inflammation et de l'excès de fibrinolyse, de ceux spécifiques de la thrombose, provenant de la dégradation de la fibrine intravasculaire, et de les mesurer quantitativement. Cette méthode permet en outre leur détermination en situation d'urgence sur un automate de laboratoire, un dispositif de biologie délocalisée ou un dispositif portable. La méthode développée est fiable, simple, rapide à mettre en œuvre, et reproductible. De plus, les Inventeurs ont affiné cette méthode de sorte que, dans sa version modifiée, la méthode est applicable à tous les patients quel que soit leur âge, et réduit le nombre de faux négatifs, permettant ainsi de détecter une thrombose veineuse en cas de thrombophilie sous-jacente, en cas d'embolie pulmonaire sous-segmentaire ou sans gravité, en cas d'infarctus pulmonaire, en cas de cancer, en cas d'infection et en cas de grossesse ainsi que pour des sujets âgés avec un terrain inflammatoire sous-jacent. Enfin, contrairement aux méthodes de dosage courantes, la méthode selon l'invention ne nécessite aucun ajustement en fonction de l'âge.

La méthode selon l'invention est basée sur trois constats: l'hyper coagulation et l'hyper fibrinolyse sont absentes ou réduites dans la thrombose, contrairement aux états d'activation de la coagulation; en revanche, l'état inflammatoire est de première importance.

Plus spécifiquement, dans un premier aspect, la présente invention concerne une première version d'une méthode de dosage des D-dimères spécifiques de la maladie thromboembolique veineuse dans un échantillon sanguin de patient, ladite méthode comprenant d'une part le dosage des D-dimères dans l'échantillon pour obtenir le taux de D-dimères dans l'échantillon ($Ddi_E$), et d'autre part, la mesure dynamique de la formation de fibrine de ce même échantillon, ladite mesure dynamique comprenant les étapes suivantes:

a) initier l'activation de la coagulation dans l'échantillon sans la déclencher;

b) incuber le mélange obtenu à l'étape a), et déclencher, dans l'échantillon incubé, la génération de thrombine et la formation d'un caillot de fibrine;

c) mesurer la variation temporelle d'au moins une propriété de l'échantillon obtenu en b), dans lequel se forme le caillot de fibrine;

d) établir le profil de formation du caillot de fibrine analysé en c), et extraire de ce profil le temps de formation de la fibrine (TFF) mesuré au point d'inflexion de la tangente de la courbe du profil, la valeur de la propriété ($Vp_{(TA)}$) de l'échantillon mesurée au temps d'atteinte (TA) du plateau de polymérisation de la fibrine;

e) calculer le taux des D-dimères résultant de la dégradation de la fibrine intravasculaire (R):

e1) en ajustant le taux de D-dimères de l'échantillon ($Ddi_E$) en fonction du taux des D-dimères générés par l'hyper coagulation en utilisant TFF déterminé en d), pour obtenir le taux de D-dimères ajustés en fonction de l'hyper coagulation ($Ddi_{E/HC}$), et

e2) en ajustant le taux de D-dimères ajustés $Ddi_{E/HC}$ obtenu en e1) en fonction du taux des D-dimères générés par l'inflammation en utilisant $Vp_{(TA)}$ déterminé en d), pour obtenir R;

f) comparer le taux de D-dimères résultant de la dégradation de la fibrine R obtenu en e), par rapport à un seuil, de préférence au seuil de 0,5 $\mu$g/mL;

g) déterminer le taux des produits de dégradation du fibrinogène générés par l'hyper fibrinolyse ($PDFg_{(HF)}$) présente dans les états d'activation de la coagulation en utilisant le taux de D-dimères résultant de la dégradation de la fibrine, R, obtenu en e) et le taux de D-dimères de l'échantillon ($Ddi_E$).

h) comparer le taux $PDFg_{(HF)}$ obtenu en g) par rapport à un seuil, et comparer le temps de formation de la fibrine (TFF) obtenu en d) par rapport à un seuil.

Dans certains modes de réalisation, la première version de la méthode selon l'invention est caractérisée en ce que dans l'étape e), calculer le taux R exprimé en unités équivalents au fibrinogène initial (FEU) est réalisé:

e1) en calculant le taux de D-dimères ajustés en fonction de l'hyper coagulation ($Ddi_{E/HC}$) en utilisant l'équation suivante:

$$\mathrm{Ddi_{E/HC}} = \mathrm{Ddi_E} \ \mathrm{x} \ \frac{\mathrm{TFF}}{\mathrm{Temps\ Témoin}}$$

où le Temps Témoin est le temps moyen de formation du caillot de fibrine d'échantillons de patients sains ne présentant pas de suspicion de thrombose, mesuré selon les étapes a)-d), et

e2) en calculant le taux R en utilisant l'équation suivante:

$$\mathrm{R} = \frac{\mathrm{Ddi_{E/HC}}}{\mathrm{[Fib]_{(Vp(TA))}}}$$

où $\mathrm{[Fib]_{(Vp(TA))}}$ est la concentration en fibrinogène déduite pour la valeur de la propriété $\mathrm{Vp_{(TA)}}$ sur une courbe étalon d'équation:

$$\mathrm{y} = \mathrm{a\ ln(x)} - \mathrm{b},$$

où:

y est la valeur de la propriété mesurée au temps d'atteinte (TA) du plateau de polymérisation de la fibrine,
x est la concentration en fibrinogène,
a et b sont les constantes de l'équation logarithmique qui relie le niveau du plateau de fibrine, et la concentration en fibrinogène,
la courbe étalon ayant été établie pour des échantillons sanguins dont la concentration en fibrinogène a été déterminée et dont la valeur de la propriété $\mathrm{(Vp_{(TA))}}$ a été déterminée par les étapes a)-d).

Dans certains modes de réalisation, la première version de la méthode selon l'invention est caractérisée en ce que dans l'étape f), un taux R obtenu en e), inférieur au seuil, préférablement au seuil de 0,5 $\mu$g/mL, permet d'exclure une thrombose chez le patient, et où un taux R supérieur au seuil préférablement au seuil de 0,5 $\mu$g/mL, est indicatif de la possibilité d'une thrombose chez le patient.

Dans certains modes de réalisation, la première version de la méthode selon l'invention est caractérisée en ce que l'étape g) comprend des étapes consistant à:

g1) déterminer le taux de PDFg correspondant au taux de D-dimères dans l'échantillon $\mathrm{(Ddi_E)}$ sur une courbe étalon établie en utilisant des échantillons sanguins avec des taux de PDFg et des taux de D-dimères connus,
g2) déterminer le taux de PDFg correspondant au taux de D-dimères ajusté (R) obtenu à l'étape e) sur la courbe étalon utilisée en g1), et
g3) déterminer le taux de PDFg générés par l'hyper fibrinolyse $\mathrm{(PDFg_{(HF))}}$ en soustrayant le taux de PDFg obtenu en g2) du taux de PDFg obtenu en g1).

Dans certains modes de réalisation, la première version de la méthode selon l'invention est caractérisée en ce que l'étape h) comprend des étapes consistant à:

h1) comparer $\mathrm{PDFg_{(HF)}}$ à un seuil, notamment un seuil de 1 $\mu$g/mL, où $\mathrm{PDFg_{(HF)}}$ inférieur au seuil ou négatif permet d'exclure la thrombose chez le patient, et $\mathrm{PDFg_{(HF)}}$ supérieur au seuil est indicatif d'une possibilité de thrombose chez le patient,
h2) comparer TFF à un seuil, notamment à un seuil égal au [Temps Témoin de e1) - 1 écart type], par exemple un seuil de 120 secondes pour un Temps Témoin de 135 secondes, où TFF inférieur au seuil est indicatif d'un patient sans thrombose mais présentant un état d'activation aigu de la coagulation, et un TFF supérieur au seuil est indicatif d'une thrombose chez le patient.

Dans certains modes de réalisation, la première version de la méthode selon l'invention est caractérisée en ce que:

- lorsque qu'une thrombose a été diagnostiquée à l'étape h2), le taux de D-dimères spécifiques de la maladie thromboembolique veineuse $\mathrm{(Ddi_{MTEV})}$ est le taux de D-dimères, R, obtenu à l'étape e),

- lorsqu'une thrombose a été exclue aux étapes h1) et h2) mais que le taux R obtenu à l'étape e) est supérieur à un seuil, préférablement au seuil de 0,5 µg/mL, la méthode comprend en outre une étape consistant à calculer le taux de D-dimères spécifiques de la maladie thromboembolique veineuse (Ddi$_{MTEV}$) en utilisant l'équation suivante:

$$Ddi_{MTEV} = 0,5 \; x \; \frac{R}{Ddi_E}$$

où DdiE est le taux de D-dimères dans l'échantillon.

Le taux Ddi$_{MTEV}$ est représentatif de l'étendue de l'embolie pulmonaire ou de la thrombose veineuse profonde, et donc de la gravité de la maladie.

La présente invention concerne également une deuxième version d'une méthode de dosage des D-dimères spécifiques de la maladie thromboembolique veineuse dans un échantillon sanguin de patient, ladite méthode comprenant d'une part le dosage des D-dimères dans l'échantillon pour obtenir le taux de D-dimères dans l'échantillon (Ddi$_E$), et d'autre part, la mesure dynamique de la formation de fibrine de ce même échantillon, ladite mesure dynamique comprenant les étapes suivantes:

a) initier l'activation de la coagulation dans l'échantillon sans la déclencher;
b) incuber le mélange obtenu à l'étape a), et déclencher, dans l'échantillon incubé, la génération de thrombine et la formation d'un caillot de fibrine;
c) mesurer la variation temporelle d'au moins une propriété de l'échantillon obtenu en b), dans lequel se forme le caillot de fibrine;
d) établir le profil de formation du caillot de fibrine analysé en c), et extraire de ce profil le temps de formation de la fibrine (TFF) mesuré au point d'inflexion de la tangente de la courbe du profil, et la valeur de la propriété (Vp$_{(TA)}$) de l'échantillon mesurée au temps d'atteinte (TA) du plateau de polymérisation de la fibrine;
e') calculer le taux des D-dimères résultant de la dégradation de la fibrine intravasculaire (R):

e'1) en ajustant le taux de D-dimères de l'échantillon, Ddi$_E$, en fonction du taux des D-dimères générés par l'inflammation en utilisant Vp$_{(TA)}$ déterminé en d), pour obtenir le taux de D-dimères ajustés pour l'inflammation (Ddi$_{E/I}$); et
e'2) en corrigeant le taux de D-dimères ajustés pour l'inflammation (Ddi$_{E/I}$) obtenu en e'1) pour les taux de D-dimères faibles, et

classer l'échantillon du patient en fonction de l'inflammation;
f') comparer le taux de D-dimères résultant de la dégradation de la fibrine R obtenu en e), par rapport à un seuil, de préférence au seuil de 0,5 µg/mL;
g') déterminer le taux des D-dimères générés par l'hyper fibrinolyse (Ddi$_{(HF)}$) en utilisant le taux de D-dimères dans l'échantillon (Ddi$_E$) et le taux R obtenu en e') ou en utilisant le taux de D-dimères ajustés en fonction de l'inflammation, Ddi$_{E/I}$, obtenu en e'1) et le taux R obtenu en e');
h') comparer le taux Ddi$_{(HF)}$ obtenu en g') par rapport à un seuil, et comparer le ratio TA/TTF par rapport à un seuil, où TA est le temps d'atteinte du plateau de polymérisation de fibrine obtenu en d), et TFF est le temps de formation de la fibrine obtenu en d).

Dans certains modes de réalisation, la deuxième version de la méthode selon l'invention est caractérisée en ce que dans l'étape e'), le taux R exprimé en unités équivalents au fibrinogène initial (FEU) est obtenu:

e'1) en calculant le taux de D-dimères ajustés en fonction de l'inflammation (Ddi$_{E/I}$) par l'équation suivante:

$$Ddi_{E/I} = \frac{Ddi_E}{[Fib]_{(Vp(TA))}}$$

où [Fib]$_{(Vp(TA))}$ est la concentration en fibrinogène déduite pour la valeur de la propriété Vp$_{(TA)}$ sur une courbe étalon d'équation:

$$y = a \ln(x) - b,$$

où:

y est la valeur de la propriété mesurée au temps d'atteinte (TA) du plateau de polymérisation de la fibrine,
x est la concentration en fibrinogène,
a et b sont les constantes de l'équation logarithmique qui relie le niveau du plateau de fibrine, et la concentration en fibrinogène,
la courbe étalon ayant été établie pour des échantillons sanguins dont la concentration en fibrinogène a été déterminée et dont la valeur de la propriété $Vp_{(TA)}$ a été déterminée par les étapes a)-d).

e'2) en corrigeant le taux $Ddi_{E/I}$ obtenu en e'1) par l'équation suivante:

$$R = Ddi_{E/I} + [0,5 - F_{Ddi-E}]$$

où $F_{Ddi-E}$ est un facteur de correction des taux de D-dimères faibles (< 4 μg/mL) dont la valeur correspond à la valeur du facteur de correction pour le taux de D-dimères de l'échantillon ($Ddi_E$) sur la courbe étalon d'équation:

$$y = a\,x^2 + b\,x + c$$

où y est le facteur de correction, F,
x est le taux de D-dimères,
a, b et c sont les constantes de l'équation polynomiale qui relie le facteur de correction, et le taux de D-dimères,
la courbe étalon ayant été établie pour des échantillons sanguins dont le taux de D-dimères a été déterminée et pour lesquels le facteur de correction a été déterminé de façon empirique pour que le taux $Ddi_{E/I}$ soit ramené au seuil de 0,5 μg/mL unités FEU (unités équivalentes au fibrinogène).

Dans certains modes de réalisation, la deuxième version de la méthode selon l'invention est caractérisée en ce que dans l'étape e'), classer l'échantillon du patient en fonction de l'inflammation comprend:

- calculer le ratio $1/[Fib]_{(Vp(TA))}$, où $Fib]_{(Vp(TA))}$ est la concentration en fibrinogène déterminée en e1'); et
- classer l'échantillon du patient dans le groupe I des patients sans inflammation si le ratio $1/[Fib]_{(Vp(TA))} > 0,20$, ou
- classer l'échantillon du patient dans le groupe II des patients avec inflammation si le ratio $1/[Fib]_{(Vp(TA))} \leq 0,20$.

Dans certains modes de réalisation, la deuxième version de la méthode selon l'invention est caractérisée en ce que dans l'étape f'), un taux R obtenu en e'), inférieur au seuil, préférablement au seuil de 0,5 μg/ml, permet d'exclure une thrombose chez le patient.

Dans certains modes de réalisation, la deuxième version de la méthode selon l'invention est caractérisée en ce que dans l'étape g'), le taux des D-dimères générés par l'hyper fibrinolyse ($Ddi_{HF}$) est calculé:

g'1) comme le ratio entre le taux R déterminé à l'étape e') et le taux de D-dimères de l'échantillon, $Ddi_E$, en utilisant l'équation:

$$Ddi_{HF} = (R/Ddi_E)_{patient} = \frac{R}{Ddi_E}$$

g'2) comme le ratio entre le taux R déterminé à l'étape e') et le taux $Ddi_{E/I}$, obtenu en e'1), en utilisant l'équation:

$$Ddi_{HF} = (R/Ddi_{E/I})_{patient} = \frac{R}{Ddi_{E/I}}$$

Dans certains modes de réalisation, la deuxième version de la méthode selon l'invention est caractérisée en ce que dans l'étape h'):

- le taux $(R/Ddi_E)_{patient}$ obtenu en g'1) est comparé à un seuil:

h'1) en déterminant pour le taux $Ddi_E$ de l'échantillon, la valeur du ratio $(R/Ddi_E)_{étalon}$ sur une courbe étalon d'équation:

$$y = a\ x^{-b}$$

où x est le taux de D-dimères,
y est le ratio entre le taux de D-dimères qui résultent de la dégradation de la fibrine intravasculaire et le taux de D-dimères, $(R/Ddi_E)$,
a, et b sont les constantes de l'équation qui relie le ratio $R/Ddi_E$, et le taux de D-dimères,
la courbe étalon ayant été établie en utilisant:

- en utilisant, si l'échantillon du patient a été classé dans le groupe I: des échantillons sanguins classés dans le groupe I par l'étape e') et dont le taux de D-dimères est connu ou a été déterminé et le taux R a été obtenu par les étapes a)-e'2); et
- en utilisant, si l'échantillon du patient a été classé dans le groupe II: des échantillons sanguins classés dans le groupe II par l'étape e') et dont le taux de D-dimères est connu ou a été déterminé et le taux R a été obtenu par les étapes a)-e'2); et

h"1) en comparant la valeur du taux de D-dimères générés par l'hyper fibrinolyse $(R/Ddi_E)_{patient}$ obtenu en g'1) avec la valeur du ratio $(R/Ddi_E)_{étalon}$ obtenu en h'1), où:
$(R/Ddi_E)_{patient}$ inférieur au ratio $(R/Ddi_E)_{étalon}$ permet d'exclure la thrombose chez le patient, et $(R/Ddi_E)_{patient}$ supérieur ou égal au ratio $(R/Ddi_E)_{étalon}$ est indicatif d'une possibilité de thrombose chez le patient;

- le taux $(R/Ddi_{E/I})_{patient}$ obtenu en g'2) est comparé à un seuil:

h'2) en déterminant pour le taux $Ddi_E$ de l'échantillon, la valeur du ratio $(R/Ddi_{E/I})_{étalon}$ sur une courbe étalon d'équation:

$$y = a\ x^{-b}$$

où x est le taux de D-dimères,
y est le ratio entre le taux de D-dimères qui résultent de la dégradation de la fibrine intravasculaire et le taux de D-dimères ajustés en fonction de l'inflammation, $(R/Ddi_{E/I})$,
a, et b sont les constantes de l'équation qui relie le ratio $R/Ddi_{E/I}$, et le taux de D-dimères,
la courbe étalon ayant été établie:

- en utilisant, si l'échantillon du patient a été classé dans le groupe I: des échantillons sanguins classés dans le groupe I par l'étape e') et dont le taux de D-dimères est connu ou a été déterminé, le taux $Ddi_{E/I}$ a été obtenu par les étapes a)-e' 1), et le taux R a été obtenu par les étapes a)-e'2); et
- en utilisant, si l'échantillon du patient a été classé dans le groupe II: des échantillons sanguins classés dans le groupe II par l'étape e') et dont le taux de D-dimères est connu ou a été déterminé, le taux $Ddi_{E/I}$ a été obtenu par les étapes a)-e' 1), et le taux R a été obtenu par les étapes a)-e'2); et

h"2) en comparant la valeur du taux de D-dimères générés par l'hyper fibrinolyse $(R/Ddi_{E/I})_{patient}$ obtenu en g'2) avec la valeur du ratio $(R/Ddi_{E/I})_{étalon}$ obtenu en h'2), où:
$(R/Ddi_{E/I})_{patient}$ inférieur au ratio $(R/Ddi_{E/I})_{étalon}$ permet d'exclure la thrombose chez le patient, et où $(R/Ddi_{E/I})_{patient}$ supérieur ou égal au ratio $(R/Ddi_{E/I})_{étalon}$ est indicatif d'une possibilité de thrombose chez le patient.

Dans certains modes de réalisation, la deuxième version de la méthode selon l'invention est caractérisée en ce que dans l'étape h'), comparer le ratio TA/TTF par rapport à un seuil, comprend:

h'3) calculer le ratio TA/TFF où TA est le temps d'atteinte du plateau de polymérisation de la fibrine déterminé à l'étape d) et TFF est le temps de formation du caillot de fibrine déterminé à l'étape d); et

h"3) si l'échantillon du patient a été classé dans le groupe I: comparer le ratio TA/TFF par rapport à un premier seuil, notamment à un premier seuil de 1,75 pour un Temps Témoin de 135 secondes, où:

un ratio TA/TFF supérieur au premier seuil permet d'exclure la thrombose et de diagnostiquer une thrombophilie ou un état d'activation de la coagulation chez le patient classé dans le groupe I, et
un ratio TA/TFF inférieur ou égal au premier seuil est indicatif d'une thrombose chez le patient classé dans le groupe I;

si l'échantillon du patient a été classé dans le groupe II: comparer le ratio TA/TFF par rapport à un deuxième seuil, notamment à un deuxième seuil de 1,65 pour un Temps Témoin de 135 secondes, où:

un ratio TA/TFF supérieur au deuxième seuil permet d'exclure la thrombose et de diagnostiquer une thrombophilie ou d'un état d'activation de la coagulation chez le patient classé dans le groupe II, et
un ratio TA/TFF inférieur ou égal au deuxième seuil est indicatif d'une thrombose chez le patient classé dans le groupe II,

où le Temps Témoin est le temps moyen de formation du caillot de fibrine d'échantillons de sujets sains ne présentant pas de suspicion de thrombose, mesuré selon les étapes a)-d).

Dans certains modes de réalisation, la deuxième version de la méthode selon l'invention est caractérisée en ce que:

- lorsqu'une thrombose a été diagnostiquée à l'étape h"3), le taux de D-dimères spécifiques de la maladie thromboembolique veineuse ($Ddi_{MTEV}$) est le taux de D-dimères, R, obtenu à l'étape e'),
- lorsqu'une thrombose a été exclue aux étapes h"1), h"2) et h"3) mais que le taux R obtenu à l'étape e') est supérieur au seuil, préférablement au seuil de 0,5 µg/mL, la méthode comprend en outre une étape consistant à calculer le taux de D-dimères spécifiques de la maladie thromboembolique veineuse ($Ddi_{MTEV}$) en utilisant l'équation suivante:

$$Ddi_{MTEV} = 0{,}5 \text{ x } \frac{R}{Ddi_E}$$

où DdiE est le taux de D-dimères dans l'échantillon.

Le taux $Ddi_{MTEV}$ est représentatif de l'étendue de l'embolie pulmonaire ou de la thrombose veineuse profonde, et donc de la gravité de la maladie.

Dans certains modes de réalisation, la première ou la deuxième version de la méthode selon l'invention est caractérisée en ce que l'échantillon sanguin a un volume compris entre 1 µL et 300 µL, de préférence compris entre 50 µL et 200 µL. De préférence, l'échantillon sanguin est non-dilué.

Le dosage des D-dimères de l'échantillon peut être effectué selon une méthode immuno-turbidimétrique ou immuno-enzymatique.

Dans certains modes de réalisation, l'étape a) d'une méthode de dosage selon l'invention est réalisée en mélangeant l'échantillon sanguin du patient avec du facteur tissulaire et optionnellement des phospholipides, préférablement en mélangeant l'échantillon sanguin du patient avec du facteur tissulaire et des phospholipides. Le facteur tissulaire de l'étape a) peut être présent en une concentration comprise entre 0,5 et 5 pM, de préférence 2 pM. Le mélange de l'étape a) peut comprendre des ions calcium pour déclencher la génération de thrombine et la formation de fibrine.

Dans certains modes de réalisation, l'étape b) d'une méthode de dosage selon l'invention est caractérisée en ce qu'elle comprend l'incubation du mélange obtenu à l'étape a) pendant un temps compris entre 20 secondes et 400 secondes, de préférence compris entre 60 secondes et 300 secondes, à une température entre 30°C et 40°C.

Dans certains modes de réalisation, dans l'étape b), déclencher la génération de thrombine et la formation d'un caillot de fibrine est effectué en ajoutant des ions calcium à l'échantillon incubé.

Dans certains modes de réalisation, l'échantillon sanguin utilisé dans une méthode de dosage selon l'invention est un échantillon de plasma. L'échantillon de plasma peut être un échantillon de plasma pauvre en plaquettes. Dans ces modes de réalisation, dans l'étape c), mesurer la variation temporelle d'au moins une propriété de l'échantillon obtenu en b) est effectué en mesurant la variation temporelle de la densité optique (DDO) à une longueur d'onde comprise entre 350 et 800 nm, de préférence à la longueur d'onde de 540 nm. Préférablement, la mesure de la densité optique de l'étape c) se fait à la même longueur d'onde que celle utilisée pour le dosage des D-dimères, 540 nm.

Dans d'autres modes de réalisation, l'échantillon sanguin utilisé dans une méthode de dosage selon l'invention est un échantillon de sang total. L'échantillon de sang total peut être un échantillon de sang total citraté. Dans ces modes de réalisation, dans l'étape c), mesurer la variation temporelle d'au moins une propriété de l'échantillon obtenu en b) est effectué par thromboélastographie, rhéométrie ou par analyse d'images.

Dans certains modes de réalisation, une méthode de dosage selon l'invention est caractérisée en ce qu'au moins les étapes c) et d) sont réalisées sur un automate de diagnostic ou sur un analyseur de biologie délocalisée, de préférence sur un analyseur de coagulation.

Dans un second aspect, la présente invention concerne une méthode *in vitro* de diagnostic de la maladie thromboembolique veineuse (MTEV) chez un patient, comprenant des étapes consistant à:

- effectuer un dosage des D-dimères spécifiques de MTEV dans un échantillon sanguin du patient en utilisant une méthode de dosage des D-dimères spécifiques de la MTEV décrite ici; et
- fournir un diagnostic concernant le patient.

Dans une méthode *in vitro* de diagnostic selon l'invention, le diagnostic concernant le patient est (i) exclusion de la thrombose, (ii) état d'activation aigu de la coagulation, ou (iii) thrombose.

Dans les modes de réalisation où le patient est une personne âgée, un patient atteint de cancer, un patient atteint d'une infection ou un patient atteint d'une thrombophilie, le dosage des D-dimères spécifiques de la maladie thromboembolique veineuse est effectué en utilisant la deuxième version de la méthode de dosage de l'invention. Dans ce cas, le diagnostic concernant le patient est (i) exclusion de la thrombose, (ii) état d'activation aigu de la coagulation, (iii) thrombophilie, ou (iv) thrombose.

Une description plus détaillée de certains modes de réalisation préférés de l'invention est donnée ci-dessous.

## Description Détaillée de l'Invention

Comme mentionné ci-dessus, la présente invention concerne une méthode de détermination du taux de D-dimères spécifiques de la thrombose dans un échantillon biologique d'un patient et son application dans le diagnostic de la maladie thromboembolique veineuse (embolie pulmonaire ou thrombose veineuse profonde) ou l'identification d'un état d'activation de la coagulation.

## I - Méthode de Dosage des D-Dimères Spécifiques de la Maladie Thromboembolique Veineuse

Une méthode de dosage selon l'invention comprend deux étapes principales: un dosage des D-dimères, d'une part, et la mesure dynamique de la formation de fibrine, d'autre part. Ces étapes principales sont effectuées sur un échantillon sanguin du patient.

## A. Echantillon Sanguin du Patient

Le terme "patient", tel qu'utilisé ici, désigne un être humain. Le terme "patient" ne désigne pas un âge particulier, et englobe donc les enfants, les adolescents et les adultes, y compris les personnes âgées. Généralement, le patient est un sujet qui est suspecté d'embolie pulmonaire ou de thrombose veineuse profonde, comme par exemple un malade pris en charge en urgence à l'hôpital pour dyspnée et/ou douleur thoracique. Un tel patient peut, par ailleurs, n'avoir aucune condition médicale connue. Alternativement, un tel patient peut être connu pour avoir une prédisposition à la thrombose (par exemple un patient atteint d'un cancer, une femme enceinte ou venant d'accoucher, un patient en phase post-opératoire, un sujet en voyage ou qui vient d'effectuer un voyage, en particulier un voyage de longue durée); un patient présentant un état d'hyper coagulation (comme un patient atteint de thrombophilie, un patient atteint d'insuffisance rénale, un patient ayant subi un trauma, une chute ou une fracture, ou un sujet âgé); un patient présentant un état d'activation de la coagulation (comme un patient atteint d'infection ou de sepsis, un patient atteint de pneumopathie, de bronchite ou d'insuffisance respiratoire, un patient atteint de maladie inflammatoire, un patient atteint de gastrite, un patient atteint de cardiomyopathie, ou un patient avec des antécédents d'accident vasculaire cérébral). Dans le contexte de la présente invention, on parle de "patient normal" ou "patient sain", lorsque le patient ne présente pas de suspicion de thrombose.

La méthode selon l'invention utilise un simple échantillon biologique sanguin du patient. Un prélèvement sanguin (ou prise de sang) est réalisé au niveau d'une veine du patient en vue de récolter l'échantillon sanguin. De préférence, cet échantillon biologique sanguin est utilisé non dilué dans la méthode.

Dans certains modes de réalisation, la méthode selon l'invention est réalisée sur un échantillon de sang total (c'est-à-dire de sang avec tous ses constituants). Le sang total peut être citraté. Dans ce cas, le sang total prélevé est récolté dans un tube citraté.

Dans d'autres modes de réalisation, la méthode selon l'invention est réalisée sur un échantillon plasmatique obtenu à partir de l'échantillon sanguin. Les méthodes d'obtention de plasma à partir de sang humain sont connues dans l'art. De préférence, l'échantillon biologique est un échantillon de plasma pauvre en plaquettes (PPP). Dans ce cas, il peut notamment être obtenu par centrifugation du tube citraté, comprenant l'échantillon de sang de patient, pendant 15 minutes, à une vitesse de 2000 à 2500 g, dans une centrifugeuse thermostatée à une température comprise entre 18 et 22°C.

Si l'échantillon de plasma pauvre en plaquettes doit être conservé, il est possible d'utiliser le protocole suivant qui consiste à:

- décanter rapidement le plasma, en laissant environ 0,5 cm de plasma au-dessus de la couche cellulaire des globules blancs et plaquettes;
- récupérer le plasma dans un tube à hémolyse ou un tube plastique;
- centrifuger ce tube pendant 15 minutes, à une vitesse de 2000 à 2500 g, dans une centrifugeuse thermostatée à une température comprise entre 18 et 22°C;
- aliquoter en fractions de 0,5 à 1 ml sans prendre le fond du tube (qui contient les débris cellulaires); puis
- congeler rapidement à une température comprise entre -70°C et -90°C, de préférence à -80°C.

Le dosage selon l'invention peut être effectué sur n'importe quel volume approprié d'échantillon sanguin. Généralement, l'échantillon sanguin est utilisé dans la présente invention en faible volume. Par exemple, l'échantillon biologique sanguin a un volume compris entre 1 $\mu$L et 300 $\mu$L, de préférence entre 50 $\mu$L et 200 $\mu$L, de préférence un volume d'environ 200 $\mu$L pour un volume final de 300 $\mu$L après addition des réactifs (voir ci-dessous), de préférence un volume d'environ 100 $\mu$L pour un volume final de 150 $\mu$L après addition des réactifs. Un tel volume suffit en effet pour l'analyse sur instrument de routine, mais peut être réduit sur un dispositif de biologie délocalisée, à condition de respecter le rapport de volume d'échantillon sur le volume final (*i.e.*, rapport d'environ 2:3). Il peut être réduit davantage à un volume compris entre 5 $\mu$L et 20 $\mu$L, de préférence 10 $\mu$L, dans le cas d'un dispositif portable dont le réactif est lyophilisé et reconstitué par l'échantillon au moment de l'utilisation au lit du malade, pourvu que les mêmes concentrations finales réactionnelles soient obtenues.

## B. Dosage des D-dimères de l'Echantillon

Dans une méthode selon l'invention, la première étape principale (le dosage des D-dimères dans l'échantillon sanguin) peut être effectuée par n'importe quelle méthode appropriée. Dans certains modes de réalisation, il sera effectué un dosage habituel des D-dimères de l'échantillon. Par "dosage habituel des D-dimères de l'échantillon", on entend un dosage effectué selon une méthode immuno-turbidimétrique, comme la méthode latex ou une méthode immuno-enzymatique, comme la méthode ELISA ("enzyme-linked immunosorbent assay") ou la méthode ELFA ("enzyme-linked fluorescent assay"). Les méthodes immuno-turbidimétriques sont basées sur le changement de turbidité d'une solution comprenant les D-dimères. Typiquement, une méthode immuno-turbidimétrique comprend (i) le mélange de l'échantillon sanguin du patient avec une suspension de microparticules de latex coatées par des anticorps monoclonaux anti-antigène D-dimère ("méthode latex"); puis (ii) le suivi de la turbidité du mélange, notamment à une longueur d'onde donnée, typiquement à 540 nm. Une méthode immuno-enzymatique comprend la capture des D-dimères de l'échantillon par des anticorps monoclonaux anti-antigène D-dimère puis leur révélation par des anticorps secondaires marqués.

## C. Mesure Dynamique de la Formation de Fibrine

La méthode selon l'invention est basée sur trois constats: l'hyper coagulation et l'hyper fibrinolyse sont absentes ou réduites dans la thrombose, contrairement aux états d'activation de la coagulation; en revanche, l'état inflammatoire est de première importance.

Comme indiqué plus haut, les présents Inventeurs ont mis au point deux versions de la méthode de dosage des D-dimères spécifiques de la maladie thromboembolique veineuse. La mesure dynamique de la formation de fibrine selon la première version de la méthode de l'invention contient plusieurs étapes: les étapes a), b), c), d), e), f), et g), et une étape de diagnostic, l'étape h). La mesure dynamique de la formation de fibrine selon la deuxième version de la méthode selon l'invention contient les mêmes étapes a), b), c), et d) que la première version, et des étapes e'), f'), et g'), et une étape de diagnostic, l'étape h') qui diffèrent de celles de la première version. Ces étapes sont décrites en détail ci-dessous.

*Etapes de la Méthode*

*Etape a)*

L'étape a) de la méthode de dosage selon l'invention consiste à initier l'activation de la coagulation dans l'échantillon sanguin sans la déclencher. L'initiation de l'activation de la coagulation peut être réalisé par n'importe quelle méthode appropriée connue dans l'art, que ce soit par la voie intrinsèque qui consiste en l'activation du facteur de Hageman ou facteur XII de la coagulation (c'est ce qui se produit lorsque celui-ci entre en contact avec le collagène dénudé d'un vaisseau lésé), ou la voie extrinsèque, qui est initiée lors d'une lésion tissulaire par des facteurs tissulaires et aboutit à la coagulation du sang. Par exemple, par la voie extrinsèque, l'étape a) peut comprendre le mélange de l'échantillon biologique sanguin avec du facteur tissulaire et optionnellement des phospholipides. Dans certains modes de réalisations préférés, l'étape a) comprend le mélange de l'échantillon sanguin avec du facteur tissulaire et optionnellement des phospholipides pour initier l'activation de la voie extrinsèque de la coagulation sans la déclencher. Dans ce cas, l'échantillon biologique sanguin est mélangé à une solution de facteur tissulaire (FT), de préférence humain, et de phospholipides (PL), de préférence semi-purifiés ou purifiés. Le facteur tissulaire et les phospholipides sont de préférence lyophilisés pour être reconstitués par l'échantillon biologique sanguin, notamment dans le cas d'un dispositif de biologie délocalisée ou d'un dispositif portable.

Le mélange de l'étape a) peut comprendre une concentration finale de phospholipides de 2 à 5 $\mu$M dans le mélange, de préférence une concentration d'environ 4 $\mu$M. De préférence, le facteur tissulaire est utilisé en quantité telle que sa concentration finale dans le mélange avec l'échantillon biologique sanguin soit comprise entre 0,5 et 20 pM, de préférence entre 1 et 5 pM, par exemple 2 pM.

Le mélange de l'étape a) peut comprendre également des ions calcium. Les ions calcium peuvent être présents à une concentration finale de 15 à 20 mM, de préférence de 17 mM.

Dans certains modes de réalisation, l'étape a) de la méthode selon l'invention comprend:

al) l'introduction de facteur tissulaire dans une solution de phospholipides et éventuellement d'ions calcium, puis
a2) le mélange de la solution obtenue en a1) avec l'échantillon biologique sanguin.

Dans d'autres modes de réalisation, l'étape a) de la méthode selon l'invention comprend :

al) l'introduction de phospholipides à une solution de facteur tissulaire, et optionnellement l'ajout d'ions calcium, puis
a2) la lyophilisation de la solution obtenue en a1) pour être reconstituée avec l'échantillon biologique sanguin.

A la fin de l'étape a), un mélange d'au moins le facteur tissulaire avec l'échantillon biologique de plasma ou un mélange d'au moins le facteur tissulaire avec l'échantillon biologique de sang total est obtenu pour la détermination du profil de formation de fibrine jusqu'au plateau de polymérisation.

*Etape b)*

L'étape b) consiste à incuber le mélange obtenu à l'étape a) puis à déclencher, dans l'échantillon incubé, la génération de thrombine et la formation d'un caillot de fibrine. Le déclenchement de la génération de thrombine et de la formation du caillot de fibrine peut être réalisé par n'importe quelle méthode connue dans l'art. Ce déclenchement implique une cascade complexe de facteurs de coagulation de la voie intrinsèque ou extrinsèque, qui débouche en la transformation du fibrinogène en fibrine polymérisée, ce qui crée le caillot de fibrine. Dans certains modes de réalisation particuliers, ce déclenchement est effectué par ajout d'ions calcium dans le mélange obtenu.

L'étape b) comprend ainsi l'incubation du mélange obtenu à l'étape a). L'incubation peut être réalisée dans n'importe quelles conditions appropriées de temps et de température. Cette incubation peut typiquement se faire pendant un temps compris entre 20 secondes et 400 secondes, de préférence compris entre 60 secondes et 300 secondes, de préférence de 300 secondes sur l'instrument de routine, à une température comprise entre 30°C et 40°C, de préférence à une température d'environ 37°C. Ce temps d'incubation peut être raccourci à moins de 60 secondes, de préférence moins de 30 secondes avec des volumes réduits, en particulier avec l'utilisation d'un instrument de biologie délocalisée, de préférence avec l'utilisation d'un dispositif portable.

Des ions calcium sont ensuite ajoutés au mélange incubé Ces ions calcium peuvent être ajoutés sous forme de solution de CaCl$_2$, à une concentration d'environ 0,1 M. Par l'ajout de calcium, en présence de facteur tissulaire, la génération de thrombine et la formation d'un caillot de fibrine sont déclenchées.

*Etape c)*

L'étape c) consiste à mesurer la variation temporelle d'au moins une propriété de l'échantillon dans lequel se forme le caillot de fibrine. La propriété peut être n'importe quelle propriété optique ou physique qui permet de suivre la formation du caillot de fibrine.

Dans certains modes de réalisation, en particulier dans les cas où l'échantillon biologique utilisé est un échantillon de plasma, la propriété mesurée est préférablement une propriété optique, en particulier la densité optique (DO) (également appelée absorbance) à au moins une longueur d'onde. La longueur d'onde peut être comprise entre 350 nm et 800 nm. De préférence, la mesure de densité optique est réalisée à la longueur d'onde de 540 nm, qui est celle utilisée pour le dosage habituel des D-dimères.

Ainsi, par exemple, lors de l'étape c), la formation du caillot de fibrine est suivie de façon dynamique, par exemple toutes les 2 secondes ou moins, par mesure de la densité optique à au moins une longueur d'onde comprise entre 350 nm et 800 nm, préférablement à 540 nm, et pendant une durée comprise entre 1 et 10 minutes, de préférence 10 minutes. Pendant cette durée, la formation du caillot de fibrine est donc analysée, notamment par les variations de la densité optique (DDO) par rapport à la densité optique au temps de base (ou temps initial $t_i$), qui peut, par exemple, être compris entre 10 et 20 secondes. Ainsi, à la longueur d'onde d'analyse $\lambda$, et au temps t de mesure, $DDO(\lambda)_t$ correspond à la valeur de la densité optique mesurée au temps t à la longueur d'onde $\lambda$ ($DO(\lambda)_t$) moins la valeur de la densité optique mesurée au temps $t_i$ à la longueur d'onde $\lambda$ ($DO(\lambda)_{ti}$) et calculé comme suit:

$$DDO(\lambda)_t = DO(\lambda)_t - DO(\lambda)_{ti}$$

Alternativement, la propriété de l'échantillon dans lequel se forme le caillot de fibrine mesurée de façon dynamique au cours de l'étape c) de la méthode selon l'invention est une propriété physique, qui peut être la turbidité, l'élasticité, la viscosité, la viscoélasticité, le module de rigidité, etc...

Ainsi, dans d'autres modes de réalisation, en particulier dans les cas où l'échantillon biologique utilisé est un échantillon de sang total, la propriété physique du caillot de fibrine suivie de façon dynamique au cours de l'étape c) est préférablement mesurée par thromboélastographie, rhéométrie ou par analyse d'images. D'une manière générale, la formation du caillot de fibrine est suivie par la variation temporelle des propriétés physiques du caillot, jusqu'à atteindre l'amplitude au temps d'atteinte du plateau de polymérisation de la fibrine, par exemple pendant une durée inférieure ou égale à 10 minutes, de préférence inférieure à 5 minutes.

*Etape d)*

L'étape d) de la méthode selon l'invention consiste tout d'abord à établir le profil de formation du caillot de fibrine analysé au cours de l'étape c).

Par "profil de formation du caillot de fibrine", on entend l'évolution d'au moins une propriété optique ou physique du caillot en fonction du temps au cours de la formation de fibrine jusqu'au plateau de polymérisation de la fibrine.

La mesure dynamique de la formation de fibrine, suivie par la détermination, en fonction du temps, d'au moins une propriété d'un échantillon sanguin dans lequel on initie la génération de thrombine et la formation d'un caillot de fibrine, fournit trois données:

- le temps de formation du caillot de fibrine (TFF),
- le temps d'atteinte (TA) du plateau de polymérisation de la fibrine, et
- la valeur de la propriété mesurée au temps TA, ($Vp_{(TA)}$).

Ainsi, l'étape d) consiste donc à extraire du profil de formation du caillot de fibrine établi, le temps de formation de la fibrine (TFF) mesuré au point d'inflexion de la tangente à la courbe et la valeur de la propriété optique ou physique ($Vp_{(TA)}$) mesurée au temps d'atteinte (TA) du plateau de polymérisation de la fibrine. L'homme du métier comprendra qu'alternativement la valeur de la propriété optique ou physique peut être mesurée au temps d'atteinte de plus de 90% du plateau de polymérisation, de préférence au temps d'atteinte de plus de 95% du plateau de polymérisation, par exemple 96%, ou 97%, ou 98% ou encore 99%.

Dans le cas où la propriété mesurée est une densité optique, c'est-à-dire en particulier dans le cas d'un échantillon de plasma, on extrait du profil de formation du caillot de fibrine (profil sigmoïde, qui ressemble à une courbe en S avec un plateau), le temps de formation de la fibrine (TFF) mesuré au point d'inflexion de la tangente de la courbe, et la $DDO_{(TA)}$ de l'échantillon mesuré au temps d'atteinte (TA) du plateau de polymérisation de la fibrine.

Dans le cas où la propriété physique est mesurée par thromboélastographie, c'est-à-dire en particulier dans le cas d'un échantillon de sang total, on extrait du profil de formation du caillot de fibrine (profil qui a la forme d'un diapason), le temps de formation de la fibrine (TFF) mesuré au point d'inflexion de la tangente de la courbe, et l'amplitude ($A_{(TA)}$) au temps d'atteinte (TA) du plateau de polymérisation de la fibrine.

Dans le cas où la propriété physique est mesurée par rhéométrie ou par analyse d'images, c'est-à-dire en particulier dans le cas d'un échantillon de sang total, on extrait du profil de formation du caillot de fibrine (profil sigmoïde, qui ressemble à une courbe en S avec un plateau), le temps de formation de la fibrine (TFF) mesuré au point d'inflexion de la tangente de la courbe, et l'amplitude ($A_{(TA)}$) au temps d'atteinte (TA) du plateau de polymérisation de la fibrine.

Cela est notamment illustré:

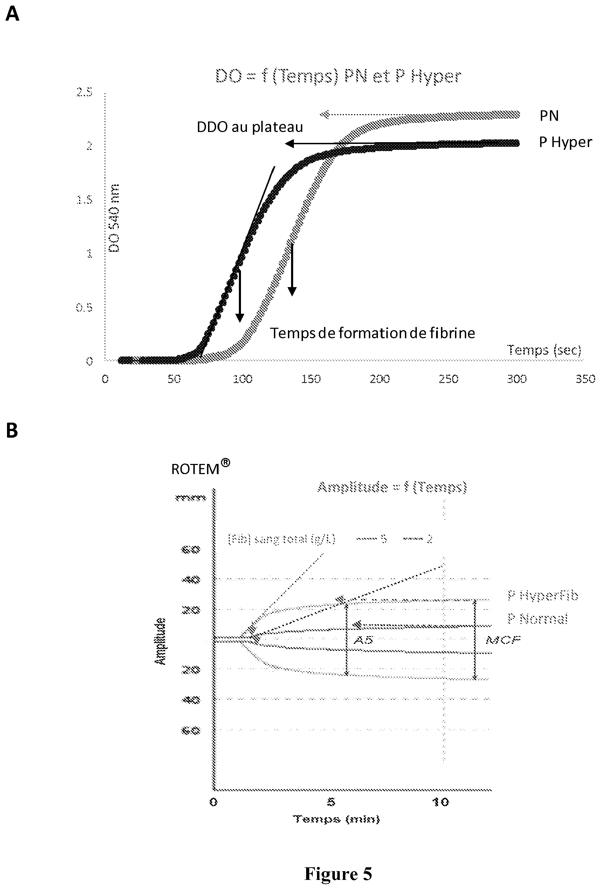

- en Figure 5a, avec un plasma de patient normal (sain) et un plasma de patient avec une hyper coagulation, et
- en Figure 5b, avec un sang total de patient normal et un sang total de patient avec une hyper fibrinogénémie.

Le temps de formation de la fibrine (TFF) déterminé au point d'inflexion de la tangente de la courbe ainsi que le temps d'atteinte (TA) du début du plateau de fibrine sont raccourcis proportionnellement à l'hyper coagulation, que ce soit avec le plasma hyper-coagulant pour la méthode utilisant la mesure de densité optique, ou avec le sang total ayant un taux de fibrinogène élevé, pour la méthode où la propriété physique est mesurée par thromboélastographie, rhéométrie ou par analyse d'images.

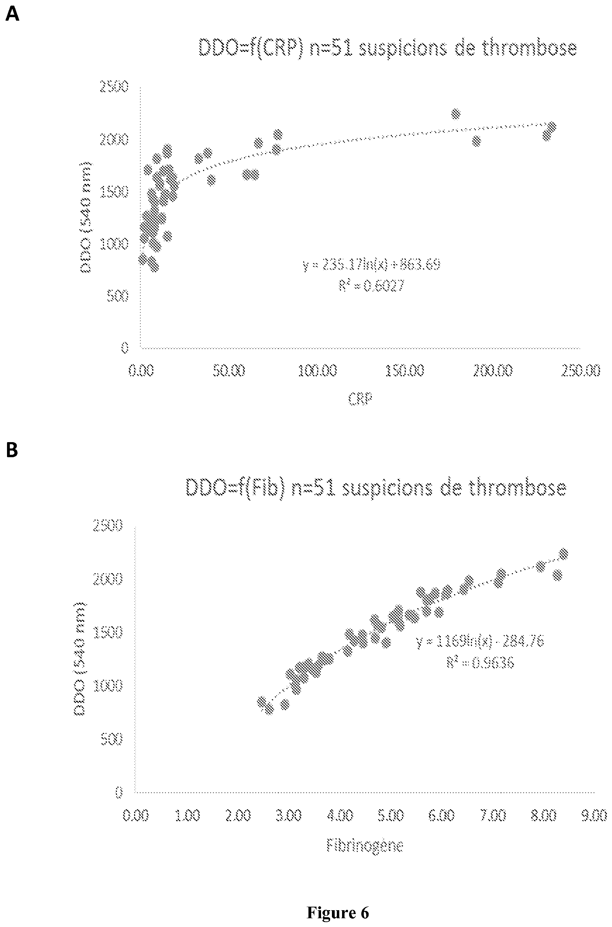

Le niveau du plateau de polymérisation de la fibrine, exprimé soit par la $DDO_{(TA)}$ au temps d'atteinte du plateau, soit par l'amplitude ($A_{(TA)}$) au temps d'atteinte du plateau, augmente avec l'inflammation, représentée soit par le taux de fibrinogène dans la Figure 5; soit par le taux de Protéine C réactive (CRP) dans la Figure 6(**A**) et le taux de fibrinogène dans la Figure 6(**B**), dans les échantillons de patients pour lesquels les deux protéines (CRP et fibrinogène) ont été dosées.

La valeur de DO au temps d'atteinte du plateau est moins élevée pour le plasma de patient avec une hyper coagulation que pour le plasma de patient normal (3,5 g/L), car il contient moins de fibrinogène (3,0 g/L).

L'amplitude A au temps d'atteinte du plateau est plus élevée pour le sang total ayant un taux de fibrinogène élevé (5g/L) que pour le sang normal qui a un taux de fibrinogène normal (2,5 g/L).

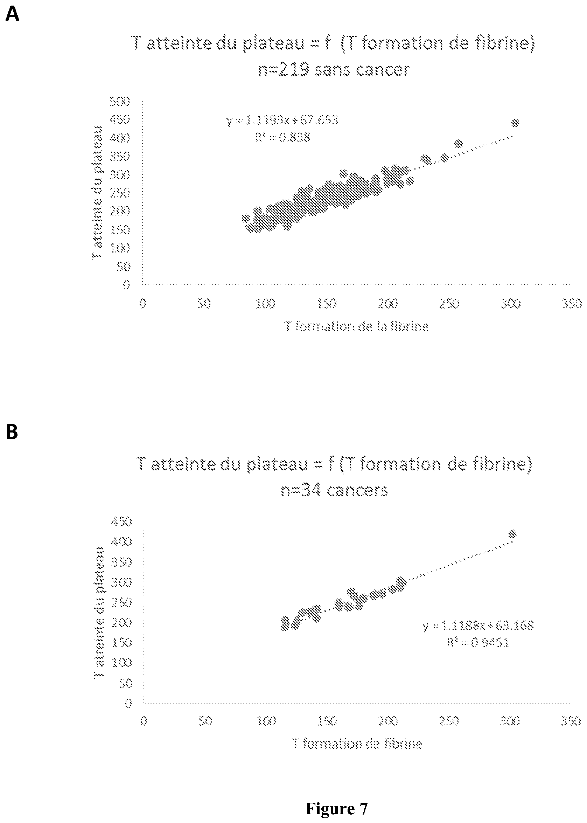

Le temps d'atteinte du plateau de polymérisation de la fibrine est proportionnel au temps de formation de la fibrine, pour tous les patients avec suspicion de thrombose, comme montré en Figure 7(**A**) pour les patients sans cancer actif, qu'ils aient ou non une thrombose veineuse et en Figure 7(**B**) pour les patients avec un cancer actif. Ce temps d'atteinte du plateau ne dépasse pas 8 minutes, quels que soient les patients, en absence de traitement anticoagulant.

***Première Version de la Méthode - Etapes e), f), g) et h)***

La première méthode selon l'invention permet de déterminer le taux des D-dimères spécifiques de la maladie thromboembolique veineuse (MTEV), c'est-à-dire le taux des D-dimères provenant spécifiquement de la dégradation de la fibrine intravasculaire, en ajustant le taux des D-dimères de l'échantillon en fonction:

- du taux des D-dimères générés par l'hyper coagulation, après initiation de la génération de la thrombine et de fibrine;
- du taux des D-dimères générés par l'inflammation; et
- du taux des D-dimères générés par l'hyper fibrinolyse.

Une telle méthode permet de déterminer le taux de D-dimères spécifiques de la maladie thromboembolique veineuse pour le patient concerné, et ce, en un temps très court (i.e., moins de 10 minutes) comme décrit notamment dans l'Exemple 1. En effet, les différents plasmas de patients avec et sans embolie pulmonaire, avec et sans thrombose veineuse profonde, et avec ou sans état d'activation de la coagulation, sont discriminés en moins de 10 minutes, sur leur profil de formation du caillot de fibrine après initiation de la génération de thrombine.

***Etape e)***

Les présents Inventeurs ont tout d'abord mis au point une première version de la méthode dans laquelle l'étape e) consiste à calculer le taux de D-dimères qui résultent de la dégradation de la fibrine intravasculaire en ajustant le taux de D-dimères de l'échantillon en fonction du taux de D-dimères générés par l'hyper coagulation et en fonction du taux des D-dimères générés par l'inflammation. Plus spécifiquement, le taux des D-dimères résultant de la dégradation de la fibrine intravasculaire (R) est calculé:

e1) en ajustant le taux de D-dimères de l'échantillon ($Ddi_E$) en fonction du taux des D-dimères générés par l'hyper coagulation en utilisant TFF déterminé en d), pour obtenir le taux de D-dimères ajustés en fonction de l'hyper coagulation, et

e2) en ajustant le taux de D-dimères ajustés en fonction de l'hyper coagulation calculé en e1) en fonction du taux des D-dimères générés par l'inflammation en utilisant $Vp_{(TA)}$ déterminé en d), pour obtenir R.

En effet, les présents Inventeurs ont observé qu'il y a un allongement systématique du temps de formation de la fibrine (TFF) dans tous les échantillons de patients avec embolie pulmonaire (EP) et/ou thrombose veineuse profonde (TVP), comparativement aux échantillons de patients sans thrombose, comme montré en Exemple 1. La fonction linéaire de l'Exemple 2 est mise à profit pour ajuster les D-dimères en fonction des D-dimères générés par l'hyper coagulation.
Le taux des D-dimères ajustés en fonction de l'hyper coagulation ($Ddi_{E/HC}$) est déterminé à partir du taux des D-dimères de l'échantillon ($Ddi_E$) par la formule suivante :

$$Ddi_{E/HC} = Ddi_E \ \text{x} \ \frac{TFF}{Temps\ Témoin}$$

où le Temps Témoin est le temps moyen de formation du caillot de fibrine d'échantillons de sujets sains normaux, ne présentant pas de suspicion de thrombose, mesuré par les étapes a)-d) de la méthode selon l'invention.
Ceci est montré notamment dans l'Exemple 3. Ainsi, l'étape e) comprend une étape de calcul des D-dimères, à partir du temps de formation de la fibrine (TFF) mesuré en d) pour prendre en compte les D-dimères résultant de l'hyper coagulation.
Le taux des D-dimères qui résultent de la dégradation de la fibrine intravasculaire (R) est calculé en ajustant le taux des D-dimères ajustés en fonction de l'hyper coagulation ($Ddi_{E/HC}$), en fonction de l'inflammation, par la formule suivante:

$$R = \frac{Ddi_{E/HC}}{[Fib]_{(Vp(TA))}}$$

où $[Fib]_{(Vp(TA))}$ est la concentration en fibrinogène déduite pour la valeur de la propriété ($Vp_{(TA)}$) sur la courbe étalon d'équation:

$$y = a \ln(x) - b,$$

où:

y est la valeur de la propriété mesurée au temps d'atteinte (TA) du plateau de polymérisation de la fibrine,
x est la concentration en fibrinogène,
a et b sont les constantes de l'équation logarithmique qui relie le niveau (la valeur ou l'amplitude) du plateau de fibrine, et la concentration en fibrinogène,
la courbe étalon ayant été établie en utilisant des échantillons sanguins dont la concentration en fibrinogène a été déterminée et dont la valeur de la propriété ($Vp_{(TA)}$) a été déterminée par les étapes a)-d) de la méthode selon l'invention.

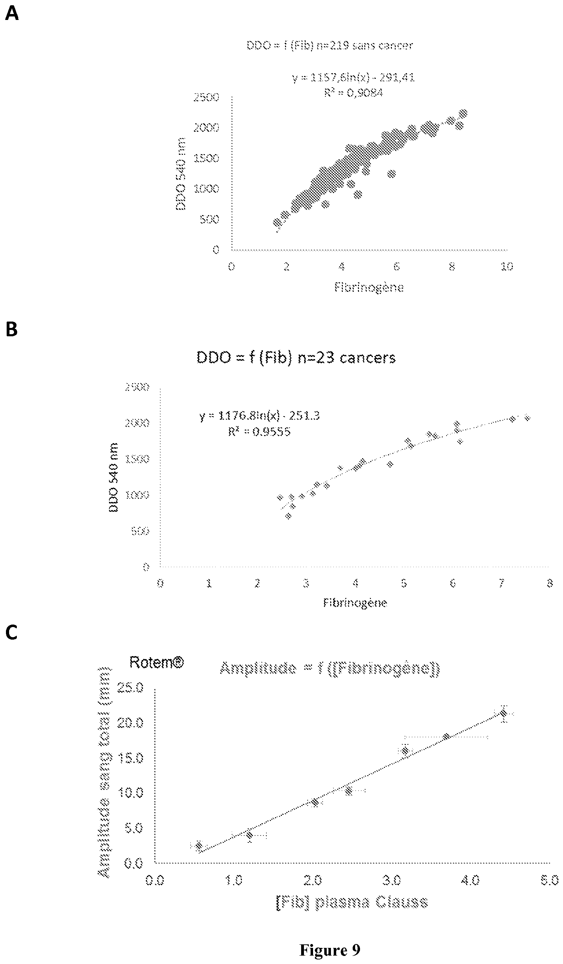

Le taux des D-dimères qui résultent de la dégradation de la fibrine intravasculaire (R) est ainsi exprimé en unités équivalentes au fibrinogène initial (FEU).
Comme indiqué plus haut, dans le cas d'une méthode basée sur la mesure de la densité optique, par exemple dans le cas d'un échantillon de plasma, $Vp_{(TA)}$ est $DDO_{(TA)}$, la densité optique mesurée au temps d'atteinte (TA) du plateau de polymérisation de la fibrine, et dans le cas de la méthode basée où la propriété physique est mesurée par thromboélastographie, rhéométrie ou par analyse d'images, par exemple dans le cas d'un échantillon de sang total, $Vp_{(TA)}$ est $A_{(TA)}$, l'amplitude mesurée au temps d'atteinte (TA) du plateau de polymérisation de la fibrine.
Des exemples de courbes étalons permettant la détermination du niveau de l'inflammation (c'est-à-dire de la concentration en fibrinogène) sont présentés dans les
Figures 9(**A**)-(**B**) et l'Exemple 4 dans le cas d'une méthode basée sur la mesure de DDO, et dans la Figure 9(C) et l'Exemple 4 dans le cas d'une méthode basée sur une mesure de propriété physique par thromboélastographie, respectivement.
Le taux de D-dimères obtenu est ainsi exprimé en unités équivalentes au fibrinogène initial (FEU).

*Etape f)*

L'étape f) de la première version de la méthode selon l'invention consiste à comparer le taux de D-dimères résultant de la dégradation de la fibrine obtenu en e), par rapport à un seuil, pour déterminer la probabilité d'une thrombose veineuse, notamment d'une embolie pulmonaire (EP) ou d'une thrombose veineuse profonde (TVP), dans l'échantillon du patient. De préférence, le seuil est de 0,50 μg/mL. En effet, de préférence, la quantité de D-dimères générés par la plasmine étant environ 50% de l'unité FEU (unités équivalents au fibrinogène) avec les anticorps 8D2 et 2.1.16 utilisés, le seuil de positivité de la mesure est ainsi de 0,50 μg/mL. De préférence, ce seuil est le même que celui du dosage habituel des D-dimères.

Un taux de D-dimères résultant de la dégradation de la fibrine, R, obtenu en e) inférieur au seuil (préférablement au seuil de 0,5 μg/mL) permet d'exclure une thrombose chez le patient. Un taux de D-dimères résultant de la dégradation de la fibrine, R, obtenu en e) supérieur au seuil (préférablement au seuil de 0,5 μg/mL) est indicatif de la possibilité d'une thrombose chez le patient.

Notamment, parmi les patients avec un taux de D-dimères obtenu en e) déterminé par le ratio R et exprimés en unités FEU (unités équivalents au fibrinogène), plus de 90% des patients sans thrombose ont un taux négatif (c'est-à-dire inférieur au seuil de 0,5 μg/mL) et tous les patients avec thrombose ont un taux positif (c'est-à-dire supérieur au seuil de 0,5 μg/mL) variant de 0,50 μg/mL à 10,5 μg/mL, comme le montre le Tableau 4. Parmi les patients sans thrombose, trouvés faussement positifs (<10%) avec un taux de 0,50 μg/mL à 2,7 μg/mL, un tiers ont un taux < 0,60 μg/mL, un tiers ont un cancer, et un tiers ont un état d'activation de la coagulation (fracture, micro-angiopathie thrombotique, grossesse ou post-opératoire). Ainsi, 80% des patients avec un taux de D-dimères faussement positif sont rendus négatifs avec l'ajustement des D-dimères en fonction des D-dimères générés par l'hyper coagulation et l'inflammation.

Généralement, lorsque la comparaison effectuée à l'étape f) permet d'exclure la thrombose chez le patient, la méthode peut être stoppée à l'étape f). Dans le cas contraire, on poursuit la méthode.

*Etape g)*

La première version de la méthode selon l'invention comprend ensuite une étape g) de détermination du taux des produits de dégradation du fibrinogène (PDFg) générés par l'hyper fibrinolyse présente dans les états d'activation de la coagulation. Ainsi, l'étape g) consiste à:

g1) déterminer le taux de PDFg à partir du taux de D-dimères dans l'échantillon (Ddi$_E$),
g2) déterminer le taux de PDFg à partir du taux de D-dimères ajusté (R) obtenu à l'étape e), et
g3) calculer le taux de PDFg générés par l'hyper fibrinolyse (PDFg$_{HF}$) en soustrayant le taux de PDFg obtenu en g2) du taux de PDFg obtenu en g1).

En effet, des taux élevés de D-dimères sont générés dans les cas de cancer et dans les états d'activation importante de la coagulation, en association avec des taux importants de produits de dégradation du fibrinogène (PDFg), formés sous l'action de la plasmine présente en grande quantité.

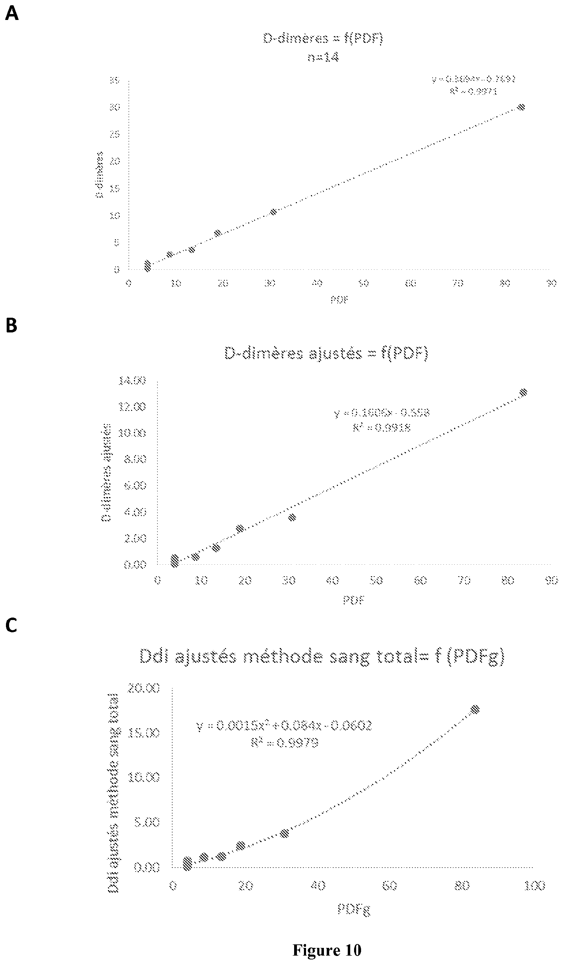

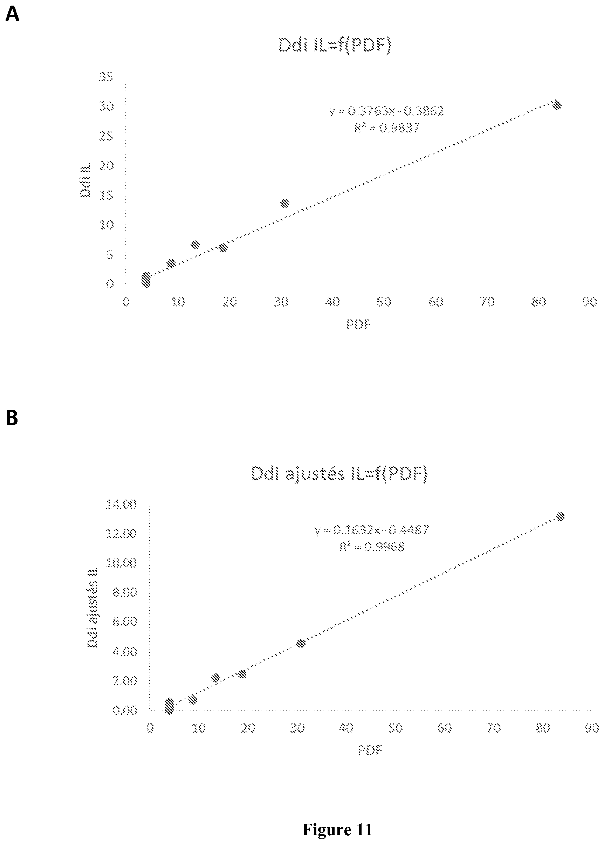

Notamment, le taux des D-dimères de l'échantillon déterminé par dosage habituel ainsi que le taux des D-dimères ajustés en fonction des D-dimères générés par l'hyper coagulation et l'inflammation selon la méthode de l'invention réalisée sur des échantillons plasmatiques (étape e) ci-dessus), sont parfaitement corrélés au taux de PDFg mesuré par la méthode latex dans ces échantillons, et résultant de l'hyper fibrinolyse, comme le montre la Figure 10; et ceci quel que soit le réactif des D-dimères utilisé, comme le montre la Figure 11. Il en est de même pour le taux des D-dimères ajustés en fonction des D-dimères générés par l'hyper coagulation et l'inflammation selon la méthode de l'invention réalisée sur des échantillons de sang total. Le taux de PDFg peut ainsi être calculé par régression linéaire à partir du taux de D-dimères obtenu.

Dans les états d'activation importante de la coagulation, les Inventeurs ont observé que:

- l'hyper fibrinolyse est importante et génère de grandes quantités de PDFg,
- les PDFg générés à partir du fibrinogène, sont systématiquement plus faibles dans les échantillons provenant de patients avec embolie pulmonaire (EP) et/ou thrombose veineuse profonde (TVP) que dans les échantillons provenant de patients sans thrombose. Ainsi, une différence positive entre les PDFg générés en fonction des D-dimères habituels et ceux générés en fonction des D-dimères ajustés obtenus en e), permet de discriminer les patients avec thrombose de ceux sans thrombose, à un seuil qui est de 1 μg/mL dans l'Exemple 6; et ceci quel que soit le réactif des D-dimères utilisé, comme le montre la Figure 11,
- le temps de formation de fibrine (TFF) est raccourci dans les états d'activation de la coagulation, comme décrit dans l'Exemple 1, au seuil du Temps Témoin - 1 écart type. Ceci est mis à profit pour exclure une thrombose et orienter sur un état d'activation aigu de la coagulation, en présence d'une hyper fibrinolyse importante, comme décrit en Exemple 6.

Cela permet de déterminer directement et rapidement la probabilité d'une embolie pulmonaire ou d'une thrombose veineuse profonde et/ou d'orienter sur une activation de la coagulation dans ledit échantillon du patient, comme montré dans les Exemples 5, 6 et 7. Plus préférentiellement, cela permet, en présence de taux élevés de D-dimères, d'augmenter la probabilité d'EP et de TVP, voire d'orienter sur l'étendue de l'embolie pulmonaire ou de la thrombose veineuse comme montré en Exemple 8.

Ainsi, une différence positive entre les PDFg générés en fonction du taux des D-dimères de l'échantillon ($Ddi_E$) et ceux générés en fonction du taux des D-dimères ajustés (R) obtenus en e), permet de discriminer les patients avec possibilité de thrombose de ceux sans thrombose, à un seuil prédéterminé. Dans le cas des Exemples présentés ci-dessous, les Inventeurs ont prédéterminé un seuil de 1 $\mu$g/mL.

Plus spécifiquement, l'étape g) consiste à:

g1) déterminer le taux de PDFg correspondant au taux de D-dimères dans l'échantillon ($Ddi_E$) sur une courbe étalon établie en utilisant des échantillons sanguins avec des taux de PDFg et des taux de D-dimères connus,
g2) déterminer le taux de PDFg correspondant au taux de D-dimères ajusté (R) obtenu à l'étape e) sur cette même courbe étalon, et
g3) calculer le taux de PDFg générés par l'hyper fibrinolyse ($PDFg_{(HF)}$) en soustrayant le taux de PDFg obtenu en g2) du taux de PDFg obtenu en g1).

## Etape h)

Enfin, l'étape h) de la première version de la méthode selon l'invention comprend la comparaison du taux de PDFg générés par l'hyper fibrinolyse ($PDFg_{(HF)}$) obtenu en g) par rapport à un seuil et la comparaison du temps de formation de la fibrine (TFF) obtenu en d) par rapport à un seuil.

Plus spécifiquement, l'étape h) comprend une première étape consistant à:
h1) comparer $PDFg_{(HF)}$ à un seuil (par exemple un seuil 1 $\mu$g/mL), où $PDFg_{(HF)}$ inférieur au seuil ou négatif permet d'exclure la thrombose chez le patient, et où $PDFg_{(HF)}$ supérieur au seuil est indicatif d'une possibilité de thrombose chez le patient.

L'homme du métier reconnaîtra que le seuil est un seuil prédéterminé qui est établi en utilisant un nombre important d'échantillons. Sur la base des échantillons étudiés, les Inventeurs ont utilisé un seuil prédéterminé de 1 $\mu$g/mL.

L'étape h) comprend ensuite une deuxième étape consistant à:
h2) comparer TFF à un seuil, notamment un seuil égal au [Temps Témoin - 1 écart type], par exemple un seuil de 120 secondes pour un Temps Témoin de 135 secondes, où TFF inférieur au seuil est indicatif d'un patient sans thrombose mais présentant un état d'activation aigu de la coagulation, et un TFF supérieur au seuil est indicatif d'une thrombose chez le patient, et où le Temps Témoin est défini à l'étape e1).

Là encore, l'homme du métier reconnaîtra que le seuil est un seuil prédéterminé qui est établi en utilisant un nombre important d'échantillons. Sur la base des échantillons étudiés, les Inventeurs ont utilisé un seuil prédéterminé de 120 secondes pour un Temps Témoin de 135 secondes.

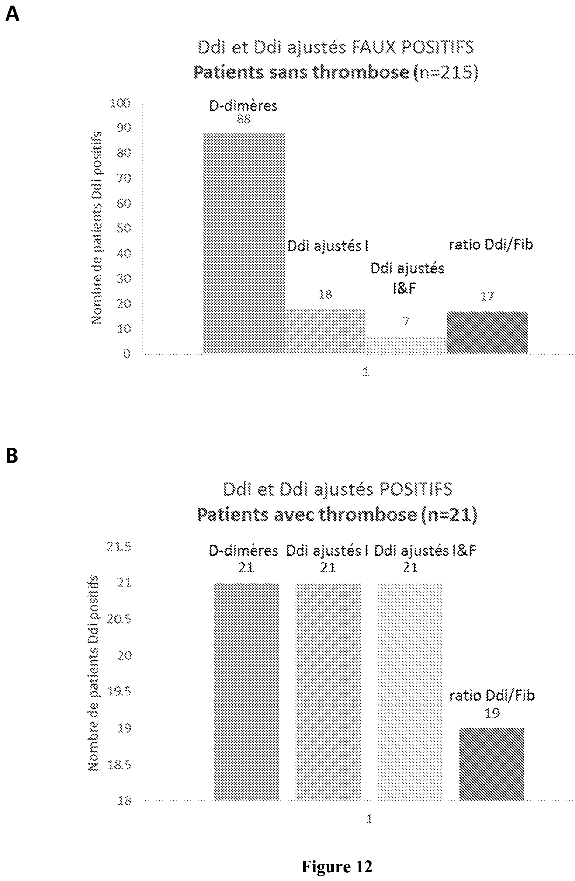

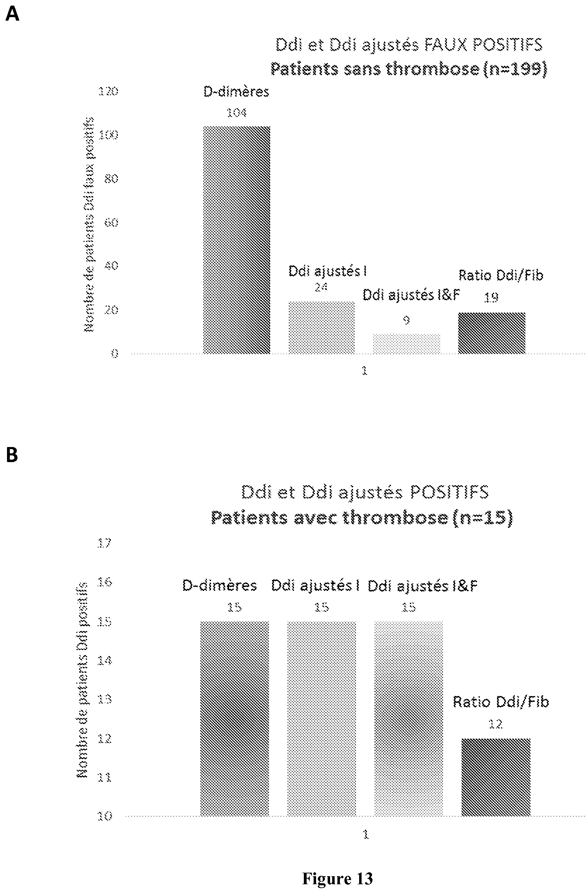

Ainsi, 92% des patients avec un taux de D-dimères faussement positif, soit 97% des patients sans thrombose, sont rendus négatifs avec l'ajustement des D-dimères selon l'étape h), notamment comme le montre la Figure 12, quel que soit le réactif de dosage des D-dimères comme le montre la Figure 13.

De préférence, des taux élevés de D-dimères spécifiques de la thrombose veineuse obtenus à la fin de la méthode sont représentatifs de l'étendue de l'embolie pulmonaire (EP) ou de la thrombose veineuse profonde (TVP). Ainsi, dans le cas où une thrombose est diagnostiquée chez le patient par une méthode selon l'invention, le taux de D-dimères, R, obtenu à l'étape e), ramené à une échelle utilisée en clinique, c'est-à-dire par rapport au seuil de 0,5 $\mu$g/mL, est proportionnel à l'étendue de l'embolie pulmonaire ou de la thrombose veineuse, donc à la gravité de la maladie. En effet, le diagnostic de gravité est habituellement déterminé à l'imagerie par le nombre et le type d'artères pulmonaires atteintes.

Plus spécifiquement, dans le cas où une thrombose est diagnostiquée chez le patient à l'étape h2) d'une méthode selon l'invention, le taux de D-dimères spécifiques de la maladie thromboembolique veineuse ($Ddi_{MTEV}$) est le taux de D-dimères, R, obtenu à l'étape e).

Dans le cas où une thrombose est exclue chez le patient aux étapes h1) et h2) d'une méthode selon l'invention mais que le taux R obtenu à l'étape e) est supérieur au seuil, préférablement au seuil de 0,5 $\mu$g/mL, le taux de D-dimères spécifiques de la maladie thromboembolique veineuse ($Ddi_{MTEV}$) est calculé par:

$$Ddi_{MTEV} = 0,5 \text{ x } \frac{R}{Ddi_E}$$

où R est le taux des D-dimères obtenu à l'étape e) et $Ddi_E$ est la taux de D-dimères de l'échantillon. Ce calcul permet de ramener le taux de D-dimères spécifiques de la maladie thromboembolique veineuse à un taux inférieur au seuil utilisé en clinique.

Les taux de D-dimères spécifiques de la thrombose veineuse ainsi fournis en fin de méthode sont alors représentatifs de l'étendue de l'embolie pulmonaire thrombose veineuse profonde.

Par "taux représentatif de l'étendue de l'EP ou de la localisation de la TVP", on entend que le taux de D-dimères spécifiques de la thrombose veineuse est directement corrélé à l'étendue de l'EP (i.e. à sa gravité) ou à la localisation de la TVP. Par "taux élevés", on entend des taux d'environ 1 $\mu$g/mL à 4 $\mu$g/mL ou plus, comme le montre le Tableau 7.

### Deuxième Version de la Méthode - Etapes e'), f'), g') et h')

La deuxième version de la méthode selon l'invention qui permet de déterminer le taux des D-dimères spécifiques de la maladie thromboembolique veineuse (MTEV) comprend les étapes a), b), c) et d) identiques à celles de la première méthode et des étapes e'), f'), g') et h') différentes de celles de la première version.

### Etape e')

Une deuxième variante de l'étape e) a été mise au point par les Inventeurs qui ont remarqué que dans la majorité (75%) des cas de maladie thromboembolique veineuse (MTEV), les taux de D-dimères sont inférieurs à 4 $\mu$g/mL. L'hyper coagulation et l'hyper fibrinolyse sont absentes ou réduites dans la thrombose, contrairement aux états d'activation de la coagulation; en revanche, l'état inflammatoire est de première importance. En effet, 80% des MTEV se trouvent chez un premier groupe de patients sans inflammation (concentration en Fibrinogène: 3,0-4,5 g/L) pour moitié, et chez un deuxième groupe de patients avec une inflammation subnormale (concentration en Fibrinogène: 4,5-6,0 g/L) pour moitié. De plus, le temps d'atteinte (TA) du plateau de la polymérisation du caillot de fibrine est plus rapide dans la thrombose, et permet de différencier une MTEV d'un état d'activation de la coagulation ou d'une thrombophilie.

En conséquence, l'étape e') de la deuxième version de la méthode selon l'invention consiste à:

- calculer le taux des D-dimères qui résultent de la dégradation de la fibrine intravasculaire:

    e'1) en ajustant le taux de D-dimères de l'échantillon en fonction du taux des D-dimères générés par l'inflammation, et
    e'2) en corrigeant ce taux ajusté pour les Ddi faibles (<4 $\mu$g/mL), et

- classer l'échantillon sanguin du patient en fonction de l'inflammation.

Ainsi, comparé à l'étape e), dans l'étape e'), l'ajustement du taux de D-dimères de l'échantillon en fonction du taux des D-dimères générés par l'hyper coagulation en utilisant le temps de formation de la fibrine (TFF) est supprimé. Cela permet de détecter une thrombose en cas de thrombophilie sous-jacente pour laquelle le TFF est court (TFF < [Temps Témoin moyen - 1 écart type], par exemple TFF ≤ 120 secondes pour un témoin à 135 secondes) et la prévalence de 2,5 pour 1000.

La correction du taux ajusté pour les Ddi faibles permet de détecter une thrombose:

    (i) en cas d'embolie pulmonaire sous-segmentaire ou sans gravité, dont les D-dimères sont en général faibles (≤ 1,5 $\mu$g/mL) et la prévalence voisine de 20% des EP, et
    (ii) en cas d'infarctus pulmonaire (complications de l'embolie pulmonaire), dont l'inflammation est importante (≥ 5 g/L) et la prévalence voisine de 15% des EP.

Enfin, le classement de l'échantillon du patient en fonction de l'inflammation permet de déterminer une thrombose en cas de cancer (20% des patients), d'infection (10% des patients), de sujets âgés avec un terrain inflammatoire sous-jacent.

Plus spécifiquement, dans l'étape e'), le taux des D-dimères qui résultent de la dégradation de la fibrine intravasculaire R est déterminé:

e'1) en calculant Ddi$_{E/I}$, le taux des D-dimères ajustés en fonction de l'inflammation, à partir du taux des D-dimères de l'échantillon (Ddi$_E$) en utilisant la valeur de la propriété mesurée au temps d'atteinte du plateau de polymérisation, Vp$_{(TA)}$, déterminée à l'étape d); et

e'2) en corrigeant le taux Ddi$_{E/I}$ obtenu en e'1) pour les taux de D-dimères < 4 $\mu$g/mL, pour obtenir le taux de D-dimères qui résultent de la dégradation de la fibrine intravasculaire (R).

*Etape e'1).* Ainsi, l'étape e'1) consiste à calculer le taux des D-dimères ajustés en fonction de l'inflammation, Ddi$_{E/I}$, à partir du taux des D-dimères de l'échantillon (Ddi$_E$) en utilisant la valeur de la propriété mesurée au temps d'atteinte du plateau de polymérisation, Vp$_{(TA)}$, déterminé à l'étape d), par l'équation suivante:

$$Ddi_{E/I} = \frac{Ddi_E}{[Fib]_{(Vp(TA)}}$$

où [Fib]$_{(Vp(TA)}$ est la concentration en fibrinogène déduite pour la valeur de la propriété (Vp$_{(TA)}$) sur la courbe étalon d'équation y = a ln(x) - b, où :

y est la valeur de la propriété mesurée au temps d'atteinte (TA) du plateau de polymérisation de la fibrine,
x est la concentration de fibrinogène,
a et b sont des constantes de l'équation logarithmique qui relie le niveau (la valeur ou l'amplitude) du plateau de fibrine, et la concentration en fibrinogène,
la courbe étalon ayant été établie en utilisant des échantillons sanguins dont la concentration en fibrinogène a été déterminée et dont la valeur de la propriété (Vp$_{(TA)}$) a été déterminée par les étapes a)-d) de la méthode selon l'invention.

Le taux des D-dimères ajustés en fonction de l'inflammation, Ddi$_{E/I}$, est ainsi exprimé en unités équivalentes au fibrinogène initial (FEU).

*Etape e'2).* L'étape e'2) consiste à calculer le taux de D-dimères qui résultent de la dégradation de la fibrine intravasculaire (R) en corrigeant le taux Ddi$_{E/I}$ obtenu en e'1) pour les taux de D-dimères faibles (< 4 $\mu$g/mL). Cette correction des D-dimères faibles en fonction du fibrinogène a pour but d'amener le ratio Ddi$_{E/I}$ au seuil de 0,5 $\mu$g/mL unités FEU (unités équivalentes au fibrinogène).

Le taux R est calculé en utilisant l'équation:

$$R = Ddi_{E/I} + [0,5 - F_{Ddi-E}]$$

où F$_{Ddi-E}$ est un facteur de correction des taux de D-dimères faibles (< 4 $\mu$g/mL) dont la valeur correspond à la valeur du facteur de correction, F, pour le taux de D-dimères de l'échantillon (Ddi$_E$) sur la courbe étalon d'équation:

$$y = a\,x^2 + b\,x + c$$

où y est le facteur de correction, F,
x est le taux de D-dimères,
a, b et c sont des constantes de l'équation polynomiale qui relie le facteur de correction, et le taux de D-dimères,
la courbe étalon ayant été établie pour des échantillons sanguins dont le taux de D-dimères a été déterminé et pour lesquels le facteur de correction a été déterminé de façon empirique pour que le ratio Ddi$_{E/I}$ soit ramené au seuil de 0,5 $\mu$g/mL unités FEU (unités équivalentes au fibrinogène).

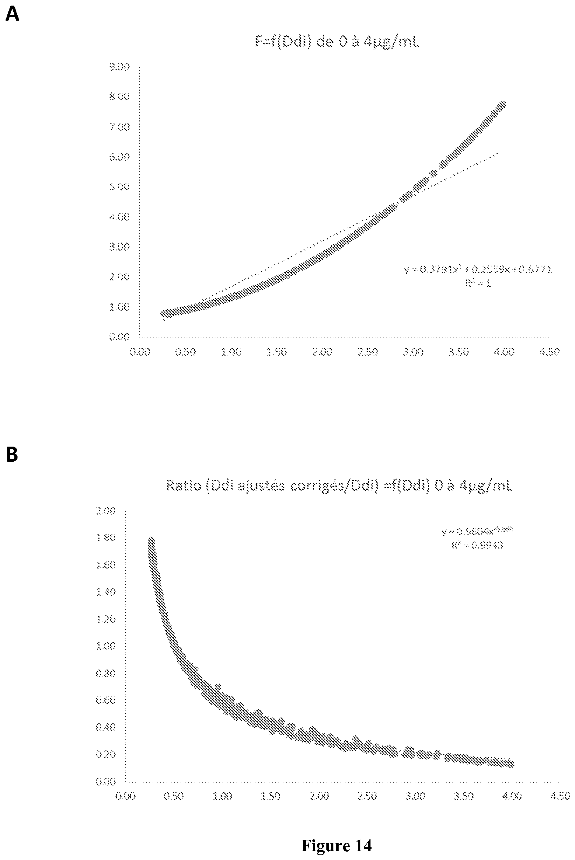

La courbe étalon qui a été établie par les présents Inventeurs est présentée en Figure 14(**B**).

*Classement de l'échantillon.* L'étape e') comprend de plus une étape consistant à classer l'échantillon en fonction de l'inflammation. Plus spécifiquement, cette étape consiste à calculer le ratio 1/[Fib]$_{(Vp(TA)}$, où [Fib]$_{(Vp(TA)}$ est la concentration en fibrinogène déduite en e'1), et classer l'échantillon du patient:

- dans le groupe I (patient sans inflammation) si le ratio 1/[Fib]$_{(Vp(TA)}$ > 0,20, (c'est-à-dire si [Fib]$_{(Vp(TA)}$ < 5 g/L), ou
- dans le groupe II (patient avec inflammation) si le ratio 1/[Fib]$_{(Vp(TA)}$ ≤ 0,20, (c'est-à-dire si [Fib]$_{(Vp(TA)}$ ≥ 5 g/L).

*Etape f')*

L'étape f') de la deuxième version de la méthode selon l'invention consiste à comparer le taux de D-dimères résultant de la dégradation de la fibrine R obtenu en e'), par rapport à un seuil.

Un taux de D-dimères résultant de la dégradation de la fibrine, R, obtenu en e') inférieur au seuil (préférablement au seuil de 0,5 $\mu$g/mL) permet d'exclure une thrombose chez le patient. Un taux de D-dimères résultant de la dégradation de la fibrine, R, obtenu en e') supérieur au seuil (préférablement au seuil de 0,5 $\mu$g/mL) est indicatif de la possibilité d'une thrombose chez le patient.

*Etape g')*

L'étape g') repose sur le fait que dans l'hyper fibrinolyse, le fibrinogène est dégradé par la plasmine générée en large excès. Il apparaît dans la circulation, à la fois, des produits de dégradation de la fibrine (PDFn) dont les D-dimères et des produits de dégradation du fibrinogène (PDFg), comme le montre la Figure 2. Ainsi, des taux élevés de D-dimères sont générés dans le cancer et dans les états d'activation importante de la coagulation, en association avec des taux importants de PDFg. Ainsi on peut mesurer indifféremment le taux des PDFg générés par l'hyper fibrinolyse ou le taux des D-dimères générés par l'hyper fibrinolyse.

La méthode selon l'invention comprend donc une étape g') de calcul du taux de D-dimères générés par l'hyper fibrinolyse présente dans les états d'activation de la coagulation en utilisant le taux de D-dimères dans l'échantillon ($Ddi_E$) et le taux de D-dimères résultant de la dégradation de la fibrine (R) obtenu en e') ou en utilisant le taux de D-dimères ajustés en fonction de l'inflammation, $Ddi_{E/I}$, obtenu en e'1) et le taux R obtenu en e').

Plus spécifiquement, l'étape g') consiste à:

g'1) calculer le taux de D-dimères générés par l'hyper fibrinolyse, $Ddi_{HF}$ ou $(R/Ddi_E)_{patient}$, comme le ratio entre le taux de D-dimères ajusté et corrigé (R) déterminé à l'étape e') et le taux de D-dimères dans l'échantillon ($Ddi_E$) en utilisant l'équation:

$$Ddi_{HF} = (R/Ddi_E)_{patient} = \frac{R}{Ddi_E}$$

g'2) calculer le taux de D-dimères générés par l'hyper fibrinolyse, $Ddi_{FF}$ ou $(R/Ddi_{E/I})_{patient}$, comme le ratio entre le taux de D-dimères ajusté et corrigé (R) déterminé à l'étape e') et le taux de D-dimères ajustés en fonction de l'inflammation, $Ddi_{E/I}$, obtenu en e'1) en utilisant l'équation:

$$Ddi_{HF} = (R/Ddi_{E/I})_{patient} = \frac{R}{Ddi_{E/I}}$$

*Etape h')*

Enfin, l'étape h') de la deuxième version de la méthode selon l'invention comprend la comparaison du taux de D-dimères générés par l'hyper fibrinolyse ($Ddi_{HF}$) obtenu en g') par rapport à un seuil, et la comparaison du ratio TA/TFF par rapport à un seuil. Le remplacement de TFF, utilisé à l'étape h), par le ratio TA/TFF, utilisé à l'étape h'), permet de différencier une thrombose dont le temps d'atteinte du plateau de la polymérisation du caillot de fibrine est plus rapide, d'un état d'activation de la coagulation et d'une thrombophilie.

Plus spécifiquement, l'étape h') comprend tout d'abord des étapes consistant à :

h'1) déterminer pour le taux $Ddi_E$ de l'échantillon, la valeur du ratio $(R/Ddi_E)_{étalon}$ sur la courbe étalon d'équation:

$$y = a\ x^{-b}$$

où x est le taux de D-dimères,
y est le ratio entre le taux de D-dimères qui résultent de la dégradation de la fibrine intravasculaire et le taux de D-dimères, $(R/Ddi_E)$,
a, et b sont les constantes de l'équation qui relie le ratio $R/Ddi_E$, et le taux de D-dimères, la courbe étalon ayant été établie:

- en utilisant, si l'échantillon du patient a été classé dans le groupe I: des échantillons sanguins classés dans le groupe I par l'étape e') de la méthode de l'invention et dont le taux de D-dimères est connu ou a été déterminé et le taux R a été obtenu par les étapes a)-e2) de la méthode selon l'invention; et
- en utilisant, si l'échantillon du patient a été classé dans le groupe II: des échantillons sanguins classés dans le groupe II par l'étape e') de la méthode de l'invention et dont le taux de D-dimères est connu ou a été déterminé et le taux R a été obtenu par les étapes a)-e'2) de la méthode selon l'invention;

h"1) comparer la valeur du taux de D-dimères générés par l'hyper fibrinolyse $(R/Ddi_E)_{patient}$ obtenu en g'1) avec la valeur du ratio $(R/Ddi_E)_{étalon}$ obtenu en h'1), où $(R/Ddi_E)_{patient}$ inférieur au ratio $(R/Ddi_E)_{étalon}$ permet d'exclure la thrombose chez le patient, et où $(R/Ddi_E)_{patient}$ supérieur ou égal au ratio $(R/Ddi_E)_{étalon}$ est indicatif d'une possibilité de thrombose chez le patient;

h'2) déterminer pour le taux $Ddi_E$ de l'échantillon, la valeur du ratio $(R/Ddi_{E/I})_{étalon}$ sur la courbe étalon d'équation:

$$y = a\ x^{-b}$$

où x est le taux de D-dimères,
y est le ratio entre le taux de D-dimères qui résultent de la dégradation de la fibrine intravasculaire et le taux de D-dimères ajustés en fonction de l'inflammation, $(R/Ddi_{E/I})$, a, et b sont les constantes de l'équation qui relie le ratio $R/Ddi_{E/I}$, et le taux de D-dimères, la courbe étalon ayant été établie:

- en utilisant, si l'échantillon du patient a été classé dans le groupe I: des échantillons sanguins classés dans le groupe I par l'étape e') de la méthode de l'invention et dont le taux de D-dimères est connu ou a été déterminé, le taux $Ddi_{E/I}$ a été obtenu par les étapes a)-e' 1) de la méthode selon l'invention, et le taux R a été obtenu par les étapes a)-e'2) de la méthode selon l'invention; et
- en utilisant, si l'échantillon du patient a été classé dans le groupe II: des échantillons sanguins classés dans le groupe II par l'étape e') de la méthode de l'invention et dont le taux de D-dimères est connu ou a été déterminé, le taux $Ddi_{E/I}$ a été obtenu par les étapes a)-e' 1) de la méthode selon l'invention, et le taux R a été obtenu par les étapes a)-e'2) de la méthode selon l'invention;

h"2) comparer la valeur du taux de D-dimères générés par l'hyper fibrinolyse $(R/Ddi_{E/I})_{patient}$ obtenu en g'2) avec la valeur du ratio $(R/Ddi_{E/I})_{étalon}$ obtenu en h'2), où $(R/Ddi_{E/I})_{patient}$ inférieur au ratio $(R/Ddi_{E/I})_{étalon}$ permet d'exclure la thrombose chez le patient, et où $(R/Ddi_{E/I})_{patient}$ supérieur ou égal au ratio $(R/Ddi_{E/I})_{étalon}$ est indicatif d'une possibilité de thrombose chez le patient.

L'étape h') comprend de plus une étape qui consiste à:

h'3) calculer le ratio TA/TFF où TA est le temps d'atteinte du plateau de polymérisation de la fibrine déterminé à l'étape d) et TFF est le temps de formation du caillot de fibrine déterminé à l'étape d); et
h"3) si l'échantillon du patient a été classé dans le groupe I: comparer le ratio TA/TFF par rapport à un premier seuil, notamment à un premier seuil de 1,75 pour un Temps Témoin de 135 secondes, où:

un ratio TA/TFF supérieur au premier seuil permet d'exclure la thrombose et de diagnostiquer une thrombophilie ou un état d'activation de la coagulation chez le patient classé dans le groupe I, et
un ratio TA/TFF inférieur ou égal au premier seuil est indicatif d'une thrombose chez le patient classé dans le groupe I ;

si l'échantillon du patient a été classé dans le groupe II: comparer le ratio TA/TFF par rapport à un deuxième seuil, notamment à un deuxième seuil de 1,65 pour un Temps Témoin de 135 secondes, où:

un ratio TA/TFF supérieur au deuxième seuil permet d'exclure la thrombose et de diagnostiquer une thrombophilie ou d'un état d'activation de la coagulation chez le patient classé dans le groupe II, et
un ratio TA/TFF inférieur ou égal au deuxième seuil est indicatif d'une thrombose chez le patient classé dans le groupe II,

où le Temps Témoin est le temps moyen de formation du caillot de fibrine d'échantillons de sujets sains ne présentant pas de suspicion de thrombose, mesuré selon les étapes a)-d).

Sur la base des échantillons testés, les Inventeurs ont déterminé un premier seuil de 1,75 pour les échantillons classés dans le groupe I à l'étape e') et un deuxième seuil de 1,55 pour les échantillons classés dans le groupe II à l'étape e'). L'homme du métier comprendra que ces seuils sont pré-déterminés et qu'ils peuvent varier en fonction du nombre total d'échantillons testés ou des lots de réactifs utilisés.

Il est possible de distinguer une thrombose (pour laquelle le ratio TA/TFF est inférieur ou égal au seuil) d'un état d'activation de la coagulation et d'une thrombose avec thrombophilie (pour lesquels le ratio TA/TFF est supérieur au seuil) en considérant le temps de formation du caillot de fibrine, TFF. En effet, le TFF est toujours court ($\leq$ [Temps Témoin - 1 écart type], par exemple < 120 secondes pour un Temps Témoin à 135 secondes) dans la thrombophilie, alors qu'il est égal au Temps Témoin dans les états d'activation de la coagulation.

De préférence, des taux élevés de D-dimères spécifiques de la thrombose veineuse obtenus à la fin de la méthode sont représentatifs de l'étendue de l'embolie pulmonaire (EP) ou de la thrombose veineuse profonde (TVP). Ainsi, dans le cas où une thrombose est diagnostiquée chez le patient par une méthode selon l'invention, le taux de D-dimères, R, obtenu à l'étape e'), ramené à une échelle utilisée en clinique, c'est-à-dire par rapport au seuil de 0,5 $\mu$g/mL, est proportionnel à l'étendue de l'EP ou de la TVP, donc à la gravité de la maladie. En effet, le diagnostic de gravité est habituellement déterminé à l'imagerie par le nombre et le type d'artères pulmonaires atteintes.

Plus spécifiquement, dans le cas où une thrombose est diagnostiquée chez le patient à l'étape h"3) d'une méthode selon l'invention, le taux de D-dimères spécifiques de la maladie thromboembolique veineuse ($Ddi_{MTEV}$) est le taux de D-dimères, R, obtenu à l'étape e').

Dans le cas où une thrombose est exclue aux étapes h"1), h"2) et h"3) mais que le taux R obtenu à l'étape e') est supérieur au seuil, préférablement au seuil de 0,5 $\mu$g/mL, la méthode comprend en outre une étape consistant à calculer le taux de D-dimères spécifiques de la maladie thromboembolique veineuse ($Ddi_{MTEV}$) en utilisant l'équation suivante:

$$Ddi_{MTEV} = 0,5 \text{ x } \frac{R}{Ddi_E}$$

où $Ddi_E$ est le taux de D-dimères dans l'échantillon. Ce calcul permet de ramener le taux de D-dimères spécifiques de la maladie thromboembolique veineuse à un taux inférieur au seuil.

Les taux de D-dimères spécifiques de la thrombose veineuse ainsi fournis en fin de méthode sont alors représentatifs de l'étendue de l'embolie pulmonaire thrombose veineuse profonde.

## C. Automatisation

La mesure de densité optique de l'étape c) peut être effectuée par n'importe quelle méthode appropriée connue dans l'art. Par exemple, la mesure de DO de l'étape c) peut être effectuée en utilisant tout instrument existant approprié, et notamment un turbidimètre ou un spectrophotomètre. En particulier, dans certains modes de réalisation, au moins les étapes c) et d) de la méthode selon l'invention sont réalisées sur un automate de diagnostic de routine, de préférence un analyseur de coagulation. Préférentiellement, toutes les étapes de la méthode selon l'invention sont mises en œuvre sur un tel automate. Plus préférentiellement, cette mesure est effectuée en même temps que le dosage habituel des D-dimères sur l'automate de routine. Par exemple, comme décrit dans les Exemples 9 et 10, la méthode selon l'invention est mise en œuvre sur les automates STA-R® Max ou STA-R® Evolution Expert Series du groupe Stago.

Un tel automate permet de charger simultanément les échantillons, d'effectuer les mélanges et l'incubation, de mesurer les densités optiques à une longueur d'onde et de déterminer le profil de formation du caillot obtenu avec un seul échantillon en situation d'urgence, ou de plusieurs échantillons en simultané. Le tout est réalisé en moins de 10 minutes. Cela en fait une méthode rapide, fiable, et reproductible chez un patient.

Une méthode selon l'invention peut également être utilisée sur un dispositif de biologie délocalisée ou un dispositif portable au lit du patient. Dans certains modes de réalisation préférés, le dispositif de biologie délocalisée ou le dispositif portable utilisé est basé sur une méthode physique, de préférence une méthode optique, comme une méthode de thromboélastographie ou de rhéométrie ou une méthode d'analyse d'images. Préférentiellement, toutes les étapes de cette variante de la méthode selon l'invention sont mises en œuvre sur un tel dispositif, pour déterminer le profil de formation du caillot de fibrine obtenu avec un seul échantillon en situation d'urgence. Le tout est réalisé en moins de 5 minutes à partir du même tube de sang que pour le dosage habituel des D-dimères.

**II - Méthodes *in vitro* de Diagnostic**

La méthode de dosage des D-dimères spécifiques de la maladie thromboembolique veineuse selon l'invention permet, à partir d'un échantillon sanguin (par exemple de plasma ou de sang total) provenant de patients, de diagnostiquer la maladie thromboembolique veineuse chez ces patients, quel que soit leur âge et quelle que soit leur condition pathologique ou physiologique sous-jacente. Comme le montrent les résultats obtenus par les présents Inventeurs, une méthode selon l'invention permet de diagnostiquer 100% des patients atteints de la maladie thromboembolique sans faux négatifs, et permet d'exclure davantage de patients qu'avec la méthode habituelle de dosage des D-dimères.

Ainsi, la présente invention concerne une méthode de diagnostic *in vitro* de la maladie thromboembolique veineuse (MTEV) chez un patient comprenant des étapes consistant à:

- effectuer un dosage quantitatif des D-dimères spécifiques de MTEV dans un échantillon sanguin du patient selon la première ou la deuxième version de la méthode décrite ici pour obtenir un taux de D-dimères spécifiques de MTEV dans l'échantillon, et
- fournir un diagnostic concernant le patient.

Généralement, on choisira préférablement la deuxième version de la méthode de dosage des D-dimères spécifiques de MTEV selon l'invention dans le cas où le patient à tester est une personne âgée, un patient atteint de cancer, un patient atteint d'une infection ou un patient atteint d'une thrombophilie. Dans les autres cas, la première version de la méthode selon l'invention peut être utilisée.

Si la première version de la méthode est utilisée, le diagnostic concernant le patient peut être (i) exclusion de la thrombose, (ii) état d'activation aigu de la coagulation, ou (iii) thrombose. Si la deuxième version de la méthode est utilisée, le diagnostic concernant le patient peut être (i) exclusion de la thrombose, (ii) état d'activation aigu de la coagulation, (iii) thrombose chez un patient avec thrombophilie, ou (iv) thrombose, y compris thrombose de faible gravité ou thrombose en cas d'infarctus pulmonaire.

A moins qu'ils ne soient définis d'une autre manière, tous les termes techniques et scientifiques utilisés dans la description ont la même signification que celle couramment comprise par un spécialiste ordinaire du domaine auquel appartient cette invention. De même, toutes les publications, demandes de brevet, tous les brevets et toutes autres références mentionnés ici sont incorporés par référence.

**Exemples**

Les exemples suivants décrivent certains modes de réalisation de la présente invention. Cependant, il est entendu que les exemples ne sont présentés qu'à titre illustratif seulement et ne limitent en aucun cas la portée de l'invention.

***Légendes des Figures***

**Figure 1:** Schéma de l'activation de la coagulation. FpA-FpB = fibrinopeptides A et B; TAT = complexe Thrombine-Anti-thrombine; et F1+2 = Fragments prothrombiniques.

**Figure 2:** Dynamique de la formation des D-dimères par l'hyper coagulation, l'inflammation, et l'hyper fibrinolyse dans les états d'activation de la coagulation et la maladie thromboembolique veineuse (MTEV).

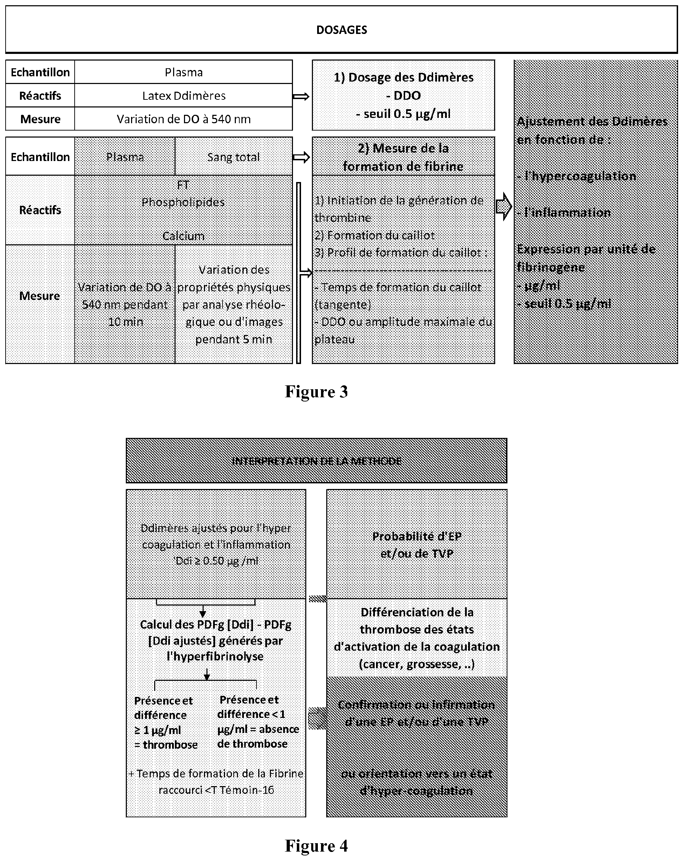

**Figure 3:** Principe de la méthode de dosage des D-dimères spécifiques de la MTEV selon l'invention, dans un échantillon de plasma ou de sang total, comprenant le dosage habituel des D-dimères de l'échantillon et la mesure dynamique de la formation de fibrine de cet échantillon.

**Figure 4:** Interprétation de la méthode de dosage des D-dimères spécifiques de la MTEV, dans un échantillon biologique, sur la base des D-dimères ajustés en fonction des D-dimères générés par l'hyper coagulation, l'inflammation, et l'hyper fibrinolyse dans ledit échantillon du patient selon la première version de la méthode.

**Figure 5:** Profil de formation du caillot de fibrine (étape d), (**A**) dans le cas d'un plasma de patient normal (PN) dont le fibrinogène est de 3,5 g/L et d'un échantillon de plasma de patient avec un état d'hyper coagulation (P Hyper), dont le fibrinogène est de 3,0 g/L, par la mesure de la densité optique (DO) en fonction du temps, selon la méthode de l'invention; et (**B**) dans le cas d'un échantillon de sang total de patient normal (PN) dont le taux de fibrinogène est de 2,0 g/L et d'un échantillon de sang total de patient hyper-fibrinogénémique (P Hyperfib) dont le taux de fibrinogène est de 5,0 g/L, par la mesure thromboelastométrique de l'amplitude au temps d'atteinte du plateau en fonction du temps, selon une variante de la méthode de l'invention.

**Figure 6:** Variation de la densité optique (DDO) au temps d'atteinte du plateau de polymérisation de la fibrine par la méthode selon l'invention, pour les 51 échantillons de plasma de patients avec suspicion de thrombose, en fonction de l'inflammation, représentée (**A**) par le taux de Protéine C réactive (CRP); et (**B**) par le taux de fibrinogène (Fib).

**Figure 7:** Temps d'atteinte du plateau en fonction du temps de formation de la fibrine, par la méthode selon l'invention (**A**) pour 219 échantillons de patients avec ou sans cancer connu, avec suspicion de thrombose); et (**B**) pour 34 échantillons de patients atteints de cancer, avec suspicion de thrombose.

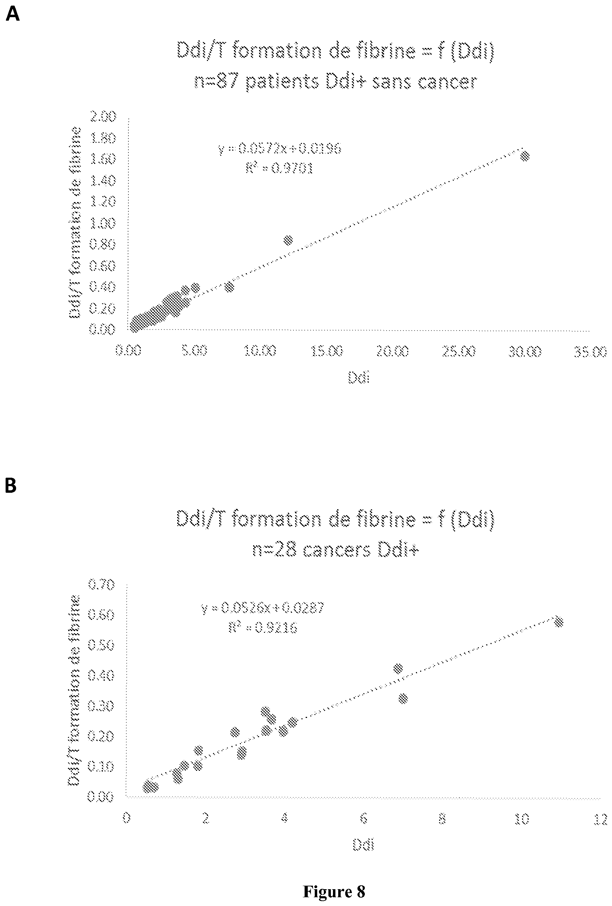

**Figure 8:** Taux de D-dimères générés par l'hyper coagulation en fonction des D-dimères de l'échantillon (étape e)) (**A**) pour 87 échantillons de patients avec suspicion de thrombose; et (**B**) pour 28 échantillons de patients avec un cancer actif.

**Figure 9:** Variations de densité optique (DDO) au temps d'atteinte du plateau de polymérisation de la fibrine mesurées selon la méthode de l'invention, en fonction du taux de fibrinogène déterminé par la méthode de référence (Clauss) (**A**) dans 219 échantillons de patients avec ou sans cancer, avec suspicion de thrombose; et (**B**) dans 23 échantillons de patients atteints de cancer actif, avec suspicion de thrombose. (**C**) Amplitude au temps d'atteinte du plateau de polymérisation de la fibrine mesurée selon la variante de la méthode selon l'invention dans 7 échantillons de sang total de patients avec suspicion de thrombose en fonction du taux de fibrinogène déterminé en plasma par la méthode de Clauss.

**Figure 10:** Corrélation dans les échantillons de 14 patients avec et sans thrombose veineuse, entre le taux de produits de dégradation du fibrinogène (PDFg) mesurés par la méthode latex STA Liatest® FDP (Stago) et le taux (**A**) des D-dimères habituels (méthode Stago); (**B**) des D-dimères (méthode Stago) ajustés en fonction des D-dimères générés par l'hyper coagulation et l'inflammation, selon la méthode de l'invention effectuée sur plasma; et (**C**) des D-dimères (méthode Stago) ajustés en fonction des D-dimères générés par l'hyper coagulation et l'inflammation, selon une variante de la méthode de l'invention effectuée sur des échantillons de sang total.

**Figure 11:** Corrélation dans les échantillons de 14 patients avec et sans thrombose veineuse, entre le taux de produits de dégradation du fibrinogène (PDFg) mesurés par méthode latex STA Liatest® FDP (Stago) et le taux (**A**) des D-dimères (méthode Instrumentation Laboratory IL); et (**B**) des D-dimères (méthode IL) ajustés en fonction des D-dimères générés par l'hyper coagulation et l'inflammation, selon la méthode de l'invention effectuée sur des échantillons de plasma.

**Figure 12:** Comparaison de l'ajustement des D-dimères (méthode Stago), par la méthode de l'invention et par le ratio [D-dimères/Fibrinogène] (**A**) dans les plasmas de 215 patients sans thrombose veineuse à l'imagerie; et (**B**) dans les plasmas de 21 patients avec EP et/ou TVP à l'imagerie. (I: Inflammation, F: hyper Fibrinolyse, Ddi: D-dimères, Fib: fibrinogène).

**Figure 13:** Comparaison de l'ajustement des D-dimères (méthode IL), par la méthode de l'invention et par le ratio [D-dimères/Fibrinogène] (**A**) dans les plasmas de 199 patients sans thrombose veineuse à l'imagerie; et (**B**) dans les plasmas de 15 patients avec EP et/ou TVP à l'imagerie. (I: Inflammation, F: hyper Fibrinolyse, Ddi: D-dimères, Fib: fibrinogène).

**Figure 14.** Corrélation entre le Facteur de correction $F_{Ddi-E}$ des taux de D-dimères faibles (< 4 $\mu$g/mL) (**A**) et le taux des D-dimères, R, résultant de la dégradation de la fibrine intravasculaire (**B**) obtenus après ajustement en fonction de l'inflammation ($Ddi_{E/I}$) et correction pour les D-dimères faibles (<4 $\mu$g/mL) par l'équation $R=Ddi_{E/I}+[0,5-F_{Ddi-E}]$ en fonction du taux de D-dimères de l'échantillon ($Ddi_E$), selon la méthode de l'invention effectuée sur des échantillons de plasma.

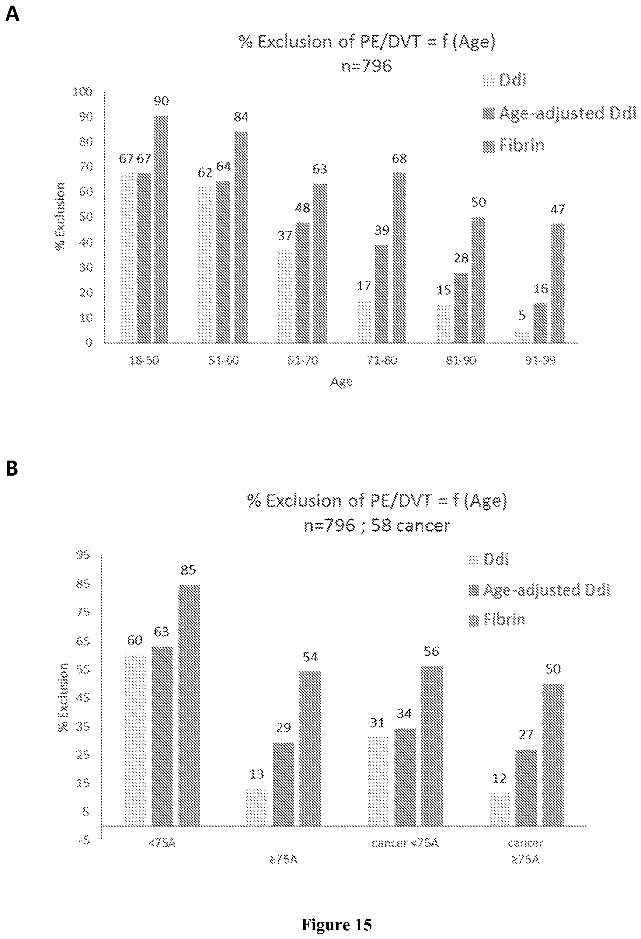

**Figure 15.** Comparaison du pourcentage d'exclusion de thrombose en fonction de l'âge, par i) le dosage des D-dimères, ii) le dosage des D-dimères ajustés à l'âge, iii) la méthode de l'invention (**A**) pour 796 patients avec suspicion de thrombose veineuse; et (**B**) pour les 58 patients atteints de cancer parmi les 796 patients avec suspicion de thrombose veineuse.

**Protocoles Expérimentaux**

Les protocoles utilisés dans les exemples sont les suivants:

*Protocole A*

Le protocole A est une méthode réalisée sur l'analyseur de coagulation automatique STAR® Evolution Expert Series (Stago).
Dans 8 cuvettes de l'automate STAR® Evolution, sont ajoutés simultanément par l'instrument (étape a)) :

- 200 μL d'échantillon de plasma pur, obtenu après centrifugation du tube citraté destiné au dosage habituel des D-dimères, pendant 15 minutes, à une vitesse de 2500 g,
- 50 μL d'un mélange de facteur tissulaire et de phospholipides [FT + PL], lyophilisé repris par de l'eau; à des concentrations finales de facteur tissulaire (FT) de 2 à 5 pM et de phospholipides (PL) de 4 μM,

L'automate agite par le bras, et procède à une incubation pendant 300 secondes à 37°C. Il ajoute ensuite 50 μL de CaCl$_2$ à 16,7 mM final, et agite par l'aiguille du réactif déclenchant (étape b)). L'automate mesure alors la densité optique (DO) à 540 nm en fonction du temps pendant 10 minutes (étape c)) et trace la courbe de formation de la fibrine.
L'algorithme de « post-traitement » de la mesure permet le calcul du temps de formation de la fibrine (TFF) à partir de la tangente de la courbe, et de la variation de DO (DDO) au temps d'atteinte (TA) du plateau, à partir de la DO au temps TA du plateau et de la DO au temps T0 à 10 secondes, comme décrit en Figure 5a (étape d)). Le taux de D-dimères ajustés en fonction des D-dimères générés par l'hyper coagulation est calculé par la formule [(X) x (TFF/Temps Témoin)], où X est le taux de D-dimères mesuré, comme le montrent la Figure 8 et l'Exemple 3.
Le taux de D-dimères ajustés en fonction des D-dimères générés par l'inflammation, est calculé par le ratio [D-dimères ajustés/inflammation]. Le niveau d'inflammation est déterminé à partir de l'équation y = a ln(x) - b, où y est la DDO, x est la concentration de fibrinogène, a et b des constantes de l'équation logarithmique qui relie le niveau du plateau de fibrine, et la concentration de fibrinogène, comme le montrent la Figure 9 et l'Exemple 4. Les D-dimères sont ainsi exprimés en unités équivalentes au fibrinogène (FEU) (étape e)).
Le temps d'atteinte de la polymérisation du caillot est calculé par le ratio TA/TFF, où TA est le temps d'atteinte du plateau de polymérisation de la fibrine déterminé à l'étape d) et TFF est le temps de formation du caillot de fibrine déterminé à l'étape d).

*Protocole B*

Le protocole B est une méthode réalisée sur l'analyseur de coagulation automatique Rotem® delta.
Dans une cuvette de l'appareil préalablement préchauffée, contenant 40 μL d'un mélange de facteur tissulaire et de phospholipides [FT + PL], lyophilisé repris par de l'eau; à des concentrations finales de facteur tissulaire (FT) de 2 à 5 pM et de phospholipides (PL) de 4 μM, et de CaCl$_2$ à 16,7 mM final, sont ajoutés 300 μL de sang total non dilué (étapes a) et b) de la variante de la méthode selon l'invention), prélevé à partir du tube citraté destiné au dosage habituel des D-dimères, préchauffé à 37°C. La mesure thromboélastométrique est démarrée (étape c) de la variante de la méthode selon l'invention) puis 300 μL du mélange sang et réactif sont analysés par l'appareil qui enregistre en continu pendant 30 minutes l'activation de la coagulation, la formation, la polymérisation, et la stabilité du caillot de fibrine, et fournit les paramètres habituels (CT, CFT, angle α, A5, A10, MCF, MCF-t).
Le temps de formation de la fibrine (TFF) à partir de la tangente et l'amplitude au temps d'atteinte (TA) du plateau de la courbe de formation de fibrine, sont calculés à partir de l'évolution temporelle de l'amplitude en fonction du temps, comme décrit en Figure 5(**B**) (étape d) de la variante de la méthode selon l'invention). Le taux de D-dimères ajustés en fonction des D-dimères générés par l'hyper coagulation est calculé par la formule [(X) x (TFF/Temps Témoin)], où X est le taux de D-dimères mesuré, comme le montre l'Exemple 3.
Le taux de D-dimères ajustés en fonction des D-dimères générés par l'inflammation, est calculé par le ratio [D-dimères ajustés/niveau de l'inflammation]. Ce niveau est déterminé à partir de l'équation linéaire qui relie l'amplitude au temps d'atteinte du plateau (mm), et la concentration de fibrinogène, comme le montrent la Figure 9 et l'Exemple 4. Les D-dimères sont ainsi exprimés en unités équivalentes au fibrinogène initial (FEU) (étape e) de la variante de la méthode selon l'invention).

*Protocole C*

Le protocole C est une méthode réalisée sur l'analyseur de coagulation automatique STAR® Evolution Expert Series (Stago).

Dans les cuvettes de l'automate STAR® Evolution, sont ajoutés simultanément par l'instrument (étape a)):

- 200 µL d'échantillon de chacun des plasmas contrôle négatif ou contrôle positif, lyophilisé et reconstitué par de l'eau,
- 50 µL d'un mélange de facteur tissulaire et de phospholipides [FT + PL], lyophilisé repris par de l'eau; à des concentrations finales de facteur tissulaire (FT) de 2 pM et de phospholipides (PL) de 4 µM.

L'automate agite par le bras, procède à une incubation pendant 300 secondes à 37°C puis ajoute 50 µL de CaCl$_2$ à 16,7 mM final, et agite par l'aiguille du réactif déclenchant (étape b)). L'automate mesure alors la densité optique (DO) à 540 nm en fonction du temps pendant 10 minutes (étape c)) et la courbe de formation de la fibrine est tracée pour chacun des plasmas contrôles.

L'algorithme de « post-traitement » de la mesure permet le calcul du temps de formation de la fibrine (TFF) à partir de la tangente de la courbe, et de la variation de DO (DDO) au temps d'atteinte (TA) du plateau à partir de la DO au temps T du plateau et de la DO au temps T0 à 10 secondes pour chacun des plasmas contrôles, comme décrit en Figure 5(**A**) (étape d)).

Le taux de D-dimères ajustés en fonction des D-dimères générés par l'hyper coagulation est calculé par la formule [(X) x (TFF/Temps Témoin)], où X est le taux de D-dimères mesuré, comme le montrent la Figure 8 et l'Exemple 3.

Le taux de D-dimères ajustés en fonction des D-dimères générés par l'inflammation, est calculé par le ratio [D-dimères ajustés/niveau de l'inflammation]. Ce niveau est déterminé à partir de l'équation $y = a \ln(x) - b$, où y est la DDO, x est la concentration de fibrinogène, a et b des constantes de l'équation logarithmique qui relient le niveau du plateau de fibrine, et la concentration de fibrinogène, comme montré en Figures 9(**A**) et (**B**) et en Exemple 4. Les D-dimères sont ainsi exprimés en unités équivalentes au fibrinogène (FEU) (étape e)).

**Exemple 1: Temps de formation de la fibrine de différents plasmas de patients normaux et de patients avec suspicion de thrombose (avec ou sans thrombose, et avec ou sans cancer)**

Le protocole utilisé dans cet exemple est le Protocole A. L'étape d) de la méthode utilisée dans cet exemple est indifféremment celle de la première ou la deuxième version de la méthode.

Parmi les échantillons de patients avec suspicion d'EP et/ou de TVP testés, les échantillons de patients avec un état d'activation de la coagulation sont différenciés des patients normaux (sujets sains normaux, ne présentant pas de suspicion de thrombose), de ceux avec une exclusion de thrombose, et de ceux avec un diagnostic positif de thrombose veineuse à l'imagerie, sur le temps de formation de la fibrine (TFF), représentatif de l'hyper coagulation, sur le temps d'atteinte (TA) du plateau, et sur le temps d'atteinte de la polymérisation du caillot (ratio TA/TFF), comme montré dans la Figure 5 et le Tableau 1 ci-après.

Le temps de formation de la fibrine (TFF) est plus court pour les patients présentant un état d'hyper coagulation; mais de façon surprenante, il n'y a pas d'état d'hyper coagulation chez les patients avec un diagnostic d'EP et/ou de TVP, contrairement aux patients avec une exclusion de thrombose, y compris dans le cancer. Ceci est mis à profit dans la première version de la méthode l'invention pour discriminer les patients avec et sans thrombose.

Le temps d'atteinte du plateau ne dépasse pas 8 minutes, tous patients confondus, excepté pour les patients sous traitement anticoagulant, pour lesquels le rendu du résultat est limité au temps de formation de fibrine.

Le temps d'atteinte de la polymérisation du caillot est plus court (ratio TA/TFF inférieur) chez les patients avec un diagnostic d'EP et/ou de TVP, que chez les patients normaux, ou avec un état d'activation de la coagulation, ou avec une exclusion de thrombose, y compris dans le cancer. Ceci est mis à profit dans la deuxième version de la méthode de l'invention pour discriminer les patients avec et sans thrombose.

**Tableau 1.** Discrimination des plasmas de patients avec un état d'hyper coagulation parmi les patients normaux et les patients avec suspicion de thrombose, qu'ils aient ou non une thrombose ou un cancer, sur le temps de formation de la fibrine (TFF) et sur le temps d'atteinte (TA) du plateau et sur le temps d'atteinte de la polymérisation du caillot (TA/TFF).

| Plasmas de patients | Diagnostic D-dimères (seuil 0,5 µg/mL) et/ou imagerie | | N | TFF (sec) | | TA (sec) | | TA/TFF |
|---|---|---|---|---|---|---|---|---|
| | | | | Moy | Gamme | Moy | Gamme | Moy |
| Normaux | Ddi négatifs | Négatif | 150 | 133 | 88-206 | 1.82 | 172-324 | 1,82 |

EP 3 417 068 B1

(suite)

| Plasmas de patients | Diagnostic D-dimères (seuil 0,5 μg/mL) et/ou imagerie | | N | TFF (sec) | | TA (sec) | | TA/TFF |
|---|---|---|---|---|---|---|---|---|
| | | | | Moy | Gamme | Moy | Gamme | Moy |
| Avec suspicion de thrombose sans cancer | Ddi négatifs | Négatif | 127 | 141 | 84-304 | 1.62 | 154-440 | 1,62 |
| | Ddi positifs sans thrombose | Négatif | 88 | 154 | 88-258 | 1.55 | 154-384 | 1,55 |
| | Ddi positifs **avec thrombose** | Positif EP et/ou TVP | 21 | **166** | **116-218** | **1.45** | 176-298 | **1,45** |
| Avec suspicion de thrombose avec cancer | Imagerie sans thrombose | Négatif | 16 | 155 | 116-210 | 1.56 | 190-304 | 1,56 |
| | Imagerie **avec thrombose** | Positif EP et/ou TVP | 6 | **176** | **126-212** | **1.46** | **204-298** | **1,46** |
| Sous anticoagulants | | | 10 | 313 | 150-694 | 447 | 220-1122 | / |

**Exemple 2: Génération des D-dimères par l'hyper coagulation, dans différents plasmas de patients avec suspicion de thrombose (avec ou sans thrombose, et avec ou sans cancer)**

Le protocole utilisé dans cet exemple est le Protocole A. L'étape e) de la méthode utilisée dans cet exemple est celle de la première version de la méthode.

Parmi les échantillons de patients avec suspicion d'EP et/ou de TVP testés, les D-dimères générés en fonction du temps de formation de la fibrine sont bien corrélés aux D-dimères initiaux de l'échantillon, que ce soit pour les patients avec un diagnostic positif de thrombose à l'imagerie ou avec une exclusion de thrombose, avec ou sans cancer, comme le montrent les Figures 8 (A, B).

La fonction linéaire est mise à profit pour ajuster les D-dimères de l'échantillon en fonction des D-dimères générés par l'hyper coagulation, par le calcul des D-dimères ajustés Y, à partir du taux de D-dimères de l'échantillon X, par la formule suivante :

$$Y = X \ \text{x} \ \frac{\text{Temps de formation de la fibrine}}{\text{Temps Témoin}}$$

Ceci permet d'amplifier les D-dimères proportionnellement à l'allongement du temps de formation de fibrine TFF chez les patients avec thrombose, et de les soustraire proportionnellement au raccourcissement du temps de formation de fibrine TFF chez les patients avec hyper coagulation.

Le ratio varie en fonction du type et du lot de réactif D-dimères utilisé pour le dosage; et en fonction du lot de réactif facteur tissulaire-phospholipides utilisé pour la mesure de la formation de fibrine. Les lots de réactifs peuvent alors avantageusement être calibrés selon cette formule.

**Exemple 3: Ajustement des D-dimères en fonction des D-dimères générés par l'hyper coagulation, pour différents plasmas de patients avec suspicion de thrombose (avec ou sans thrombose, et avec ou sans cancer)**

Le protocole utilisé dans cet exemple est le Protocole A. L'étape e) de la méthode utilisée dans cet exemple est celle de la première version de la méthode.

La corrélation linéaire y = ax + b de l'Exemple 2, est mise à profit dans la méthode selon l'invention, pour ajuster les D-dimères en fonction des D-dimères générés par l'hyper coagulation, comme décrit notamment dans l'Exemple 2 et le Tableau 2 ci-dessous. Les patients avec et sans thrombose sont surtout discriminés sur la base d'un taux élevé de D-dimères qui sont 3,6 fois plus élevés pour les patients avec thrombose que pour les patients sans thrombose et 2,3 fois plus élevés pour les patients cancéreux avec une thrombose que pour les patients sans thrombose, comme le montre le Tableau 2 ci-dessous.

L'ajustement des D-dimères en fonction des D-dimères générés par l'hyper coagulation permet de soustraire les D-dimères générés par l'hyper coagulation dans les états d'activation de la coagulation.

**Tableau 2.** Ajustement des D-dimères en fonction des D-dimères générés par l'hyper coagulation dans des plasmas de patients avec suspicion de thrombose (avec ou sans thrombose, et avec ou sans cancer).

| Plasmas de patients | Diagnostic D-dimères et/ou imagerie | Nombre | Taux de D-dimères initiaux (µg/mL) | | Taux de D-dimères ajustés/hyper coag (µg/mL) | |
|---|---|---|---|---|---|---|
| | | | Moy | Gamme | Moy | Gamme |
| Normaux | Ddi négatifs | 150 | 0,28 | 0,27-0,47 | 0,28 | 0,27-0,54 |
| Avec suspicion de thrombose avec ou sans cancer | Ddi négatifs | 127 | 0,31 | 0,27-0,49 | 0,33 | 0,19-0,66 |
| | Ddi positifs sans thrombose | 88 | 1,60 | 0,51-11,6 | 2,00 | 0,43-19,0 |
| | Ddi positifs **avec thrombose** | 21 | **5,72** | **1,33-30,0** | **7,19** | **1,41-41,0** |
| Variation +/- thrombose | | | x 3.6 | | x 3.6 | |
| Avec cancer Avec suspicion de thrombose avec cancer | Imagerie sans thrombose | 16 | 2,52 | 0,27-11,6 | 2,94 | 0,59-19,0 |
| | Imagerie **avec thrombose** | 6 | **5,21** | **2,73-10,9** | **6,89** | **2,59-15,5** |
| Variation +/- thrombose | | | x 2.1 | | x 2.3 | |

**Exemple 4: Niveau du plateau de fibrine, de différents plasmas de patients normaux et de patients avec suspicion de thrombose (avec ou sans thrombose, et avec ou sans cancer), par la méthode de l'invention**

*Echantillons de Plasma*

Le protocole utilisé dans la première partie de cet exemple est le Protocole A. L'étape d) de la méthode utilisée dans cet exemple est indifféremment celle de la première ou la deuxième version de la méthode.

Parmi les échantillons de patients avec suspicion d'EP et/ou de TVP testés, les échantillons de patients avec un diagnostic positif de thrombose à l'imagerie ou avec une exclusion de thrombose, sont différenciés des patients avec un état d'activation de la coagulation et des patients normaux (sujets sains normaux ne présentant pas de suspicion de thrombose) sur le niveau du plateau de formation de la fibrine, représentatif de l'inflammation, comme montré dans les Figures 6, 9, et le Tableau 3 ci-après. Le niveau du plateau de DDO par la méthode de l'invention est d'autant plus élevé que l'inflammation, représentée par la protéine C réactive (CRP) et par le fibrinogène dans les Figures 6(**A, B**) est importante, indépendamment de la présence ou de l'exclusion de thrombose.

Le niveau du plateau de formation de la fibrine, qui représente la polymérisation maximale du caillot, est bien corrélé au taux de fibrinogène de l'échantillon, quels que soient les patients selon une équation logarithmique y = a lnx + b, où y est la variation de densité optique au temps de début du plateau, et lnx est le log népérien de x qui représente le taux de fibrinogène, comme décrit en Figures 9(**A, B**). La variable x est calculée à partir de l'équation, par la fonction exponentielle du log népérien de x. Le facteur reliant le taux de fibrinogène et la DDO est constant quels que soient les patients, comme le montre le Tableau 3 ci-dessous.

Les patients avec et sans thrombose, y compris dans le cas de patients ayant un cancer, sont discriminés sur la base de l'inflammation, représentée par la quantité de fibrinogène et/ou de DDO, comme le montre le Tableau 3 ci-dessous.

**Tableau 3.** Niveau du plateau de fibrine des plasmas de patients avec suspicion de thrombose (avec ou sans thrombose, et avec ou sans cancer) déterminé par la DDO au temps d'atteinte du plateau de formation de la fibrine.

| Plasmas de patients | Diagnostic D-di et/ou imagerie | Nombre | Taux de Fibrinogène (µg/mL) | | DDO 540 nm | | Delta DDO/Fib |
|---|---|---|---|---|---|---|---|
| | | | Moy | Gamme | Moy | Gamme | |
| Normaux | Ddi négatifs | 150 | 2,93 | 2,12-4,56 | 0,937 | 0,618-1,423 | 0,32 |
| Avec suspicion de thrombose avec ou sans cancer | Ddi négatifs | 127 | 3,79 | 1,93-8,40 | 1,225 | 0,581-2,237 | 0,32 |
| | Ddi positifs sans thrombose | 88 | 4,50 | 1,67-8,27 | 1,437 | 0,458-2,119 | 0,32 |
| | Ddi positifs **avec thrombose** | 21 | **4,83** | **3,00-7,54** | **1,509** | **1,007-2,059** | 0,31 |
| Avec suspicion de thrombose avec cancer | Imagerie sans thrombose | 16 | 4,15 | 2,63-7,23 | 1,424 | 0,716-2,051 | 0,34 |
| | Imagerie **avec thrombose** | 6 | **4,91** | **3,43-7,54** | **1,598** | **1,134-2,059** | 0,33 |

### *Echantillons de Sang Total*

Le protocole utilisé dans la deuxième partie de cet exemple est le Protocole B. Cette méthode est effectuée en présence d'un inhibiteur anti-plaquettaire, en l'occurrence la cytochalasine D.

Le niveau de l'inflammation est déterminé à partir de l'équation linéaire, qui relie l'amplitude au temps d'atteinte du plateau, selon la méthode alternative en sang total comme montré dans la Figure 5(**B**), et le taux de fibrinogène en Clauss, comme le montre la Figure 9(**C**). Les patients testés avec la variante de la méthode selon l'invention, sont aussi bien discriminés avec la méthode basée sur l'amplitude au temps d'atteinte du plateau, qu'avec la méthode de l'invention basée sur la détermination de la DDO.

### Exemple 5: Ajustement des D-dimères en fonction des D-dimères générés par l'inflammation, pour différents plasmas de patients normaux et de patients avec suspicion de thrombose (avec ou sans thrombose, et avec ou sans cancer), par la méthode de l'invention

Le protocole utilisé dans cet exemple est le Protocole A. Les étapes e) et f) de la méthode utilisée dans cet exemple sont indifféremment celles de la première ou de la deuxième version de la méthode.

La fonction logarithmique de l'Exemple 4, est mise à profit dans la méthode selon l'invention, pour ajuster les D-dimères en fonction des D-dimères générés par l'inflammation par le ratio R :

$$R = \frac{\text{D-dimères ajustés en fonction des D-dimères générés par l'hyper coagulation}}{\text{Niveau d'inflammation}}$$

et les exprimer en unités équivalentes au fibrinogène FEU.

La quantité de D-dimères générés par la plasmine étant environ 50% de l'unité FEU avec les anticorps 8D2 et 2.1.16 du réactif latex utilisé, le seuil de positivité de la mesure est ainsi de 0,5 µg/mL.

Le taux des D-dimères ajusté est déterminé par rapport à un seuil, de préférence un seuil de 0,5 µg/mL, pour déterminer la probabilité d'une embolie pulmonaire (EP) ou d'une thrombose veineuse profonde (TVP) dans l'échantillon du patient, comme le montrent les Figures 12(**A, B**) et le Tableau 4 ci-dessous.

Les 127 patients exclus sur les D-dimères ont tous un taux de D-dimères ajustés négatifs. Parmi les 88 patients sans thrombose, dont les D-dimères sont faussement positifs, 80% ont un taux de D-dimères ajustés négatif et évitent l'imagerie. Plus de 90% des patients sans thrombose évitent ainsi l'imagerie avec la méthode de l'invention.

100% des patients avec thrombose ont un taux de D-dimères ajustés positif variant de 0,52 µg/mL à 10,5 µg/mL, comme montré dans le Tableau 4 ci-dessous.

Parmi les échantillons de patients sans thrombose, trouvés faussement positifs en D-dimères ajustés avec un taux de 0,50 µg/mL à 2,73 µg/mL, 1/3 ont un taux < 0,60 µg/mL, 1/3 ont un cancer, et 1/3 ont un état d'activation de la coagulation.

**Tableau 4.** Discrimination des plasmas de patients avec suspicion de thrombose (avec ou sans thrombose, et avec ou sans cancer), avec un ajustement des D-dimères en fonction des D-dimères générés par l'hyper coagulation et en fonction des D-dimères ajustés pour l'inflammation.

| Plasmas de patients | Diagnostic Ddi et/ou imagerie | N | Taux de D-dimères ajustés /hyper coag H | | Taux de D-dimères ajustés /inflammation I | | Résultat rendu |
|---|---|---|---|---|---|---|---|
| | | | Moy | Gamme | Moy | Gamme | |
| Avec suspicion de thrombose avec ou sans cancer | Ddi négatifs | 127 | 0,33 | 0,19-0,66 | 0,09 | 0,04-0,21 | 0 Faux + |
| | Ddi positifs sans thrombose | 88 | 2,00 | 0,43-19,0 | **0,42** | 0,11-2,73 | 18 Faux + |
| | Ddi positifs **avec thrombose** | 21 | 7,19 | 1,41-41,0 | **1,65** | 0,52-10,5 | **0 Faux -** |
| Avec suspicion de thrombose avec cancer | Imagerie sans thrombose | 16 | 4,24 | 0,59-19,0 | **0,72** | 0,12-2,73 | 7 Faux + |
| | Imagerie **avec thrombose** | 6 | 7,20 | 2,59-15,5 | **1,44** | 0,58-3,65 | **0 Faux -** |

**Exemple 6: Différenciation des D-dimères générés par la fibrine intravasculaire, de ceux générés dans les états d'activation importante de la coagulation sur l'hyper fibrinolyse**

Le protocole utilisé dans cet exemple est le Protocole A. L'étape g) de la méthode utilisée dans cet exemple est celle de la première version de la méthode.

La différenciation entre les D-dimères présents dans ces états d'activation et ceux de la thrombose se fait sur l'hyper fibrinolyse. La génération de produits de dégradation du fibrinogène PDFg par la plasmine dans les états d'activation aigus, permet leur exclusion. L'hyper fibrinolyse est calculée à partir des équations linéaires de la Figure 10(**A**, **B**) pour le réactif Stago, et Figure 11(**A, B**) pour le réactif IL (étape g)).

En présence d'une hyper fibrinolyse (PDFg > 7 $\mu$g/mL), les PDFg générés avec la fibrine intravasculaire sont systématiquement plus faibles dans les échantillons provenant de patients avec thrombose que dans ceux de patients sans thrombose; ceci quel que soit le réactif des D-dimères utilisé. Ceci permet de discriminer les patients avec thrombose de ceux sans thrombose, sur la base d'une différence entre les PDFg avant et après ajustement des D-dimères positive, au seuil de 1 $\mu$g/mL, comme montré dans le Tableau 5 ci-dessous. A l'inverse, une différence négative ou <1.0 chez les patients sans thrombose permet d'orienter sur un état d'activation aigu de la coagulation.

**Tableau 5.** Discrimination des plasmas de patients avec suspicion de thrombose (avec ou sans thrombose, et avec ou sans cancer), en fonction de l'hyper fibrinolyse avec déduction des PDFg, par la méthode optique sur STAR® (Stago).

| Plasmas de patient | Diagnostic Ddi positif et imagerie | N | Ddi ajustés | PDFg /Ddi | PDFg Ddi ajustés | Différence PDFg seuil >1 µg/mL | Résultat rendu |
|---|---|---|---|---|---|---|---|
| | Ddi Nég | 127 | 0,09 | Nég | Nég | / | Négatifs |
| Avec suspicion thrombose avec ou sans cancer | Sans thrombose | 69 | 0,26 | Nég | Nég | / | Négatifs |
| | | 4 | **0,51** | Nég | Nég | / | **4 Faux +** |
| | | 9 | 1,13 | 11,2 | 10,9 | **+0,3** | Négatifs |
| | | 6 | 0,91 | 15,3 | 9,2 | **+6,1** (**+2,6 à+20,3**) | **6 Faux +** |
| | **Avec thrombose** | 2 | **0,55** | Nég | Nég | / | Positifs |
| | | 19 | 1,77 (0,58-10,5) | 18,8 (8,3-83,3) | 14,5 (7,1-68,7) | **+4,3** (**+1,2 à+14,6**) | Positifs |
| Avec suspicion de thrombose avec cancer | Sans thrombose | 10 | 0,26 | Nég | Nég | / | Négatifs |
| | | 2 | 1,97 | 16,1 | 15,8 | **+0,3** | Négatifs |
| | | 1 | **0,51** | Nég | Nég | / | **1 Faux+** |
| | | 3 | 0,97 | 18.8 | 9,5 | **+9,2** | **3 Faux+** |
| | **Avec thrombose** | 6 | **1,44** (0,58-3,65) | 16,2 (9,5-31,7) | 12,4 (7,1-26,2) | **+3,8** (**+1,3 à +6,7**) | Positifs Positifs |

Ainsi, 95% des patients évitent l'imagerie sur la base d'un taux négatif de D-dimères ajustés en fonction des D-dimères générés à la fois par l'hyper coagulation (H), l'inflammation (I) et l'hyper fibrinolyse (F).

Parmi les faux positifs, 3 sont en limite de seuil, 4 sont des cancers et 3 ont une cause d'activation de la coagulation et peuvent éviter l'imagerie sur la base d'un raccourcissement important du temps de formation de fibrine, sans générer de faux négatifs, comme montré en Exemple 7.

**Exemple 7: Ajustement des D-dimères par la méthode de l'invention comparativement au ratio [Ddi/Fibrinogène par la méthode de Clauss]**

Le protocole utilisé dans cet exemple est le Protocole A. L'étape h) de la méthode utilisée dans cet exemple est celle de la première version de la méthode.

L'ajustement des D-dimères en fonction des D-dimères générés par l'hyper coagulation (H), l'inflammation (I) et l'hyper fibrinolyse (F) selon la première version de la méthode de l'invention (étape h)) est plus performant que le ratio [D-dimères /Fibrinogène], comme le montrent la Figure 12 et le Tableau 6 ci-dessous. Les patients avec thrombose sont discriminés de ceux sans thrombose sur la base d'un taux élevé de D-dimères ajustés, qui sont multipliés par un facteur 5,3, comme le montre le Tableau 6 ci-dessous. Ainsi, 92% des patients avec un taux de D-dimères faussement positif, soit 97% des patients sans thrombose sont rendus négatifs avec l'ajustement des D-dimères selon l'étape h), comme le montre la Figure 12. Parmi les 7 faux +, 4 sont des cancers et 3 sont en limite de seuil.

**Tableau 6.** Ajustement des D-dimères par la méthode optique de l'invention, comparativement à l'ajustement par le ratio [D-dimères/Fibrinogène par la méthode de Clauss], dans les plasmas de patients avec suspicion de thrombose (avec ou sans thrombose, et avec ou sans cancer) (H: hyper Coagulation, I: inflammation, F: hyper Fibrinolyse, Ddi: D-dimères, Fib: fibrinogène)

| Plasmas de patients | Diagnostic Ddi et/ou imagerie | N | Ddi ajustés [Ddi / (H+I+F)] | | Résultat | Ratio [Ddi/Fib] Clauss | | Résultat |
|---|---|---|---|---|---|---|---|---|
| | | | Moy | Gamme | | Moy | Gamme | |
| Avec suspicion d'EP et/ou TVP avec ou sans cancer | Ddi négatifs | 127 | 0,09 | 0,04-0,21 | Nég | 0,09 | 0,04-0,16 | Nég |
| | Ddi positifs Sans thrombose | 88 | **0,31** | 0,11-1,56 | 7 Faux+ | **0.40** | 0,09-3,60 | 17 Faux+ |
| | Ddi positifs **Avec thrombose** | 21 | **1,65** | 0,52-10,5 | 0 Faux- | **1,35** | 0,38-7,71 | **2 Faux-** |
| Variation +/- thrombose | | | x 5,3 | | | x 3,4 | | |
| Avec suspicion de thrombose avec cancer | Sans thrombose | 16 | **0,45** | 0,12-1,56 | 4 Faux+ | **0.75** | 0.09-3,60 | 7 Faux+ |
| | **Avec thrombose** | 6 | **1,44** | 0,58-3,65 | 0 Faux- | **1,18** | 0,38-3,0 | **1 Faux-** |
| Variation +/- thrombose | | | x 3,2 | | | x 1,6 | | |

Les faux négatifs avec le ratio [D-dimères/Fibrinogène] sont détectés par la méthode de l'invention, ce qui permet de ne pas passer à côté d'une thrombose.

Il y a moins de faux positifs par la méthode de l'invention qu'avec le ratio [D-dimères/Fibrinogène], ce qui permet de diminuer le nombre de patients diagnostiqués par l'imagerie.

**Exemple 8: Influence du taux de D-dimères ajustés selon la méthode de l'invention, sur la probabilité d'EP et/ou de TVP**

Le protocole utilisé dans cet exemple est le Protocole A. L'étape h) de la méthode utilisée dans cet exemple est celle de la première version de la méthode.

Le seuil des D-dimères ajustés selon la méthode de l'invention n'a avantageusement pas été ajusté en fonction de l'âge. En effet, les taux des D-dimères, l'activation de la coagulation, et l'inflammation augmentent progressivement en fonction de l'âge après 50 ans. De ce fait, le ratio n'est pas ajusté en fonction de l'âge.

Plus les taux de D-dimères ajustés selon la méthode de l'invention sont élevés, plus le dénominateur de la formule avec le temps de formation de fibrine et du ratio avec la DDO est élevé, plus la probabilité d'EP ou de TVP est élevée.

Les D-dimères ajustés sont d'autant plus importants que l'EP est étendue et que la TVP est proximale plutôt que distale ou superficielle, comme montré dans le Tableau 7 ci-dessous.

**Tableau 7.** Probabilité d'EP et de TVP en fonction du taux des D-dimères ajustés pour l'hyper coagulation (H), l'inflammation (I) et l'hyper fibrinolyse (F), (TFF: temps de formation de fibrine, T: Temps Témoin moyen - 1 écart-type = 120 sec), PDFg: produits de dégradation du fibrinogène, Ddi: D-dimères, Act: état d'activation de la coagulation).

| Nombre | Ddi | Ddi ajustés [Ddi / C+I+F] | Delta PDFg | TFF ≤ T | Probabilité EP et/ou TVP | Plasmas de patients avec EP et/ou TVP |
|---|---|---|---|---|---|---|
| 1 | 1,56 | **0,58** | / | 190 | Moyen | EP sous segmentaire |
| 3 | 3,60 | **0,92** | +2.6 | 157 | Elevé | EP segmentaire |
| 1 | 7,0 | **1,75** | **+6,7** | 212 | Elevé | EP artère pulmonaire bilatérale |
| 1 | 10,9 | **3,65** | **+5,5** | 188 | Elevé | EP proximale et distale |

(suite)

| Nombre | Ddi | Ddi ajustés [Ddi / C+I+F] | Delta PDFg | TFF ≤ T | Probabilité EP et/ou TVP | Plasmas de patients avec EP et/ou TVP |
|---|---|---|---|---|---|---|
| 1 | 30,0 | **10,47** | **+14,6** | 182 | Très élevé | EP massive |
| 1 | 0,72 | 0,25 | / | **88** | Activation | Inflammation veine péronière |
| 2 | 1,44 | 0,49 | / | 162 | Faible | TVP superficielle |
| 1 | **2,28** | **0,58** | +1,2 | 162 | Moyen | TVP distale |
| 1 | **3,60** | **0,91** | +2,7 | 218 | Elevé | TVP proximale |
| 1 | **7,70** | **1,62** | **+9,3** | 192 | Elevé | |
| 1 | **12,1** | **4,19** | +5,3 | 144 | Elevé | TVP jugulaire volumineuse |

**Exemple 9: Reproductibilité inter-instruments de la mesure de formation de fibrine**

Le protocole utilisé dans cet exemple est le Protocole C sur STA-R®, qui a été utilisé comme suit, selon les étapes a), b), c) et d) de la première ou de la deuxième version de la méthode.

Dans 8 cuvettes de l'automate STA-R® Evolution Expert Series (Stago), sont ajoutés simultanément par l'instrument:

- 200 μL d'échantillon de plasmas contrôles: normal (STA® CCN); hyper-coagulant (plasma déplété en protéine S, STA DEF PS) et de plasma contrôle hypo fibrinolytique (plasma contenant de l'inhibiteur de l'activateur du plasminogène PAI-1, PAI 4C),
- 50 μL d'un mélange de facteur tissulaire et de phospholipides [FT 2 pM final + PL 4 μM final].

Après agitation, et incubation pendant 300 secondes à 37°C, l'automate ajoute 50 μL de CaCl$_2$ à 16,7 mM final, et agite par l'aiguille. L'automate mesure enfin le temps de formation de la fibrine et la variation de densité optique DDO au temps d'atteinte du plateau (TA); à la longueur d'onde de 540 nm, en fonction du temps pendant 10 minutes.

La reproductibilité inter-instruments de la méthode a été déterminée sur trois instruments STAR®, comme suit: 12 séries de 2 mesures, soit 24 mesures, ont été réalisées sur chacun des 3 instruments, pendant 5 jours consécutifs, à partir de 2 flacons de chacun des plasmas contrôles normal (CCN) et pathologiques (DPS: plasma contrôle déplété en protéine S, hypercoagulant, PAI: plasma contrôle hypo fibrinolytique), soit 48 mesures pour chaque plasma contrôle. Les valeurs aberrantes ont été éliminées selon Rosner.

Les coefficients de variation (CV) obtenus sur le temps de formation de fibrine TFF sont tous inférieurs à 6%; ceux obtenus sur la DO au temps du plateau et sur la DO maximale sont tous inférieurs à 3%, quel que soit le plasma contrôle, comme montré dans le Tableau 8 ci-dessous. Les variations inter-STAR® sont inférieures à 2.5% quel que soit le paramètre et le plasma contrôle.

**Tableau 8.** Reproductibilité inter-instruments de la mesure de formation de fibrine (CCN: plasma contrôle normal, DPS: plasma contrôle déplété en protéine S, hyper-coagulant, PAI: plasma contrôle hypo fibrinolytique).

| N=48 mesures | Temps de formation de fibrine TFF (sec) | | | DO au temps du plateau | | | DO maximale | | |
|---|---|---|---|---|---|---|---|---|---|
| Plasma contrôle | CCN | DPS | PAI | CCN | DPS | PAI | CCN | DPS | PAI |
| STAR® 1 | | | | | | | | | |
| moyenne | 100,4 | 82,4 | 117,8 | 2,200 | 1,977 | 2,280 | 2,223 | 2,004 | 2,306 |
| min | 86,0 | 78.0 | 102 | 2,157 | 1,751 | 2,217 | 2,174 | 1,768 | 2,239 |
| max | 110,0 | 86,0 | 126 | 2,278 | 2,053 | 2,347 | 2,310 | 2,071 | 2,384 |
| CV | 5,8% | 2,8% | 4,5% | 1,4% | 2,8% | 1,4% | 1,5% | 2.9% | 1.5% |

(suite)

| STAR® 2 | | | | | | | | | |
|---|---|---|---|---|---|---|---|---|---|
| moyenne | 99,7 | 83,1 | 118,2 | 2,222 | 2,015 | 2,309 | 2,337 | 2,043 | 2,337 |
| min | 88,0 | 78,0 | 100,0 | 2,073 | 1,788 | 2,248 | 2,265 | 1,804 | 2,265 |
| max | 116,0 | 90,0 | 138,0 | 2,280 | 2,114 | 2,381 | 2,422 | 2,154 | 2,422 |
| CV | 5,6% | 3,3% | 5,7% | 1,9% | 2,7% | 1,3% | 1,5% | 2,8% | 1,5% |
| STAR® 3 | | | | | | | | | |
| moyenne | 100,1 | 84,2 | 118,7 | 2,245 | 2,030 | 2,328 | 2,265 | 2,057 | 2,352 |
| min | 90,0 | 76,0 | 106,0 | 2,192 | 1,879 | 2,261 | 2,213 | 1,904 | 2,279 |
| max | 110,0 | 90,0 | 134,0 | 2,323 | 2,112 | 2,374 | 2,365 | 2,129 | 2,395 |
| CV | 4,4% | 3,5% | 4,6% | 1,2% | 2,6% | 1,2% | 1,3% | 2,4% | 1,1% |
| Variations inter-STAR® | | | | | | | | | |
| moyenne | 99,9 | 83,2 | 118,2 | 2,222 | 2,007 | 2,306 | 2,275 | 2,035 | 2,332 |
| écart-type | 0,35 | 0,90 | 0,42 | 0,02 | 0,03 | 0,02 | 0,06 | 0,03 | 0,02 |
| CV | 0,3% | 1,1% | 0,4% | 1,0% | 1,4% | 1,1% | 2,5% | 1,3% | 1,0% |

**Exemple 10: Répétabilité de la mesure de formation de fibrine**

Le protocole utilisé dans cet exemple est le Protocole A sur STA-R®, qui a été utilisé comme suit, selon les étapes a), b), c) et d) de la première ou de la deuxième version de la méthode.

Dans 8 cuvettes de l'automate STA-R® Evolution Expert Series (Stago), sont ajoutés simultanément par l'instrument:

- 200 μL d'échantillon de plasma citraté de patients normaux, ou de patients avec et sans suspicion de thrombose veineuse,
- 50 μL d'un mélange de facteur tissulaire et de phospholipides [FT 2 pM final + PL 4μM final].

Après agitation, et incubation pendant 300 secondes à 37°C, l'automate ajoute 50 μL de $CaCl_2$ à 16,7 mM final, et agite par l'aiguille. L'automate mesure enfin le temps de formation de la fibrine et la variation de densité optique DDO au temps d'atteinte du plateau (TA); à la longueur d'onde de 540 nm, en fonction du temps pendant 10 minutes.

La répétabilité des doublons de mesure a été déterminée sur 4 instruments STAR®, pour chaque paramètre de la méthode, et pour chacun des échantillons. Les 154 plasmas de patients normaux ont été testés avec un premier lot de réactifs sur un des instruments STAR®, les 83 échantillons de patients avec suspicion de thrombose ont été testés avec un second lot de réactif sur les 3 autres instruments STAR® pour les D-dimères, le temps de formation de la fibrine, la DDO au plateau et le fibrinogène.

La moyenne et l'écart-type des variations obtenues sur chacun des paramètres sont reportés dans les Tableaux 9 et 10 ci-dessous.

La moyenne des variations obtenues sur les mesures de formation de la fibrine est inférieure à 2% pour les 154 patients normaux; elle est inférieure à 1% pour les 83 patients avec et sans thrombose veineuse, comme le montrent les Tableaux 9 et 10.

Les mesures des paramètres de formation de la fibrine sont aussi répétables que celles du fibrinogène en méthode de Clauss.

Les mesures des D-dimères spécifiques sont aussi répétables que celles des D-dimères et du ratio [D-dimères/Fibrinogène], la moyenne des variations est inférieure à 2,3 %.

Parmi les 5 échantillons rendus positifs en D-dimères spécifiques et négatifs par le ratio [Ddi/Fib] sur les doublons, 2 sont issus de patients avec une embolie pulmonaire confirmée à l'imagerie, 2 ont une hyper-activation de la coagulation et 1 est issu d'un patient avec une cardiopathie.

Ainsi, 2 patients avec une EP, trouvés faussement négatifs par le ratio [Ddi/Fib], peuvent être diagnostiqués par la méthode de l'invention; alors qu'un seul patient sans EP, requiert une imagerie par la méthode de l'invention.

**Tableau 9.** Répétabilité des doublons de mesure de la formation de fibrine dans les échantillons de patients normaux.

| Echantillons de Patients normaux Nombre de doublons | | Mesures de formation de fibrine | | Variations des mesures | |
|---|---|---|---|---|---|
| | | Moyenne | Ecart type | Moyenne | Ecart type |
| Temps de formation de la fibrine TFF (secondes) | 150 | 134 82-206 | 24 | **-1,1%** | **7,1%** |
| DDO au plateau (540 nm) | 150 | 0.937 0,618-1,423 | 0.20 | **-0,5%** | **1,8%** |

**Tableau 10.** Répétabilité des doublons de mesure de la formation de fibrine, des D-dimères et D-dimères ajustés selon la méthode de l'invention, ainsi que du ratio [D-dimères/Fibrinogène] dans les échantillons de patients avec et sans thrombose à l'imagerie.

| Echantillons de Patients avec et sans thrombose à l'imagerie Nombre de doublons | | Mesures de formation de fibrine | | Variations des mesures | |
|---|---|---|---|---|---|
| | | Moyenne | Ecart type | Moyenne | Ecart type |
| Fibrinogène (g/L) | 83 | 3,97 1,92-8,40 | 1,10 | 0,4% | 3,0% |
| Temps de formation de la fibrine TFF (secondes) | 83 | 151.6 88-382 | 41,4 | 0,9% | 5,0% |
| DDO au plateau (540 nm) | 83 | 1.262 0,559-2,241 | 335 | 0,2% | 2,0% |
| D-dimères positifs > 0.5 $\mu$g/mL | 30 | 2.20 0,52-12,13 | 3,15 | 2,1% | 7% |
| **Ratio [D-dimères /Fibrinogène]** | **30** | **0,67 0,12-4,92** | **1,14** | **2,1%** | **6,7%** |
| Ratio négatif | 25 | 0,28 0,12-0,53 | 0,13 | 2,4% | 7,3% |
| Ratio positif | 5 | 2,61 0,60-4,92 | 1,85 | 0,2% | 1,8% |
| **D-dimères spécifiques de la thrombose ($\mu$g/mL)** | **30** | **0.71 0,14-4,28** | **1,00** | **2,3%** | **6,8%** |
| Ddi spécifiques négatifs | 21 | 0,30 0,14-0,50 | 0,11 | 3,6% | 7,7% |
| Ddi spécifiques positifs | 9 | 1.66 0,51-4,28 | 1,44 | -0,6% | 1,8% |

**Exemple 11: Ajustement des D-dimères selon la méthode de l'invention avec un autre réactif de dosage des D-dimères**

Le protocole utilisé dans cet exemple est le Protocole A. Les étapes e) à h) de la méthode utilisée dans cet exemple sont celles de la première version de la méthode.

Les D-dimères sont ajustés en fonction des D-dimères générés par l'hyper coagulation (H) par la formule de l'Exemple 2, de l'inflammation (I) par la fonction logarithmique de l'Exemple 4 et le ratio R de l'Exemple 5, et de l'hyper fibrinolyse (F) par les équations linéaires de l'Exemple 6, avec la méthode de dosage des D-dimères Instrumentation Laboratory (IL).

L'ajustement des D-dimères en fonction des D-dimères générés par l'hyper coagulation permet de soustraire les D-dimères générés par l'hyper coagulation dans les états d'activation de la coagulation, mais ne permet pas de discriminer les patients avec et sans thrombose, comme le montre le Tableau 11.

**Tableau 11.** Ajustement des D-dimères avec la méthode de dosage des D-dimères IL en fonction des D-dimères générés par l'hyper coagulation dans des plasmas de patients avec suspicion de thrombose.

| Plasmas de patients | Diagnostic D-dimères et/ou imagerie | Nombre | Taux de D-dimères initiaux ($\mu$g/mL) | | Taux de D-dimères ajustés/hyper coag ($\mu$g/mL) | |
|---|---|---|---|---|---|---|
| | | | Moy | Gamme | Moy | Gamme |
| Avec suspicion de thrombose | Ddi négatifs | 120 | 0,43 | 0,11-3,32 | 0,46 | 0,09-3,35 |
| | Ddi positifs sans thrombose | 79 | 1,75 | 0,20-7,34 | 2,12 | 0,21-16,7 |
| | Ddi positifs **avec thrombose** | 15 | **8,42** | **1,39-25,4** | **10,9** | **1,82-34,7** |

L'ajustement des D-dimères en fonction des D-dimères générés par l'inflammation permet de discriminer les patients avec et sans thrombose, comme le montrent la Figure 13 et le Tableau 12. Ainsi, 90% des patients environ peuvent éviter l'imagerie avec l'un ou l'autre des 2 réactifs Stago et IL, sur un taux de D-dimères ajustés négatif.

**Tableau 12.** Ajustement des D-dimères avec la méthode de dosage des D-dimères IL en fonction des D-dimères générés par l'hyper coagulation et par l'inflammation dans des plasmas de patients avec suspicion de thrombose.

| Plasmas de patients | Diagnostic Ddi et/ou imagerie | N | Taux de D-dimères ajustés ($\mu$g/mL) /hyper coag H | | Taux de D-dimères ajustés ($\mu$g/mL) /inflammation I | | Résultat Faux + Faux - |
|---|---|---|---|---|---|---|---|
| | | | Moy | Gamme | Moy | Gamme | |
| Plasmas de patients avec suspicion de thrombose | Ddi négatifs | 120 | 0,46 | 0,09-3,35 | 0,12 | 0,02-1,04 | 2 Fx + |
| | Ddi positifs sans thrombose | 79 | 2,12 | 0,21-16,7 | **0,45** | 0,06-2,47 | 22 Fx+ |
| | Ddi positifs **avec thrombose** | 15 | **10,9** | **1,82-34,7** | **2,45** | 0,49-8,84 | **1 lim** |

L'ajustement des D-dimères en fonction des D-dimères générés par l'hyper fibrinolyse (F), en plus de l'hyper coagulation (H) et de l'inflammation (I), permet de discriminer davantage de patients, comme le montrent la Figure 13 et les Tableaux 13 et 14 ci-dessous. Ainsi, plus de 95% des patients peuvent éviter l'imagerie, sur un taux négatif de D-dimères ajustés selon la méthode de l'invention. Parmi les 9 faux +, 3 sont des cancers avec métastases, 3 sont en limite de seuil ($\leq$0.60) et 3 sont des patients âgés (>80 ans).

**Tableau 13.** Discrimination des plasmas de patients avec suspicion de thrombose (avec ou sans thrombose), par la méthode de l'invention, avec la méthode de dosage des D-dimères IL, en fonction des D-dimères générés par l'hyper fibrinolyse avec la déduction des PDFg.

| Plasmas de patients | Diagnostic Ddi positif et imagerie | N | Ddi ajustés | PDFg Ddi | PDFg Ddi ajustés | PDFg Seuil $\geq$1.0 | Résultat |
|---|---|---|---|---|---|---|---|
| Avec suspicion thrombose | Ddi négatifs | 120 | 0,12 | / | / | / | Nég |
| | Sans thrombose | 79 | 0,45 | 5,67 | 5,52 | / | **11 Fx+** |
| | **Avec thrombose** | 15 | 2,45 | 23,4 | 17,7 | **+5,66** | **Positifs 1 limite** |

Cet ajustement selon la méthode de l'invention est plus performant que le ratio [D-dimères /Fibrinogène], comme le montrent la Figure 13 et le Tableau 14 ci-dessous. Les faux négatifs avec le ratio [D-dimères/Fibrinogène] sont détectés par la méthode de l'invention, ce qui permet de ne pas passer à côté d'une thrombose. Il y a moins de faux positifs par la méthode de l'invention qu'avec le ratio [D-dimères/Fibrinogène], ce qui permet de diminuer le nombre de patients diagnostiqués à l'imagerie.

**Tableau 14.** Comparaison de l'ajustement des D-dimères par la méthode optique de l'invention avec le réactif de dosage des D-dimères IL et par le ratio [D-dimères/Fibrinogène], dans les plasmas de patients avec ou sans thrombose (H: hyper coagulation, I: inflammation, F: hyper Fibrinolyse, Ddi: D-dimères, Fib : fibrinogène).

| Plasmas de patients | Diagnostic Ddi et/ou imagerie | Nombre | Ddi ajustés [Ddi / (C+I+F)] | | Résultat | Ratio [Ddi / Fib] (Clauss) | | Résultat Faux + Faux - |
|---|---|---|---|---|---|---|---|---|
| | | | Moy | Gamme | | Moy | Gamme | |
| Avec suspicion d'EP et/ou TVP | Ddi négatifs | 120 | 0,12 | 0,02-0,41 | 0 Fx + | 0,12 | 0,01-1,01 | **2 Fx +** |
| | Ddi positifs Sans thrombose | 79 | **0,37** | 0,06-2,23 | **9 Fx+** | **0,41** | 0,05-2,26 | 17Fx+ |
| | Ddi positifs **Avec thrombose** | 15 | **2,45** | 0,49-8,84 | **1 lim** | **2,01** | 0,37-6,52 | **3 Fx -** |

**Exemple 12: Performances Diagnostiques de la Méthode selon l'Invention pour les Patients avec Suspicion de Thrombose (avec ou sans Thrombose, avec ou sans Cancer) en Fonction de l'Âge**

Le protocole utilisé dans cet exemple est le Protocole A. Les étapes e) à h) de la méthode utilisée dans cet exemple sont celles de la deuxième version de la méthode.

Le taux des D-dimères qui résultent de la dégradation de la fibrine intravasculaire R, a été calculé selon l'étape e') en ajustant le taux de D-dimères de l'échantillon en fonction du taux des D-dimères générés par l'inflammation, et en corrigeant ce taux ajusté pour les D-dimères faibles (<4 $\mu$g/mL). Le taux de D-dimères générés par l'hyper fibrinolyse a été calculé selon l'étape g'), pour chacun des échantillons classés en fonction de l'inflammation. Puis le ratio TA/TFF a été comparé par rapport à un seuil, afin d'exclure ou de diagnostiquer une thrombose chez le patient, ou d'orienter sur un état d'activation de la coagulation chez le patient.

Un total de 218 patients avec suspicion de thrombose (avec ou sans thrombose, avec ou sans cancer) ont été testés. Parmi ces 218 patients testés, 47% avaient plus de 50 ans, 17% avaient plus de 75 ans et 15% avaient un cancer. Le diagnostic d'EP et/ou de TVP a été exclu sur la base d'un test d'imagerie ou d'un dosage de D-dimères négatif selon la probabilité clinique, chez 199 patients (91.3%). Le diagnostic d'EP et/ou de TVP a été confirmé sur la base d'un test d'imagerie positif chez 19 patients soit 8.7%. L'exclusion et/ou le diagnostic d'une thrombose ou d'un état d'activation de la coagulation ont été réalisés pour les 218 patients en utilisant la deuxième version de la méthode de l'invention. Les performances diagnostiques de la méthode ont été comparées à celles du dosage habituel des D-dimères et à celles des D-dimères ajustés à l'âge.

Les performances diagnostiques de la méthode de l'invention ont ensuite été déterminées sur un plus grand nombre de patients avec suspicion de thrombose (avec ou sans thrombose, avec ou sans cancer). Sur un total de 796 patients testés, 49% avaient plus de 50 ans, 19% avaient plus de 75 ans et 7.3% avaient un cancer. Le diagnostic d'EP et/ou de TVP a été exclu chez 730 patients (91.7%). Le diagnostic d'EP et/ou de TVP a été confirmé chez 66 patients soit 8.3%.

L'utilité clinique de la détermination du taux des D-dimères est limitée chez les patients âgés. En effet, le seuil de D-dimères doit être ajusté à l'âge pour exclure l'embolie pulmonaire (Righini et al., JAMA, 2014, 311: 1117-1124). Ce seuil ajusté correspond à l'âge multiplié par 10 pour les patients de 50 ans et plus. La combinaison du seuil de D-dimères ajusté pour l'âge et de l'évaluation prétest de la probabilité clinique est associée à un plus grand nombre de patients chez lesquels l'embolie pulmonaire aurait pu être exclue (Righini et al., JAMA, 2014, 311: 1117-1124). Des méthodes d'évaluation prétest de la probabilité clinique d'embolie pulmonaire (EP) et de thrombose veineuse profonde (TVP) sont connues dans l'art (voir Kolok et al., Arch. Intern. Med., 2008, 168 : 21-31, et Wells et al., NEJM, 2003, 349 : 1227-1235, respectivement).

Le seuil des D-dimères ajustés selon la méthode de l'invention n'a avantageusement pas été ajusté en fonction de l'âge. En effet, les taux des D-dimères, l'activation de la coagulation, et l'inflammation augmentent progressivement en fonction de l'âge après 50 ans. De ce fait, le ratio n'est pas ajusté en fonction de l'âge. Plus les taux de D-dimères ajustés selon la méthode de l'invention sont élevés, plus le dénominateur du ratio avec la DDO est élevé, plus la probabilité d'EP ou de TVP est élevée.

Pour les 218 patients avec suspicion de thrombose, la méthode de l'invention permet d'exclure davantage de patients (environ 20% de plus) que le dosage habituel des D-dimères ou des D-dimères ajustés à l'âge comme le montre le Tableau 15 ci-dessous. Ainsi plus de 75% des patients, testés par la méthode de l'invention n'ont pas besoin de tests d'imagerie complémentaire.

Les performances du Tableau 15 sont données au seuil habituel de 0,50 μg/mL pour les D-dimères et les D-dimères ajustés à l'âge, et au seuil de 0,51 μg/mL dans cet exemple avec la méthode de l'invention. La sensibilité des trois méthodes est de 100% vu qu'il n'y a pas eu de diagnostic faussement négatif; par contre, la spécificité de la méthode de l'invention est très supérieure à celle des deux autres méthodes, comme le montre le Tableau 15. Le rapport de vraisemblance positif est supérieur à celui des deux autres méthodes, confirmant que le patient a trois fois plus de chance d'avoir une thrombose lorsque le résultat est positif, qu'avec les autres méthodes.

Des performances diagnostiques similaires sont retrouvées lorsque la méthode selon l'invention est appliquée à un plus grand nombre de patients. Parmi les 796 patients avec suspicion de thrombose, la méthode permet d'exclure 79% des patients (plus de 20% de plus que les autres méthodes) comme le montre le Tableau 16, et quel que soit l'âge, comme le montrent le Tableau 17 et la Figure 15(**A**). La méthode selon l'invention permet également d'exclure davantage de patients (<75 ans d'âge) souffrant d'un cancer (voir Figure 15(**B**)). Enfin, la méthode selon l'invention permet non seulement une meilleure exclusion des patients âgés et des patients souffrant d'un cancer mais également des patients âgés souffrant d'un cancer (voir Figure 15(B)). Les résultats sur un plus grand nombre de patients démontrent une plus grande spécificité de la méthode selon l'invention comparée à la méthode de référence (voir Tableau 16). La valeur prédictive positive est environ deux fois plus élevée que celle de la méthode de référence.

**Tableau 15.** Comparaison des performances diagnostiques de la méthode de l'invention à celles des dosages des D-dimères et des D-dimères ajustés à l'âge, pour les 218 patients avec suspicion de thrombose (19 avec thrombose et 199 sans thrombose).

| Performances diagnostiques N=218 patients | Nombre de patients exclus pour le diagnostic d'EP ou de TVP | Sensibilité % | Spécificité % | Rapport de vraisemblance Positif (LR+) | Rapport de vraisemblance Négatif (LR-) |
|---|---|---|---|---|---|
| Méthode de l'invention | 166/218 (76.2%) | 100% | 83.2% | 6.03 | 0.00 |
| D-dimères | 115/218 (52.8%) | 100% | 57.8% | 2.37 | 0.00 |
| D-dimères ajustés à l'âge | 123/218 (56.4%) | 100% | 61.8% | 2.62 | 0.00 |

**Tableau 16.** Comparaison des performances diagnostiques de la méthode de l'invention à celles des dosages des D-dimères et des D-dimères ajustés à l'âge, pour les 796 patients avec suspicion de thrombose (66 avec thrombose et 730 sans thrombose).

| Performances diagnostiques N=796 patients | % Exclusion | Sensibilité | Spécificité | VPP (valeur prédictive positive) |
|---|---|---|---|---|
| Méthode de l'invention | 79% (+28%) | 99,8% | 86% | 39% (X2) |
| D-dimères | 51% | 100% | 56% | 17% |
| D-dimères ajustés à l'âge | 57% (+6%) | 100% | 62% | 17% |

**Tableau 17.** Taux de D-dimères (D-di) résultant de la dégradation de la fibrine intravasculaire selon la méthode de l'invention, pour les 386 patients de moins de 50 ans et les 379 patients de plus de 50 ans dont le taux de D-dimères (D-di) est < 5,1 μg/mL parmi les 796 patients avec suspicion de thrombose.

| Age | Diagnostic EP et/ou TVP | n | D-di μg/mL | Méthode de l'invention D-di μg/mL | Age moyen |
|---|---|---|---|---|---|
| < 50A N=386 | Positif EP ou TVP | 13 | 2.23 | 0.69 0.52-1.62 | 38A |
| | Négatif | 375 | 0.55 | 0.51 0.45-1 .39 | 35A |

(suite)

| Age | Diagnostic EP et/ou TVP | n | D-di μg/mL | Méthode de l'invention D-di μg/mL | Age moyen |
|---|---|---|---|---|---|
| >= 50A N=379 | Positif EP ou TVP | 36 | 2.25 | 0.65 0.55-0.95 | 72A |
| | Négatif | 343 | 1.04 | 0.56 0.46-1 .76 | 68A |

Les taux de D-dimères résultant de la dégradation de la fibrine intravasculaire selon la méthode de l'invention sont similaires pour les patients avec un diagnostic de thrombose quel que soit leur âge (âge moyen 38 ans ou 72 ans). Par contre, chez les patients sans thrombose, les D-dimères sont plus élevés en fonction de l'âge comme attendu.

**Exemple 13: Performances Diagnostiques de la Méthode selon l'Invention par Groupe de Patients**

Le protocole utilisé dans cet exemple est le Protocole A. Les étapes e) et h) de la méthode utilisée dans cet exemple sont celles de la deuxième version de la méthode.

Le taux des D-dimères qui résultent de la dégradation de la fibrine intravasculaire R, a été calculé selon l'étape e') en ajustant le taux de D-dimères de l'échantillon en fonction du taux des D-dimères générés par l'inflammation, et en corrigeant ce taux ajusté pour les D-dimères faibles (<4 μg/mL). Le taux de D-dimères générés par l'hyper fibrinolyse a été calculé selon l'étape g'), pour chacun des échantillons classés en fonction de l'inflammation. Puis le ratio TA/TFF a été comparé par rapport à un seuil, afin d'exclure ou de diagnostiquer une thrombose chez le patient, ou d'orienter sur un état d'activation de la coagulation chez le patient.

Les performances diagnostiques de la méthode de l'invention ont été déterminées sur un total de 796 patients avec suspicion de thrombose en utilisant la deuxième version de la méthode de l'invention. Les résultats obtenus par la méthode de l'invention sur les 8,3% de patients avec une thrombose, les 7,3% de patients avec un cancer, sur les patients présentant une prédisposition à la thrombose, ceux présentant un état d'hyper coagulation et ceux avec un état d'activation de la coagulation sont présentés dans les tableaux 18 à 21 ci-dessous.

***Diagnostic de l'Embolie Pulmonaire (EP) et de la Thrombose Veineuse Profonde (TVP)***

Les performances diagnostiques de la méthode de l'invention obtenues sur 46 patients avec une embolie pulmonaire (EP), 16 patients avec une thrombose veineuse profonde (TVP), 4 patients avec à la fois une EP et une TVP dont 15 patients ont des antécédents (ATCD) de thrombose veineuse, sont décrites dans le tableau 18.

**Tableau 18.** Performances diagnostiques de la méthode de l'invention obtenues sur les 66 patients avec un diagnostic de thrombose parmi les 796 patients avec suspicion de thrombose.

| Diagnostic de thrombose | n | Ddi (μg/mL) | Inflammation (DDO) | Méthode de l'invention Ddi (μg/mL) | Performances Vrais Positifs (VP) |
|---|---|---|---|---|---|
| Positif EP+ | 46 | 5,2 0,68-30 | 4,8 2,6-9,9 | 1,43 0,52-7,64 | 97,8% VP 1 SSPE* sans gravité |
| Positif TVP+ | 16 | 4,1 0,77-13 | 4,4 2,6-6,6 | 1,28 0,55-5,05 | 100% VP |
| Positif EP+ TVP+ | 4 | 5,0 1,15-10 | 4,3 3,3-5,4 | 1,36 0,61-3,03 | 100% VP |
| ATCD EP ou TVP | 15 | 5,8 0,68-20 | 4,5 2,6-8,4 | 1,83 0,54-7,24 | 100% VP |
| Négatif TV superficielle | 3 | 1,1 | 4,0 | 0,60 | 2 Faux Positifs |
| Négatif insuffisance veineuse | 2 | 0,52 | 4,2 | 0,50 | 2 Vrais Négatifs |
| * SSPE: embolie pulmonaire sous-segmentaire. | | | | | |

Les résultats obtenus montrent que la méthode selon l'invention permet un diagnostic de près de 100% des patients atteints de la maladie thromboembolique veineuse, y compris en cas d'infarctus pulmonaire dont le niveau d'inflammation est élevé et en cas d'embolie pulmonaire sous-segmentaire isolée, de faible gravité, comme le montre le Tableau 19. A noter que les taux de D-dimère qui résultent de la dégradation de la fibrine intravasculaire déterminés sont plus élevés dans le cas d'antécédent (ATCD) de maladie thromboembolique veineuse (embolie pulmonaire ou thrombose veineuse profonde), comme le montre le Tableau 18.

**Tableau 19.** Performances diagnostiques de la méthode de l'invention obtenues sur les 66 patients avec un diagnostic de thrombose parmi les 796 patients avec suspicion de thrombose.

| Diagnostic de thrombose | N | D-dimères | Inflammation (DDO) N : 2 à 4.5 g/L | Méthode de l'invention Ddi ($\mu$g/mL) VP=Vrais Positifs |
|---|---|---|---|---|
| EP massive, bilatérale ou segmentaire | 24 | 7.1 1.2-30.0 | 4.8 2.8-8.4 | 1.91 0.57-7.52 (100% VP) |
| EP distale | 7 | 1.7 0.89-2.77 | 5.4 3.7-9.9 | 0.60 0.55-0.65 (100% VP= |
| Infarctus pulmonaire | 7 | 2.5 1.1-5.1 | 5.8 3.1-8.4 | 0.73 0.52-1.62 (100% VP) |
| SSPE associée à EP | 6 | 3.3 1.15-5.1 | 5.0 3.1-7.5 | 0.80 0.52-1.62 (100% VP) |
| SSPE isolée | 6 | 1.9 0.68-3.8 | 4.2 2.6-6.9 | 0.62 0.54-0.58 (1 Faux Négatif) |

***Etats de Prédisposition à la Thrombose***

Les 113 patients présentant une prédisposition à la thrombose testés dans cette étude étaient des patients atteints d'un cancer connu (58 patients, dont 47 diagnostiqués négatifs pour MTEV, et 11 diagnostiqué positifs), des patients atteints d'un cancer non connu (21 patients, dont 17 diagnostiqués négatifs, et 4 diagnostiqués positifs), des femmes enceintes (14 patientes toutes diagnostiquées négatives), des femmes ayant accouché (4 patientes toutes diagnostiquées négatives), des patients en phase post-opératoire (2 patients tous diagnostiqués négatifs), et des patients qui venaient d'effectuer un voyage de longue durée (14 patients, tous diagnostiqués négatifs, sauf 1 diagnostiqué positif).

**Tableau 20.** Performances diagnostiques de la méthode de l'invention obtenues sur 113 patients avec un état de prédisposition à la thrombose, dont 16 ont un diagnostic de thrombose, parmi les 796 patients avec suspicion de thrombose.

| Patients avec un état de prédisposition à la thrombose | Diagnostic de thrombose | n | Ddi | Inflammation (DDO) | Méthode de l'invention Ddi ($\mu$g/mL) | Performances |
|---|---|---|---|---|---|---|
| Cancer connu (n=58) | **Positif** | **11** | **5,0** | **4,7** | **1,50** | **100% Vrai Positifs** |
| | Négatif | 47 | 1,7 | 4,7 | 0,68 | 53% Exclusion |
| Cancer non connu (n=21) | **Positif** | **4** | **6,4** | **4,2** | **2,0** | **100% Vrai Positifs** |
| | Négatif | 17 | 2,0 | 4,8 | 0,67 | 48% Exclusion |
| Grossesse | Négatif | 14 | 0,78 | 3,8 | 0,56 | 93% Exclusion |
| Accouchement | Négatif | 4 | 0,67 | 3,5 | 0,54 | 1 00% Exclusion |
| Post opératoire | Négatif | 2 | 1,40 | 3,9 | 0,61 | 1 00% Exclusion |

(suite)

| Patients avec un état de prédisposition à la thrombose | Diagnostic de thrombose | n | Ddi | Inflammation (DDO) | Méthode de l'invention Ddi ($\mu$g/mL) | Performances |
|---|---|---|---|---|---|---|
| Voyage N=14 | **Positif** | **1** | **2,23** | **8,4** | **0,58** | **100% Vrai Positif** |
| | Négatif | 13 | 0,76 | 3,7 | 0,54 | 93% Exclusion |

Les résultats obtenus chez les patients avec un état de prédisposition à la thrombose montrent clairement que la méthode selon l'invention permet un diagnostic de 100% des patients atteints de la maladie thromboembolique veineuse. La méthode de l'invention permet également l'exclusion de plus de 93% des patients non-cancéreux, comme le montre le Tableau 20. Chez les patients atteints de cancer, la méthode de l'invention permet l'exclusion de 50% des patients contre 22% avec la méthode habituelle de détermination des D-dimères.

*Etats d'Hyper coagulation*

Les 436 patients présentant un état d'hyper coagulation testés dans cette étude étaient des patients atteints de thrombophilie (4 patients, tous diagnostiqués négatifs pour MTEV, sauf 1 diagnostiqué positif), des patients atteints d'insuffisance rénale (26 patients, tous diagnostiqués négatifs sauf 1 diagnostiqué positif), des patients ayant subi un trauma, une chute ou une fracture (13 patients, tous diagnostiqués négatifs), et un groupe de sujets de 50 ans ou plus (393 sujets, dont 357 diagnostiqués négatifs et 36 diagnostiqués positifs).

**Tableau 21.** Performances diagnostiques de la méthode de l'invention obtenues sur 436 patients avec un état d'hyper coagulation, dont 38 ont un diagnostic de thrombose, parmi les 796 patients avec suspicion de thrombose.

| Patients avec un état d'hyper coagulation | Diagnostic de thrombose | n | Ddi | Inflammation (DDO) | Méthode de l'invention Ddi ($\mu$g/mL) | Performances |
|---|---|---|---|---|---|---|
| Thrombophilie | **Positif** | **1** | **4,0** | **4,8** | **0,82** | **Vrai Positif** |
| | Négatif | 3 | 0,37 | 3,8 | 0,48 | 1 00% Exclusion |
| Insuffisance rénale (IR) néphropathie | **Positif** | **1** | **2,2** | **8,4** | **0,58** | **Vrai Positif** |
| | Négatif | 25 | 3,0 | 5,1 | 0,78 | 50% Exclusion |
| Trauma, chute, fractures | Négatif | 13 | 1,86 | 3,9 | 0,70 | 77% Exclusion |
| Âge $\geq$ 50 ans | **Positif** | **36** | **2,25** | **5,0** | **0,65** | **100% Vrai Positifs** |
| Âge moyen: 69 ans | Négatif | 357 | 1,21 | 4,4 | 0,58 | 77% Exclusion vs 94% pour < 50 ans |

Les résultats obtenus chez les patients présentant un état d'hypercoagulabilité montrent clairement que la méthode selon l'invention permet un diagnostic de 100% des patients atteints de la maladie thromboembolique veineuse. La méthode de l'invention permet également l'exclusion de plus de 75% des patients, y compris chez les patients âgés, comme le montre le Tableau 21. Parmi les patients âgés non-exclus par la méthode de l'invention, 1/3 ont un cancer et 2/3 souffrent d'une insuffisance rénale ou d'une activation de la coagulation.

*Etats d'Activation de la Coagulation*

Les 144 patients avec un état d'activation de la coagulation testés dans cette étude étaient des patients atteints d'infections ou de sepsis (5 patients, tous diagnostiqués négatifs pour MTEV), des patients atteints de pneumopathie, bronchite ou insuffisance respiratoire (71 patients, tous diagnostiqués négatifs sauf 1 diagnostiqué positif), des patients atteints de maladies inflammatoires (6 patients, tous diagnostiqués négatifs), des patients atteints de gastrites (7 patients, tous diagnostiqués négatifs), des patients atteints de cardiomyopathies (45 patients, tous diagnostiqués négatifs sauf 1 diagnostiqué positif), et des patients avec des antécédents d'accident vasculaire cérébral (10 patients, tous diagnostiqués négatifs).

**Tableau 22.** Performances diagnostiques de la méthode de l'invention obtenues sur 144 patients avec un état d'activation de la coagulation, dont 2 ont un diagnostic de thrombose, parmi les 796 patients avec suspicion de thrombose.

| Patients avec un état d'hyper coagulation | Diagnostic de thrombose | n | Ddi | Inflammation (DDO) | Méthode de l'invention Ddi ($\mu$g/mL) | Performances |
|---|---|---|---|---|---|---|
| Infections, sepsis | Négatif | 5 | 2,88 | 5,3 | 0,80 | 1 00% Exclusion |
| Pneumopathie, bronchite, insuff. respiratoire | **Positif** | **1** | **2.76** | **9,9** | **0,56** | **Vrai Positif** |
| | Négatif | 70 | 0,86 | 5,0 | 0,53 | 83% Exclusion |
| Maladies inflam. | Négatif | 6 | 2,16 | 4,3 | 0,85 | 1 00% Exclusion |
| Gastrites | Négatif | 7 | 0,72 | 4,3 | 0,51 | 86% Exclusion |
| Cardiomyopathi es (SCA, Ins. cardiaque,... ) | **Positif** | **1** | **2,77** | **4,1** | **0,65** | **Vrai Positif** |
| | Négatif | 44 | 1,19 | 4,3 | 0,57 | 66% Exclusion |
| ATCD AVC | Négatif | 10 | 0,74 | 4,0 | 0,53 | 90% Exclusion |

Les résultats obtenus chez les 144 patients avec un état d'activation de la coagulation montrent clairement que la méthode selon l'invention permet un diagnostic de 100% des patients atteints de la maladie thromboembolique veineuse ainsi que l'exclusion de 80% des patients négatifs (à l'exception des patients âgés qui ont une cardiomyopathie d'âge moyen: 80 ans), comme le montre le Tableau 22.

**Revendications**

1. Méthode de dosage des D-dimères spécifiques de la maladie thromboembolique veineuse dans un échantillon sanguin de patient, ladite méthode comprenant d'une part le dosage des D-dimères de l'échantillon pour obtenir le taux de D-dimères dans l'échantillon ($Ddi_E$), et d'autre part, la mesure dynamique de la formation de fibrine de ce même échantillon, ladite mesure dynamique comprenant les étapes suivantes:

   a) initier l'activation de la coagulation dans l'échantillon sans la déclencher;
   b) incuber le mélange obtenu à l'étape a), et déclencher, dans l'échantillon incubé, la génération de thrombine et la formation d'un caillot de fibrine;
   c) mesurer la variation temporelle d'au moins une propriété de l'échantillon obtenu en b), dans lequel se forme le caillot de fibrine;
   d) établir le profil de formation du caillot de fibrine analysé en c), et extraire de ce profil le temps de formation de la fibrine (TFF) mesuré au point d'inflexion de la tangente de la courbe du profil, la valeur de la propriété ($Vp_{(TA)}$) de l'échantillon mesurée au temps d'atteinte (TA) du plateau de polymérisation de la fibrine;
   e) calculer le taux des D-dimères résultant de la dégradation de la fibrine intravasculaire (R):

      e1) en ajustant le taux de D-dimères de l'échantillon ($Ddi_E$) en fonction du taux des D-dimères générés par l'hyper coagulation en utilisant TFF déterminé en d), pour obtenir le taux de D-dimères ajustés en fonction de l'hyper coagulation ($Ddi_{E/HC}$), et
      e2) en ajustant le taux de D-dimères ajustés $Ddi_{E/HC}$ obtenu en e1) en fonction du taux des D-dimères générés par l'inflammation en utilisant $Vp_{(TA)}$ déterminé en d), pour obtenir R;

   f) comparer le taux de D-dimères résultant de la dégradation de la fibrine R obtenu en e), par rapport à un seuil, de préférence au seuil de 0,5 $\mu$g/mL;
   g) déterminer le taux des produits de dégradation du fibrinogène générés par l'hyper fibrinolyse ($PDFg_{(HF)}$)

présente dans les états d'activation de la coagulation en utilisant le taux de D-dimères résultant de la dégradation de la fibrine, R, obtenu en e) et le taux de D-dimères de l'échantillon (Ddi$_E$).

h) comparer le taux PDFg$_{(HF)}$ obtenu en g) par rapport à un seuil, et comparer le temps de formation de la fibrine (TFF) obtenu en d) par rapport à un seuil.

**2.** Méthode selon la revendication 1, **caractérisée en ce que** dans l'étape e), calculer le taux R exprimé en unités équivalents au fibrinogène initial (FEU) est réalisé:

e1) en calculant le taux de D-dimères ajustés en fonction de l'hyper coagulation (Ddi$_{E/HC}$) en utilisant l'équation suivante:

$$\text{Ddi}_{E/HC} = \text{Ddi}_E \ \text{x} \ \frac{\text{TFF}}{\text{Temps Témoin}}$$

où le Temps Témoin est le temps moyen de formation du caillot de fibrine d'échantillons de patients sains ne présentant pas de suspicion de thrombose, mesuré selon les étapes a)-d), et

e2) en calculant le taux R en utilisant l'équation suivante:

$$R = \frac{\text{Ddi}_{E/HC}}{[\text{Fib}]_{(Vp(TA))}}$$

où $[\text{Fib}]_{(Vp(TA))}$ est la concentration en fibrinogène déduite pour la valeur de la propriété Vp$_{(TA)}$ sur une courbe étalon d'équation:

$$y = a \ \ln(x) - b,$$

où:

y est la valeur de la propriété mesurée au temps d'atteinte (TA) du plateau de polymérisation de la fibrine,
x est la concentration en fibrinogène,
a et b sont les constantes de l'équation logarithmique qui relie le niveau du plateau de fibrine, et la concentration en fibrinogène,
la courbe étalon ayant été établie pour des échantillons sanguins dont la concentration en fibrinogène a été déterminée et dont la valeur de la propriété (Vp$_{(TA)}$) a été déterminée par les étapes a)-d).

**3.** Méthode selon la revendication 1 ou la revendication 2, **caractérisée en ce que** dans l'étape f), un taux R obtenu en e), inférieur au seuil, préférablement au seuil de 0,5 $\mu$g/mL, permet d'exclure une thrombose chez le patient et un taux R obtenu en e), supérieur au seuil, préférablement au seuil de 0,5 $\mu$g/mL, est indicatif de la possibilité d'une thrombose chez le patient.

**4.** Méthode selon l'une quelconque des revendications 1 à 3, **caractérisée en ce que** l'étape g) comprend des étapes consistant à:

g1) déterminer le taux de PDFg correspondant au taux de D-dimères dans l'échantillon (Ddi$_E$) sur une courbe étalon établie en utilisant des échantillons sanguins avec des taux de PDFg et des taux de D-dimères connus,
g2) déterminer le taux de PDFg correspondant au taux de D-dimères ajusté (R) obtenu à l'étape e) sur la courbe étalon utilisée en g1), et
g3) déterminer le taux de PDFg générés par l'hyper fibrinolyse (PDFg$_{(HF)}$) en soustrayant le taux de PDFg obtenu en g2) du taux de PDFg obtenu en g1).

**5.** Méthode selon la revendication 4, **caractérisée en ce que** l'étape h) comprend des étapes consistant à:

h1) comparer PDFg$_{(HF)}$ à un seuil, notamment un seuil de 1 $\mu$g/mL, où PDFg$_{(HF)}$ inférieur au seuil ou négatif permet d'exclure la thrombose chez le patient, et PDFg$_{(HF)}$ supérieur au seuil est indicatif d'une possibilité de thrombose chez le patient,

h2) comparer TFF à un seuil, notamment un seuil égal au [Temps Témoin de e1) - 1 écart type], plus notamment encore un seuil de 120 secondes pour un Temps Témoin de 135 secondes, où TFF inférieur au seuil est indicatif d'un patient sans thrombose mais présentant un état d'activation aigu de la coagulation, et un TFF supérieur au seuil est indicatif d'une thrombose chez le patient.

**6.** Méthode selon la revendication 5, **caractérisée en ce que**:

- lorsque qu'une thrombose a été diagnostiquée à l'étape h2), le taux de D-dimères spécifiques de la maladie thromboembolique veineuse ($Ddi_{MTEV}$) est le taux de D-dimères, R, obtenu à l'étape e),
- lorsqu'une thrombose a été exclue aux étapes h1) et h2) mais que le taux R obtenu à l'étape e) est supérieur à un seuil, préférablement au seuil de 0,5 $\mu$g/mL, la méthode comprend en outre une étape consistant à calculer le taux de D-dimères spécifiques de la maladie thromboembolique veineuse ($Ddi_{MTEV}$) en utilisant l'équation suivante:

$$Ddi_{MTEV} = 0{,}5 \text{ x } \frac{R}{Ddi_{E}}$$

où DdiE est le taux de D-dimères dans l'échantillon.

**7.** Méthode de dosage des D-dimères spécifiques de la maladie thromboembolique veineuse dans un échantillon sanguin de patient, ladite méthode comprenant d'une part le dosage des D-dimères de l'échantillon pour obtenir le taux de D-dimères dans l'échantillon ($Ddi_{E}$) et d'autre part, la mesure dynamique de la formation de fibrine de ce même échantillon, ladite mesure dynamique comprenant les étapes suivantes:

a) initier l'activation de la coagulation dans l'échantillon sans la déclencher;
b) incuber le mélange obtenu à l'étape a), et déclencher, dans l'échantillon incubé, la génération de thrombine et la formation d'un caillot de fibrine;
c) mesurer la variation temporelle d'au moins une propriété de l'échantillon obtenu en b), dans lequel se forme le caillot de fibrine;
d) établir le profil de formation du caillot de fibrine analysé en c), et extraire de ce profil le temps de formation de la fibrine (TFF) mesuré au point d'inflexion de la tangente de la courbe du profil, et la valeur de la propriété ($Vp_{(TA)}$) de l'échantillon mesurée au temps d'atteinte (TA) du plateau de polymérisation de la fibrine;
e') calculer le taux des D-dimères résultant de la dégradation de la fibrine intravasculaire (R):

e'1) en ajustant le taux de D-dimères de l'échantillon, $Ddi_{E}$, en fonction du taux des D-dimères générés par l'inflammation en utilisant $Vp_{(TA)}$ déterminé en d), pour obtenir le taux de D-dimères ajustés pour l'inflammation ($Ddi_{E/I}$); et
e'2) en corrigeant le taux de D-dimères ajustés pour l'inflammation ($Ddi_{E/I}$) obtenu en e'1) pour les taux de D-dimères faibles, et

classer l'échantillon du patient en fonction de l'inflammation;
f') comparer le taux de D-dimères résultant de la dégradation de la fibrine R obtenu en e), par rapport à un seuil, de préférence au seuil de 0,5 $\mu$g/mL;
g') déterminer le taux des D-dimères générés par l'hyper fibrinolyse ($Ddi_{(HF)}$) en utilisant le taux de D-dimères dans l'échantillon ($Ddi_{E}$) et le taux R obtenu en e') ou en utilisant le taux de D-dimères ajustés en fonction de l'inflammation, $Ddi_{E/I}$, obtenu en e'1) et le taux R obtenu en e');
h') comparer le taux $Ddi_{(HF)}$ obtenu en g') par rapport à un seuil, et comparer le ratio TA/TTF par rapport à un seuil, où TA est le temps d'atteinte du plateau de polymérisation de fibrine obtenu en d), et TFF est le temps de formation de la fibrine obtenu en d).

**8.** Méthode selon la revendication 7, **caractérisée en ce que** dans l'étape e'), le taux R exprimé en unités équivalents au fibrinogène initial (FEU) est obtenu :

e'1) en calculant le taux de D-dimères ajustés en fonction de l'inflammation ($Ddi_{E/I}$) par l'équation suivante:

$$\mathrm{Ddi}_{E/I} = \frac{\mathrm{Ddi}_{E}}{[\mathrm{Fib}]_{(Vp(TA))}}$$

où $[\mathrm{Fib}]_{(Vp(TA))}$ est la concentration en fibrinogène déduite pour la valeur de la propriété $Vp_{(TA)}$ sur une courbe étalon d'équation :

$$y = a \ln(x) - b,$$

où:

y est la valeur de la propriété mesurée au temps d'atteinte (TA) du plateau de polymérisation de la fibrine,
x est la concentration en fibrinogène,
a et b sont les constantes de l'équation logarithmique qui relie le niveau du plateau de fibrine, et la concentration en fibrinogène,
la courbe étalon ayant été établie pour des échantillons sanguins dont la concentration en fibrinogène a été déterminée et dont la valeur de la propriété $Vp_{(TA)}$ a été déterminée par les étapes a)-d).

e'2) en corrigeant le taux $\mathrm{Ddi}_{E/I}$ obtenu en e'1) par l'équation suivante:

$$R = \mathrm{Ddi}_{E/I} + [0{,}5 - F_{\mathrm{Ddi\text{-}E}}]$$

où $F_{\mathrm{Ddi\text{-}E}}$ est un facteur de correction des taux de D-dimères faibles (< 4 $\mu$g/mL) dont la valeur correspond à la valeur du facteur de correction pour le taux de D-dimères de l'échantillon ($\mathrm{Ddi}_{E}$) sur la courbe étalon d'équation:

$$y = a\, x^{2} + b\, x + c$$

où y est le facteur de correction, F,
x est le taux de D-dimères, a, b et c sont les constantes de l'équation polynomiale qui relie le facteur de correction, et le taux de D-dimères,
la courbe étalon ayant été établie pour des échantillons sanguins dont le taux de D-dimères a été déterminée et pour lesquels le facteur de correction a été déterminé de façon empirique pour que le taux $\mathrm{Ddi}_{E/I}$ soit ramené au seuil de 0,5 $\mu$g/mL unités FEU (unités équivalentes au fibrinogène).

9. Méthode selon la revendication 7 ou la revendication 8, **caractérisée en ce que** dans l'étape e'), classer l'échantillon du patient en fonction de l'inflammation comprend:

- calculer le ratio $1/[\mathrm{Fib}]_{(Vp(TA))}$, où $\mathrm{Fib}]_{(Vp(TA))}$ est la concentration en fibrinogène déterminée en e1'); et
- classer l'échantillon du patient dans le groupe I des patients sans inflammation si le ratio $1/[\mathrm{Fib}]_{(Vp(TA))} > 0{,}20$, ou
- classer l'échantillon du patient dans le groupe II des patients avec inflammation si le ratio $1/[\mathrm{Fib}]_{(Vp(TA))} \leq 0{,}20$.

10. Méthode selon l'une quelconque des revendications 7 à 9, **caractérisée en ce que** dans l'étape f'), un taux R obtenu en e'), inférieur au seuil, préférablement au seuil de 0,5 $\mu$g/ml, permet d'exclure une thrombose chez le patient, et un taux R obtenu en e'), supérieur au seuil, préférablement au seuil de 0,5 $\mu$g/mL, est indicatif de la possibilité d'une thrombose chez le patient.

11. Méthode selon la revendication 9 ou la revendication 10, **caractérisée en ce que** dans l'étape g'), le taux des D-dimères générés par l'hyper fibrinolyse ($\mathrm{Ddi}_{HF}$) est calculé:

g'1) comme le ratio entre le taux R déterminé à l'étape e') et le taux de D-dimères de l'échantillon, $\mathrm{Ddi}_{E}$, en utilisant l'équation:

$$Ddi_{HF} = (R/Ddi_E)_{patient} = \frac{R}{Ddi_E}$$

g'2) comme le ratio entre le taux R déterminé à l'étape e') et le taux $Ddi_{E/I}$, obtenu en e'1), en utilisant l'équation:

$$Ddi_{HF} = (R/Ddi_{E/I})_{patient} = \frac{R}{Ddi_{E/I}}$$

**12.** Méthode selon la revendication 11, **caractérisée en ce que** dans l'étape h'):

le taux $(R/Ddi_E)_{patient}$ obtenu en g'1) est comparé à un seuil:

h'1) en déterminant pour le taux $Ddi_E$ de l'échantillon, la valeur du ratio $(R/Ddi_E)_{étalon}$ sur une courbe étalon d'équation:

$$y = a\,x^{-b}$$

où x est le taux de D-dimères,
y est le ratio entre le taux de D-dimères qui résultent de la dégradation de la fibrine intravasculaire et le taux de D-dimères, $(R/Ddi_E)$, a, et b sont les constantes de l'équation qui relie le ratio $R/Ddi_E$, et le taux de D-dimères,
la courbe étalon ayant été établie:

- en utilisant, si l'échantillon du patient a été classé dans le groupe I: des échantillons sanguins classés dans le groupe I par l'étape e') et dont le taux de D-dimères est connu ou a été déterminé et le taux R a été obtenu par les étapes a)-e'2); et
- en utilisant, si l'échantillon du patient a été classé dans le groupe II: des échantillons sanguins classés dans le groupe II par l'étape e') et dont le taux de D-dimères est connu ou a été déterminé et le taux R a été obtenu par les étapes a)-e'2); et

h"1) en comparant la valeur du taux de D-dimères générés par l'hyper fibrinolyse $(R/Ddi_E)_{patient}$ obtenu en g'1) avec la valeur du ratio $(R/Ddi_E)_{étalon}$ obtenu en h'1), où:
$(R/Ddi_E)_{patient}$ inférieur au ratio $(R/Ddi_E)_{étalon}$ permet d'exclure la thrombose chez le patient, et $(R/Ddi_E)_{patient}$ supérieur ou égal au ratio $(R/Ddi_E)_{étalon}$ est indicatif d'une possibilité de thrombose chez le patient;

le taux $(R/Ddi_{E/I})_{patient}$ obtenu en g'2) est comparé à un seuil:

h'2) en déterminant pour le taux $Ddi_E$ de l'échantillon, la valeur du ratio $(R/Ddi_{E/I})_{étalon}$ sur une courbe étalon d'équation:

$$y = a\,x^{-b}$$

où x est le taux de D-dimères,
y est le ratio entre le taux de D-dimères qui résultent de la dégradation de la fibrine intravasculaire et le taux de D-dimères ajustés en fonction de l'inflammation, $(R/Ddi_{E/I})$,
a, et b sont les constantes de l'équation qui relie le ratio $R/Ddi_{E/I}$, et le taux de D-dimères,
la courbe étalon ayant été établie:

- en utilisant, si l'échantillon du patient a été classé dans le groupe I: des échantillons sanguins classés dans le groupe I par l'étape e') et dont le taux de D-dimères est connu ou a été déterminé, le taux $Ddi_{E/I}$ a été obtenu par les étapes a)-e' 1), et le taux R a été obtenu par les étapes a)-e'2); et
- en utilisant, si l'échantillon du patient a été classé dans le groupe II: des échantillons sanguins classés dans le groupe II par l'étape e') et dont le taux de D-dimères est connu ou a été déterminé, le taux $Ddi_{E/I}$ a été obtenu par les étapes a)-e' 1), et le taux R a été obtenu par les étapes a)-e'2); et

h"2) en comparant la valeur du taux de D-dimères générés par l'hyper fibrinolyse $(R/Ddi_{E/I})_{patient}$ obtenu en g'2) avec la valeur du ratio $(R/Ddi_{E/I})_{étalon}$ obtenu en h'2), où:
$(R/Ddi_{E/I})_{patient}$ inférieur au ratio $(R/Ddi_{E/I})$étalon permet d'exclure la thrombose chez le patient, et où $(R/Ddi_{E/I})_{patient}$ supérieur ou égal au ratio $(R/Ddi_{E/I})_{étalon}$ est indicatif d'une possibilité de thrombose chez le patient.

**13.** Méthode selon la revendication 12, **caractérisée en ce que** dans l'étape h'), comparer le ratio TA/TTF par rapport à un seuil, comprend:

h'3) calculer le ratio TA/TFF où TA est le temps d'atteinte du plateau de polymérisation de la fibrine déterminé à l'étape d) et TFF est le temps de formation du caillot de fibrine déterminé à l'étape d); et
h"3) si l'échantillon du patient a été classé dans le groupe I:
comparer le ratio TA/TFF par rapport à un premier seuil, notamment à un premier seuil de 1,75, où

un ratio TA/TFF supérieur au premier seuil permet d'exclure la thrombose et de diagnostiquer une thrombophilie ou un état d'activation de la coagulation chez le patient classé dans le groupe I, et
un ratio TA/TFF inférieur ou égal au seuil est indicatif d'une thrombose chez le patient classé dans le groupe I;

si l'échantillon du patient a été classé dans le groupe II:
comparer le ratio TA/TFF par rapport à un premier seuil, notamment à un premier seuil de 1,65, où

un ratio TA/TFF supérieur au premier seuil permet d'exclure la thrombose et de diagnostiquer une thrombophilie ou d'un état d'activation de la coagulation chez le patient classé dans le groupe II, et
un ratio TA/TFF inférieur ou égal au seuil est indicatif d'une thrombose chez le patient classé dans le groupe II.

**14.** Méthode selon la revendication 13, **caractérisée en ce que**:

- lorsqu'une thrombose a été diagnostiquée à l'étape h"3), le taux de D-dimères spécifiques de la maladie thromboembolique veineuse ($Ddi_{MTEV}$) est le taux de D-dimères, R, obtenu à l'étape e'),
- lorsqu'une thrombose est exclue aux étapes h"1), h"2) et h"3) mais que le taux R obtenu à l'étape e') est supérieur au seuil, préférablement au seuil de 0,5 $\mu$g/mL, la méthode comprend en outre une étape consistant à calculer le taux de D-dimères spécifiques de la maladie thromboembolique veineuse ($Ddi_{MTEV}$) en utilisant l'équation suivante:

$$Ddi_{MTEV} = 0,5 \times \frac{R}{Ddi_E}$$

où DdiE est le taux de D-dimères dans l'échantillon.

**15.** Méthode selon l'une quelconque des revendications 1 à 14, **caractérisée en ce que** l'échantillon sanguin a un volume compris entre 1 $\mu$L et 300 $\mu$L, de préférence compris entre 50 $\mu$L et 200 $\mu$L.

**16.** Méthode selon l'une quelconque des revendications 1 à 15, **caractérisée en ce que** l'échantillon sanguin est non-dilué.

**17.** Méthode selon l'une quelconque des revendications 1 à 16, **caractérisée en ce que** le dosage des D-dimères de l'échantillon est effectué selon une méthode immuno-turbidimétrique ou immuno-enzymatique.

**18.** Méthode selon l'une quelconque des revendications 1 à 17, **caractérisée en ce que** l'étape a) est réalisée en mélangeant l'échantillon sanguin du patient avec du facteur tissulaire et optionnellement des phospholipides, préférablement en mélangeant l'échantillon sanguin du patient avec du facteur tissulaire et des phospholipides.

**19.** Méthode selon la revendication 18, **caractérisée en ce que** le facteur tissulaire de l'étape a) est présent en une concentration comprise entre 0,5 et 5 pM, de préférence 2 pM.

**20.** Méthode selon la revendication 18 ou la revendication 19, **caractérisée en ce que** le mélange de l'étape a) comprend des ions calcium.

**21.** Méthode selon l'une quelconque des revendications 1 à 20, **caractérisée en ce que** l'étape b) comprend l'incubation du mélange obtenu à l'étape a) pendant un temps compris entre 20 secondes et 400 secondes, de préférence compris entre 60 secondes et 300 secondes, à une température entre 30°C et 40°C.

**22.** Méthode selon l'une quelconque des revendications 1 à 21, **caractérisée en ce que**, dans l'étape b), déclencher la génération de thrombine et la formation d'un caillot de fibrine est effectué en ajoutant des ions calcium à l'échantillon incubé.

**23.** Méthode selon l'une quelconque des revendications 1 à 22, **caractérisée en ce que** l'échantillon sanguin de patient est un échantillon de plasma.

**24.** Méthode selon la revendication 23, **caractérisée en ce que** l'échantillon de plasma est un échantillon de plasma pauvre en plaquettes.

**25.** Méthode selon l'une quelconque des revendications 23 à 24, **caractérisée en ce que**, dans l'étape c), mesurer la variation temporelle d'au moins une propriété de l'échantillon obtenu en b) est effectué en mesurant la variation temporelle de la densité optique (DDO) à une longueur d'onde comprise entre 350 et 800 nm, de préférence à la longueur d'onde de 540 nm.

**26.** Méthode selon la revendication 25, **caractérisée en ce que** la mesure de la densité optique de l'étape c) se fait à la même longueur d'onde que celle utilisée pour le dosage des D-dimères, 540 nm.

**27.** Méthode selon l'une quelconque des revendications 1 à 22, **caractérisée en ce que** l'échantillon sanguin de patient est un échantillon de sang total.

**28.** Méthodes selon la revendication 27, **caractérisée en ce que** l'échantillon de sang total est un échantillon de sang total citraté.

**29.** Méthode selon la revendication 27 ou la revendication 28, **caractérisée en ce que** mesurer la variation temporelle d'au moins une propriété de l'échantillon obtenu en b) est effectué par thromboélastographie, rhéométrie ou par analyse d'images.

**30.** Méthode selon l'une quelconque des revendications 1 à 29, **caractérisée en ce qu'**au moins les étapes c) et d) sont réalisées sur un automate de diagnostic ou sur un analyseur de biologie délocalisée, de préférence sur un analyseur de coagulation.

**31.** Méthode *in vitro* de diagnostic de la maladie thromboembolique veineuse (MTEV) chez un patient, comprenant des étapes consistant à:

- effectuer un dosage des D-dimères spécifiques de MTEV dans un échantillon sanguin du patient en utilisant une méthode selon l'une quelconque des revendications 3, 5, 9, 12, 13, et 14; et
- fournir un diagnostic concernant le patient.

**32.** Méthode *in vitro* de diagnostic selon la revendication 31, **caractérisée en ce que** le diagnostic concernant le patient est (i) exclusion de la thrombose, (ii) état d'activation aigu de la coagulation, ou (iii) thrombose.

**33.** Méthode *in vitro* de diagnostic selon la revendication 31, **caractérisée en ce que** si le patient est une personne âgée, un patient atteint de cancer, un patient atteint d'une infection ou un patient atteint d'une thrombophilie, le dosage des D-dimères spécifiques de MTEV est effectuée en utilisant une méthode selon l'une quelconque des revendications 12, 13 et 14.

**34.** Méthode *in vitro* de diagnostic selon la revendication 32, **caractérisée en ce que** le diagnostic concernant le patient est (i) exclusion de la thrombose, (ii) état d'activation aigu de la coagulation, (iii) thrombophilie, ou (iv) thrombose.

**35.** Méthode *in vitro* de diagnostic selon la revendication 34, **caractérisée en ce que** le diagnostic concernant le patient est une thrombose de faible gravité ou thrombose en cas d'infarctus pulmonaire.

36. Méthode selon l'une quelconque des revendications 6, 14 et 15-30, **caractérisée en ce qu'**un taux élevé de D-dimères spécifiques de la maladie thromboembolique veineuse, $Ddi_{MTEV}$, est représentatif de l'étendue de l'embolie pulmonaire ou de la thrombose veineuse profonde.

**Patentansprüche**

1. Verfahren zur Bestimmung von D-Dimeren, die spezifisch für eine venöse thromboembolische Erkrankung sind, in einer Blutprobe eines Patienten, wobei das Verfahren einerseits die Bestimmung der D-Dimere der Probe umfasst, um den Wert der D-Dimere in der Probe ($Ddi_E$) zu erhalten, und andererseits die dynamische Messung der Fibrinbildung in der gleichen Probe umfasst, wobei die dynamische Messung die folgenden Schritte umfasst:

   a) Initiieren der Gerinnungsaktivierung in der Probe, ohne diese auszulösen;
   b) Inkubieren der in Schritt a) erhaltenen Mischung und Auslösen der Bildung von Thrombin und der Bildung eines Fibringerinnsels in der inkubierten Probe;
   c) Messen der zeitlichen Variation mindestens einer Eigenschaft der in b) erhaltenen Probe, in der sich das Fibringerinnsel bildet;
   d) Ermitteln des Profils der Bildung des in c) analysierten Fibringerinnsels und Extraktion der Fibrinbildungszeit (TFF) aus diesem Profil, gemessen am Wendepunkt der Tangente der Profilkurve, des Wertes der Eigenschaft ($Vp_{(TA)}$) der Probe, gemessen zum Zeitpunkt des Erreichens (TA) des Fibrinpolymerisationsplateaus;
   e) Berechnen des Wertes der D-Dimere, die sich aus dem Abbau des intravaskulären Fibrins (R) ergeben, durch:

   e1) Einstellen des D-Dimer-Wertes der Probe ($Ddi_E$) entsprechend des Wertes der durch Hyperkoagulation erzeugten D-Dimere unter Verwendung der in d) bestimmten TFF, um den Wert der entsprechend der Hyperkoagulation angepassten D-Dimere ($Ddi_{E/HC}$) zu erhalten, und
   e2) Anpassen des in e1) erhaltenen D-Dimer-Wertes $Ddi_{E/HC}$ entsprechend des Wertes der durch die Entzündung erzeugten D-Dimere unter Verwendung des in d) bestimmten $Vp_{(TA)}$, um R zu erhalten;

   f) Vergleichen des in e) erhaltenen Wertes der aus dem Fibrinabbau R resultierenden D-Dimere mit einem Schwellenwert, bevorzugt mit dem Schwellenwert 0,5 $\mu$g/ml;
   g) Bestimmen des Wertes der durch Hyperfibrinolyse erzeugten Fibrinogenabbauprodukte ($PDFg_{(HF)}$), die in den Koagulationsaktivierungszuständen vorliegen, unter Verwendung des in e) erhaltenen Wertes der aus dem Fibrinabbau R resultierenden D-Dimere und des D-Dimer-Wertes der Probe ($Ddi_E$);
   h) Vergleichen des in g) erhaltenen $PDFg_{(HF)}$-Wertes mit einem Schwellenwert und Vergleichen der in d) ermittelten Fibrinbildungszeit (TFF) mit einem Schwellenwert.

2. Verfahren nach Anspruch 1, **dadurch gekennzeichnet, dass** in Schritt e) das Berechnen des Wertes R, ausgedrückt in Einheiten, die dem anfänglichen Fibrinogen (FEU) entsprechen, durchgeführt wird:

   e1) durch Berechnen des abhängig von der Hyperkoagulation angepassten D-Dimer-Wertes ($Ddi_{E/HC}$) unter Verwendung der folgenden Gleichung:

$$Ddi_{E/HC} = Ddi_E \quad \frac{TFF}{Kontrollzeit}$$

   wobei die Kontrollzeit die Durchschnittszeit der Bildung des Fibringerinnsels in Proben gesunder, nicht thromboseverdächtiger Patienten ist, die gemäß den Schritten a)-d) gemessen wurde, und
   e2) Berechnen des Wertes R unter Verwendung der folgenden Gleichung:

$$R = \frac{Ddi_{E/HC}}{[Fib]_{(Vp(TA))}}$$

   wobei $[Fib]_{(Vp(TA))}$ die Fibrinogenkonzentration ist, die für den Wert der Eigenschaft $Vp_{(TA)}$ auf einer Referenzkurve der folgenden Gleichung abgeleitet wird:

$$y = a \ln(x) - b,$$

wobei gilt:

y ist der Wert der Eigenschaft, gemessen zum Zeitpunkt des Erreichens (TA) des Fibrinpolymerisationsplateaus,
x ist die Fibrinogen-Konzentration,
a und b sind die Konstanten der logarithmischen Gleichung, die die Höhe des Fibrinplateaus und die Fibrinogen-Konzentration in Beziehung setzt,
wobei die Referenzkurve für Blutproben erstellt wurde, deren Fibrinogen-Konzentration bestimmt wurde und wobei der Wert der Eigenschaft ($Vp_{(TA)}$) durch die Schritte a)-d) bestimmt wurde.

3. Verfahren nach Anspruch 1 oder Anspruch 2, **dadurch gekennzeichnet, dass** in Schritt f) ein in e) erhaltener Wert R, der unter dem Schwellenwert, bevorzugt unter dem Schwellenwert 0,5 μg/ml liegt, erlaubt, eine Thrombose beim Patienten auszuschließen, und ein in e) erhaltener Wert R, der über dem Schwellenwert, bevorzugt über dem Schwellenwert 0,5 μg/ml liegt, auf die Möglichkeit einer Thrombose beim Patienten hinweist.

4. Verfahren nach einem der Ansprüche 1 bis 3, **dadurch gekennzeichnet, dass** Schritt g) die folgenden Schritte umfasst:

g1) Bestimmen des PDFg-Wertes, der dem D-Dimer-Wert in der Probe ($Ddi_E$) entspricht, auf einer Referenzkurve, die anhand von Blutproben mit bekannten PDFg-Werten und bekannten D-Dimer-Werten erstellt wurde,
g2) Bestimmen des PDFg-Wertes, der dem in Schritt e) erhaltenen angepassten D-Dimer-Wert (R) auf der in g1) verwendeten Referenzkurve entspricht, und
g3) Bestimmen des Wertes der durch Hyperfibrinolyse erzeugten PDFg ($PDFg_{(HF)}$) durch Subtraktion des in g2) erhaltenen PDFg-Wertes von dem in g1) erhaltenen PDFg-Wert.

5. Verfahren nach Anspruch 4, **dadurch gekennzeichnet, dass** Schritt h) die folgenden Schritte umfasst:

h1) Vergleichen von $PDFg_{(HF)}$ mit einem Schwellenwert, insbesondere einem Schwellenwert von 1 μg/ml, wobei ein $PDFg_{(HF)}$ unter dem Schwellenwert oder ein negativer Wert erlaubt, eine Thrombose beim Patienten ausschließen und ein $PDFg_{(HF)}$ über dem Schwellenwert auf die Möglichkeit einer Thrombose beim Patienten hinweist,
h2) Vergleichen von TFF mit einem Schwellenwert, insbesondere einem Schwellenwert gleich [Kontrollzeit aus e1) - 1 Standardabweichung], plus insbesondere ein Schwellenwert von 120 Sekunden für eine Kontrollzeit von 135 Sekunden, wobei ein TFF unter dem Schwellenwert auf einen Patienten ohne Thrombose hinweist, der aber einen akuten Zustand der Gerinnungsaktivierung aufweist, und wobei ein TFF über dem Schwellenwert auf eine Thrombose beim Patienten hinweist.

6. Verfahren nach Anspruch 5, **dadurch gekennzeichnet, dass**:

- wenn in Schritt h2) eine Thrombose diagnostiziert wurde, der Wert der für die venöse Thromboembolie spezifischen D-Dimere ($Ddi_{VTE}$), der in Schritt e) erhaltene Wert R ist,
- wenn in den Schritten h1) und h2) eine Thrombose ausgeschlossen wurde, der in Schritt e) erhaltene Wert R jedoch über einem Schwellenwert liegt, bevorzugt über dem Schwellenwert von 0,5 μg/ml, das Verfahren ferner einen Schritt zur Berechnen der Höhe der für die venöse Thromboembolie ($Ddi_{MTEV}$) spezifischen D-Dimere unter Verwendung der folgenden Gleichung umfasst:

$$Ddi_{MTEV} = 0,5 \times \frac{R}{Ddi_E}$$

wobei $Ddi_E$ der D-Dimer-Wert der Probe ist.

7. Verfahren zur Bestimmung von D-Dimeren, die spezifisch für die venöse thromboembolische Erkrankung ist, in einer Blutprobe eines Patienten, wobei das Verfahren einerseits das Bestimmen der D-Dimere in der Probe umfasst, um den D-Dimer-Wert in der Probe ($Ddi_E$) zu erhalten, und andererseits die dynamische Messung der Bildung von Fibrin in derselben Probe umfasst, wobei die dynamische Messung die folgenden Schritte umfasst:

> a) Initiieren der Gerinnungsaktivierung in der Probe, ohne diese auszulösen;
> b) Inkubieren der in Schritt a) erhaltenen Mischung und Auslösen der Bildung von Thrombin und der Bildung eines Fibringerinnsels in der inkubierten Probe;
> c) Messen der zeitlichen Variation mindestens einer Eigenschaft der in b) erhaltenen Probe, in der sich das Fibringerinnsel bildet;
> d) Ermitteln des Profils der Bildung des in c) analysierten Fibringerinnsels und Extraktion der Fibrinbildungszeit (TFF) aus diesem Profil, gemessen am Wendepunkt der Tangente der Profilkurve, des Wertes der Eigenschaft ($V_{P(TA)}$) der Pro-be, gemessen zum Zeitpunkt des Erreichens (TA) des Fibrinpolymerisationsplateaus;
> e') Berechnen des Wertes der D-Dimere, die sich aus dem Abbau des intravaskulären Fibrins (R) ergeben, durch:

>> e'1) Anpassen des D-Dimer-Wertes der Probe ($Ddi_E$) entsprechend dem Wert der durch die Entzündung erzeugten D-Dimere unter Verwendung des in d) bestimmten $V_{P(TA)}$, um den Wert der entsprechend der Entzündung angepassten D-Dimere ($Ddi_{E/I}$) zu erhalten, und
>> e'2) Korrigieren des in e'1) erhaltenen Wertes der entsprechend der Entzündung angepassten D-Dimere ($Ddi_{E/I}$) für niedrige D-Dimer-Werte, und Klassifizieren der Probe des Patienten entsprechend der Entzündung;

> f') Vergleichen des in e) erhaltenen Wertes der aus dem Fibrinabbau R resultierenden D-Dimere mit einem Schwellenwert, bevorzugt dem Schwellenwert 0,5 $\mu$g/ml;
> g') Bestimmen des Wertes der durch Hyperfibrinolyse erzeugten D-Dimere ($Ddi_{(HF)}$) unter Verwendung des Wertes der D-Dimere in der Probe ($Ddi_E$) und des in e') erhaltenen Wertes R oder unter Verwendung des in e') erhaltenen Wertes der entsprechend der Entzündung angepassten D-Dimere ($Ddi_{E/I}$) und des in e') erhaltenen Wertes R;
> h') Vergleichen des in g') ermittelten $Ddi_{(HF)}$-Wertes mit einem Schwellenwert und Vergleichen des Verhältnisses TA/TFF mit einem Schwellenwert, wobei TA für die in d) erhaltene Wartezeit des Fibrinpolymerisationsplateaus steht und TFF die in d) erhaltene Fibrinbildungszeit ist.

8. Verfahren nach Anspruch 7, **dadurch gekennzeichnet, dass** in Schritt e') der Wert R, ausgedrückt in Einheiten, die dem initialen Fibrinogen entsprechen (FEU), erhalten wird:

> e' 1) durch Berechnen des Wertes der entsprechend der Entzündung angepassten D-Dimere ($Ddi_{E/I}$) durch die folgende Gleichung:

$$ Ddi_{E/I} = \frac{Ddi_E}{[Fib]_{(Vp(TA))}} $$

wobei $[Fib]_{(Vp(TA))}$ die Fibrinogenkonzentration ist, die für den Wert der Eigenschaft $Vp_{(TA)}$ auf einer Referenzkurve der folgenden Gleichung abgeleitet wird:

$$ y = a \, 1n(x) - b, $$

wobei gilt:

> y ist der Wert der zum Zeitpunkt des Erreichens (TA) des Fibrinpolymerisationsplateaus gemessenen Eigenschaft,
> x ist die Fibrinogen-Konzentration,
> a und b sind die Konstanten der logarithmischen Gleichung, die die Höhe des Fibrinplateaus und die Fibrinogen-Konzentration in Beziehung setzt, wobei die Referenzkurve für Blutproben erstellt wurde, deren Fibrinogen-Konzentration bestimmt wurde und deren Wert der Eigenschaft $Vp_{(TA)}$ durch die Schritte a)-d) bestimmt wurde.

e'2) durch Korrigieren des in e'1) durch folgende Gleichung erhaltenen $Ddi_{E/I}$-Wertes:

$$R = Ddi_{E/I} + [0{,}5 - F_{Ddi-E}]$$

wobei $F_{Ddi-E}$ ein Korrekturfaktor für niedrige D-Dimerwerte (<4 µg/ml) ist, dessen Wert dem Wert des Korrekturfaktors für den D-Dimerwert der Probe ($Ddi_E$) auf der Referenzkurve folgender Gleichung entspricht:

$$y = a\,x^2 + b\,x + c,$$

wobei gilt

y ist der Korrekturfaktor, F,
x ist der D-Dimer-Wert,
a, b und c sind die Konstanten der Polynomgleichung, die den Korrekturfaktor und den D-Dimer-Wert verbindet,
wobei die Referenzkurve für Blutproben erstellt wurde, deren D-Dimer-Wert bestimmt wurde und für die der Korrekturfaktor empirisch ermittelt wurde, so dass der $Ddi_{E/I}$-Wert auf den Schwellenwert von 0,5 µg/ml FEU-Einheiten (Fibrinogen-äquivalente Einheiten) reduziert wird.

9. Verfahren nach Anspruch 7 oder Anspruch 8, **dadurch gekennzeichnet, dass** in Schritt e') die Klassifizierung der Patientenprobe abhängig von der Entzündung umfasst:

- Berechnen des Verhältnisses $1/[Fib]_{(Vp(TA))}$, wobei $[Fib]_{(Vp(TA))}$ die in e1') bestimmte Fibrinogenkonzentration ist; und
- Einstufen der Patientenprobe in Gruppe I der Patienten ohne Entzündung, wenn das Verhältnis $1/[Fib]_{(Vp(TA))}$ > 0,20 ist, oder
- Einstufen der Patientenprobe in Gruppe II der Patienten mit Entzündung, wenn das Verhältnis $1/[Fib]_{(Vp(TA))}$ ≤ 0,20 ist.

10. Verfahren nach einem der Ansprüche 7 bis 9, **dadurch gekennzeichnet, dass** in Schritt f') ein in e') erhaltener Wert R, der unter dem Schwellenwert, bevorzugt unter dem Schwellenwert 0,5 µg/ml, liegt, erlaubt, eine Thrombose beim Patienten auszuschließen, und ein in e') erhaltener Wert R, der über dem Schwellenwert, bevorzugt über dem Schwellenwert 0,5 µg/ml, liegt, auf die Möglichkeit einer Thrombose beim Patienten hinweist.

11. Verfahren nach Anspruch 9 oder Anspruch 10, **dadurch gekennzeichnet, dass** in Schritt g') der Wert der durch Hyperfibrinolyse erzeugten D-Dimere ($Ddi_{HF}$) berechnet wird:

g'1) als das Verhältnis zwischen dem in Schritt e') ermittelten Wert R und dem Wert der D-Dimere der Probe, $Ddi_E$, unter Anwendung der Gleichung:

$$Ddi_{HF} = (R/Ddi_E)_{patient} = \frac{R}{Ddi_E}$$

g'2) als das Verhältnis zwischen dem in Schritt e') ermittelten Wert R und dem in e1') erhaltenen Wert $Ddi_{E/I}$, unter Anwendung der Gleichung:

$$Ddi_{HF} = (R/Ddi_{E/I})_{patient} = \frac{R}{Ddi_{E/I}}$$

12. Verfahren nach Anspruch 11, **dadurch gekennzeichnet, dass** in Schritt h'):

der in g'1) erhaltene Wert $(R/Ddi_E)_{Patient}$ mit einem Schwellenwert verglichen wird:

h'1) indem für den Wert $Ddi_E$ der Probe der Wert des Verhältnisses $(R/Ddi_E)$ auf einer Referenzkurve der folgenden Gleichung bestimmt wird:

$$y=a\,x^{-b},$$

wobei gilt

x ist der D-Dimer-Wert,
y ist das Verhältnis zwischen dem Wert der D-Dimere, die aus dem intravaskulären Fibrinabbau resultieren und dem Wert der D-Dimere $(R/Ddi_E)$,
a und b sind die Konstanten der Gleichung, die das Verhältnis $R/Ddi_E$ und den D-Dimer-Wert in Beziehung setzt,
wobei die Referenzkurve wie folgt erstellt wurde:

- wenn die Patientenprobe in die Gruppe I eingestuft wurde unter Verwendung:

von Blutproben, die in Schritt e') in die Gruppe I eingestuft wurden und deren D-Dimer-Wert bekannt ist oder bestimmt wurde und deren R-Wert mittels der Schritte a)-e'2) ermittelt wurde; und

- wenn die Patientenprobe in die Gruppe II eingestuft wurde, unter Verwendung:

von Blutproben, die in Schritt e') in die Gruppe II eingestuft wurden und deren D-Dimer-Wert bekannt ist oder bestimmt wurde und deren R-Wert
durch die Schritte a)-e'2) erhalten wurde; und

h''1) durch Vergleichen des in g'1) erhaltenen Wertes der durch Hyperfibrinolyse erzeugten D-Dimere $(R/Ddi_E)_{Patient}$ mit dem Wert des in h'1) erhaltenen Verhältnisses $(R/Ddi_E)_{Referenz}$ wobei:
ein $(R/Ddi_E)_{Patient}$ kleiner als das Verhältnis $(R/Ddi_E)_{Referenz}$ eine Thrombose beim Patienten ausschließt, und ein $(R/Ddi_E)_{Patient}$ größer oder gleich dem Verhältnis $(R/Ddi_E)_{Referenz}$ auf die Möglichkeit einer Thrombose beim Patienten hinweist;

wobei der in g'2) erhaltene Wert $(R/Ddi_E)_{Patient}$ mit einem Schwellenwert verglichen wird:

h'2) durch Bestimmen des Wertes des Verhältnisses $(RDdi_{E/I})_{Referenz}$ für den Wert $Ddi_E$ der Probe auf einer Referenzkurve der folgenden Gleichung:

$$y=a\,x^{-b},$$

wobei gilt:

x ist der D-Dimer-Wert,
y ist das Verhältnis zwischen dem Wert der D-Dimere, die sich aus dem intravaskulären Fibrinabbau ergeben, und dem Wert der Di-Dimere $(R/Ddi_{E/I})$, die entsprechend der Entzündung angepasst werden, a und b sind die Konstanten der Gleichung, die das Verhältnis $R/Ddi_{E/I}$ und den D-Dimer-Wert in Beziehung setzt,
wobei die Referenzkurve wie folgt erstellt wurde:

- wenn die Patientenprobe in die Gruppe 1 eingestuft wurde: unter Verwendung:
von Blutproben, die in Schritt e') in die Gruppe 1 eingestuft wurden und deren D-Dimer-Wert bekannt ist oder bestimmt wurde, deren $Ddi_{E/I}$-Wert mittels der Schritte a)-e'1) ermittelt wurde und deren R-Wert mittels der Schritte a)-e'2) ermittelt wurde; und
- wenn die Patientenprobe in die Gruppe II eingestuft wurde: unter Verwendung:
von Blutproben, die in Schritt e') in die Gruppe II eingestuft wurden und deren D-Dimer-Wert bekannt ist oder bestimmt wurde, deren $Ddi_{E/I}$-Wert mittels der Schritte a)-e'1) ermittelt wurde und deren R-Wert durch die Schritte a)-e'2) erhalten wurde; und

h"2) Vergleichen des in g'2) erhaltenen Wertes der durch Hyperfibrinolyse erzeugten D-Dimere $(R/Ddi_E)_{Patient}$ mit dem Wert des in h'2) erhaltenen Verhältnisses $(R/Ddi_E)_{Referenz}$ wobei:
ein $(R/Ddi_E)_{Patient}$ kleiner als das Verhältnis $(R/Ddi_E)_{Referenz}$ eine Thrombose beim Patienten ausschließt, und ein $(R/Ddi_E)_{Patient}$ größer oder gleich dem Verhältnis $(R/Ddi_E)_{Referenz}$ auf die Möglichkeit einer Thrombose beim Patienten hinweist.

**13.** Verfahren nach Anspruch 12, **dadurch gekennzeichnet, dass** in Schritt h') das Vergleichen des TA/TTF-Verhältnisses in Bezug auf einen Schwellenwert umfasst:

h'3) Berechnen des TA/TFF-Verhältnisses, wobei TA der in Schritt d) bestimmte Zeitpunkt des Erreichens (TA) des Fibrinpolymerisationsplateaus ist und TFF der in Schritt d) bestimmte Zeitpunkt der Bildung des Fibringerinnsels ist; und
h"3) wenn die Patientenprobe in die Gruppe I eingestuft wurde:

Vergleichen des TA/TFF-Verhältnisses in Bezug auf einen ersten Schwellenwert, insbesondere einen ersten Schwellenwert von 1,75,
wobei ein TA/TFF-Verhältnis über dem ersten Schwellenwert erlaubt, eine Thrombose auszuschließen und eine Thrombophilie oder einen Zustand der Gerinnungsaktivierung bei dem in Gruppe I eingestuften Patienten zu diagnostizieren, und
wenn die Patientenprobe in die Gruppe II eingestuft wurde:
Vergleichen des TA/TFF-Verhältnisses mit einem ersten Schwellenwert, insbesondere mit einem ersten Schwellenwert von 1,65, wobei

ein TA/TFF-Verhältnis über dem ersten Schwellenwert erlaubt, eine Thrombose auszuschließen und eine Thrombophilie zu diagnostizieren oder einen Zustand der Gerinnungsaktivierung bei dem in Gruppe II eingestuften Patienten, und
ein TA/TFF-Verhältnis, das kleiner oder gleich dem Schwellenwert ist, auf eine Thrombose bei dem in Gruppe II eingestuften Patienten hinweist.

**14.** Verfahren nach Anspruch 13, **dadurch gekennzeichnet, dass**:

- wenn in Schritt h"3) eine Thrombose diagnostiziert wurde, der Wert der für die venöse thromboembolische Erkrankung spezifischen D-Dimere $(Ddiv_{TE})$ dem in Schritt e') erhaltenen D-Dimer-Wert R entspricht,
- wenn in den Schritten h"1), h"2) und h"3) eine Thrombose ausgeschlossen wird, aber der in Schritt e') erhaltene Wert R über dem Schwellenwert, bevorzugt über dem Schwellenwert von 0,5 µg/ml, liegt, das Verfahren ferner einen Schritt zur Berechnen des Wertes der für die venöse thromboembolische Erkrankung spezifischen D-Dimere $(Ddi_{VTE})$ umfasst, unter Verwendung der folgenden Gleichung:

$$Ddi_{MTEV} = 0,5 \text{ x } \frac{R}{Ddi_E}$$

wobei $Ddi_E$ der Wert der D-Dimere in der Probe ist.

**15.** Verfahren nach einem der Ansprüche 1 bis 14, **dadurch gekennzeichnet, dass** die Blutprobe ein Volumen zwischen 1 µl und 300 µl, bevorzugt zwischen 50 µl und 200 µl, aufweist.

**16.** Verfahren nach einem der Ansprüche 1 bis 15, **dadurch gekennzeichnet, dass** die Blutprobe unverdünnt ist.

**17.** Verfahren nach einem der Ansprüche 1 bis 16, **dadurch gekennzeichnet, dass** die Bestimmung der D-Dimere der Probe durch ein immunoturbidimetrisches oder Enzymimmunoassay-Verfahren durchgeführt wird.

**18.** Verfahren nach einem der Ansprüche 1 bis 17, **dadurch gekennzeichnet, dass** Schritt a) durch Mischen der Blutprobe des Patienten mit Gewebefaktor und gegebenenfalls Phospholipiden, bevorzugt durch Mischen der Blutprobe des Patienten mit Gewebefaktor und Phospholipiden, durchgeführt wird.

**19.** Verfahren nach Anspruch 18, **dadurch gekennzeichnet, dass** der Gewebefaktor aus Schritt a) in einer Konzentration zwischen 0,5 und 5 pM, bevorzugt 2pM, vorliegt.

**20.** Verfahren nach Anspruch 18 oder 19, **dadurch gekennzeichnet, dass** die Mischung aus Schritt a) Calciumionen enthält.

**21.** Verfahren nach einem der Ansprüche 1 bis 20, **dadurch gekennzeichnet, dass** Schritt b) das Inkubieren der in Schritt a) erhaltenen Mischung für eine Zeit zwischen 20 Sekunden und 400 Sekunden, bevorzugt zwischen 60 Sekunden und 300 Sekunden, bei einer Temperatur zwischen 30°C und 40°C umfasst.

**22.** Verfahren nach einem der Ansprüche 1 bis 21, **dadurch gekennzeichnet, dass** in Schritt b) die Erzeugung von Thrombin und die Bildung eines Fibrin-Gerinnsels durch Zugabe von Calcium-Ionen zu der inkubierten Probe initiiert werden.

**23.** Verfahren nach einem der Ansprüche 1 bis 22, **dadurch gekennzeichnet, dass** die Blutprobe des Patienten eine Plasmaprobe ist.

**24.** Verfahren nach Anspruch 23, **dadurch gekennzeichnet, dass** die Plasmaprobe eine Probe von plättchenarmem Plasma ist.

**25.** Verfahren nach einem der Ansprüche 23 bis 24, **dadurch gekennzeichnet, dass** in Schritt c) die Messung der zeitlichen Variation wenigstens einer Eigenschaft der in b) erhaltenen Probe durch Messung der zeitlichen Variation der optischen Dichte (ODD) bei einer Wellenlänge zwischen 350 und 800 nm, bevorzugt bei der Wellenlänge von 540 nm, durchgeführt wird.

**26.** Verfahren nach Anspruch 25, **dadurch gekennzeichnet, dass** die Messung der optischen Dichte in Schritt c) bei der gleichen Wellenlänge wie die für die D-Dimer-Bestimmung verwendeten 540 nm durchgeführt wird.

**27.** Verfahren nach einem der Ansprüche 1 bis 22, **dadurch gekennzeichnet, dass** die Blutprobe des Patienten eine Vollblutprobe ist.

**28.** Verfahren nach Anspruch 27, **dadurch gekennzeichnet, dass** die Vollblutprobe eine citrierte Vollblutprobe ist.

**29.** Verfahren nach Anspruch 27 oder Anspruch 28, **dadurch gekennzeichnet, dass** die Messung der zeitlichen Variation mindestens einer Eigenschaft der in b) erhaltenen Probe durch Thromboelastographie, Rheometrie oder Bildanalyse durchgeführt wird.

**30.** Verfahren nach einem der Ansprüche 1 bis 29, **dadurch gekennzeichnet, dass** zumindest die Schritte c) und d) auf einem Diagnoseautomaten oder auf einem biologischen Fernanalysegerät, bevorzugt auf einem Gerinnungsanalysegerät, durchgeführt werden.

**31.** In vitro-Verfahren zur Diagnose einer venösen thromboembolischen Erkrankung (VTE) bei einem Patienten, umfassend die folgenden Schritte:

   - Durchführen einer Bestimmung von VTE-spezifischen D-Dimeren in einer Blutprobe des Patienten unter Verwendung eines Verfahrens nach einem der Ansprüche 3, 5, 9, 12, 13, und 14; und
   - Erstellen einer Diagnose für den Patienten.

**32.** In-vitro-Diagnoseverfahren nach Anspruch 31, **dadurch gekennzeichnet, dass** die Diagnose für den Patienten (i) der Ausschluss einer Thrombose, (ii) der akute Gerinnungsaktivierungszustand oder (iii) Thrombose ist.

**33.** In-vitro-Diagnoseverfahren nach Anspruch 31, **dadurch gekennzeichnet, dass**, wenn der Patient ein älterer Mensch, ein Krebspatient, ein Patient mit einer Infektion oder ein Patient mit Thrombophilie ist, die Bestimmung von VTE-spezifischen D-Dimeren unter Verwendung eines Verfahrens nach einem der Ansprüche 12, 13 und 14 durchgeführt wird.

**34.** In vitro-Diagnoseverfahren nach Anspruch 32, **dadurch gekennzeichnet, dass** die Diagnose für den Patienten (i) der Ausschluss einer Thrombose, (ii) der akute Gerinnungsaktivierungszustand, (iii) Thrombophilie oder (iv) Thrombose ist.

**35.** In-vitro-Diagnoseverfahren nach Anspruch 34, **dadurch gekennzeichnet, dass** die Diagnose für den Patienten eine Thrombose geringen Schweregrades oder eine Thrombose im Falle eines Lungeninfarkts ist.

**36.** Verfahren nach einem der Ansprüche 6, 14 und 15-30, **dadurch gekennzeichnet, dass** ein erhöhter Wert der für die venöse thromboembolische Erkrankung spezifischen D-Dimere, $Ddi_{VTE}$, repräsentativ für das Ausmaß der Lungenembolie oder der tiefen Venenthrombose ist.

**Claims**

**1.** Method for assaying D-dimers that are specific to venous thromboembolism in a patient blood sample, said method comprising on the one hand the assay of D-dimers of the sample in order to obtain the level of D-dimers in the sample ($Ddi_E$), and on the other hand, the dynamic measurement of the fibrin formation in this same sample, said dynamic measurement comprising the following steps:

a) initiating the activation of the coagulation in the sample without triggering it;
b) incubating the mixture obtained in step a), and triggering, in the incubated sample, the generation of thrombin and the formation of a fibrin clot;
c) measuring the temporal variation of at least one property of the sample obtained in b), wherein the fibrin clot is formed;
d) establishing the formation profile of the fibrin clot analysed in c), and extracting from this profile the fibrin formation time (TFF) measured at the inflection point of the tangent of the curve of the profile, the value of the property ($Vp_{(TA)}$) of the sample measured at the reaching time (TA) of the polymerisation plateau of the fibrin;
e) calculating the level of D-dimers resulting from the degradation of the intravascular fibrin (R):

e1) by adjusting the level of D-dimers of the sample ($Ddi_E$) according to the level of the D-dimers generated by hypercoagulation by using TFF determined in d), in order to obtain the level of adjusted D-dimers according to hypercoagulation ($Ddi_{E/HC}$), and
e2) by adjusting the level of adjusted D-dimers $Ddi_{E/HC}$ obtained in e1) according to the level of the D-dimers generated by inflammation by using $Vp_{(TA)}$ determined in d), in order to obtain R;

f) comparing the level of D-dimers resulting from degradation of the fibrin R obtained in e), with respect to a threshold, preferably to the threshold of 0.5 $\mu$g/mL;
g) determining the level of fibrinogen degradation products generated by hyperfibrinolysis ($PDFg_{(HF)}$) present in the coagulation activation by using the level of D-dimers resulting from degradation of the fibrin, R, obtained in e) and the level of D-dimers of the sample ($Ddi_E$).
h) comparing the level $PDFg_{(HF)}$ obtained in g) with respect to a threshold, and comparing the fibrin formation time (TFF) obtained in d) with respect to a threshold.

**2.** Method according to claim 1, **characterised in that** in step e), calculating the level R expressed in initial fibrinogen equivalent units (FEU) is carried out:

e1) by calculating the level of adjusted D-dimers according to hypercoagulation ($Ddi_{E/HC}$) by using the following equation:

$$Ddi_{E/HC} = Ddi_E \ \text{x} \ \frac{TFF}{\text{Control Time}}$$

wherein the Control Time is the average formation time of the fibrin clot of samples of healthy patients who do not have suspected thrombosis, measured according to steps a)-d), and
e2) by calculating the level R by using the following equation:

$$R = \frac{Ddi_{E/HC}}{[Fib]_{(Vp(TA))}}$$

wherein $[Fib]_{(Vp(TA))}$ is the concentration in fibrinogen deduced for the value of the property $Vp_{(TA)}$ on a standard curve of equation:

$$y = a \ln(x) - b,$$

wherein:

y is the value of the property measured at the reaching time (TA) of the polymerisation plateau of the fibrin,
x is the concentration in fibrinogen,
a and b are the constants of the logarithmic equation that connects the level of the fibrin plateau, and the concentration in fibrinogen,
the standard curve having been established for blood samples of which the concentration in fibrinogen has been determined and of which the value of the property $(Vp_{(TA)})$ has been determined by steps a)-d).

3. Method according to claim 1 or claim 2, **characterised in that** in step f), a level R obtained in e), less than the threshold, preferably less than the threshold of 0.5 $\mu$g/ml, makes it possible to exclude a thrombosis in the patient and a level R obtained in e), greater than the threshold, preferably greater than the threshold of 0.5 $\mu$g/ml, is indicative of the possibility of a thrombosis in the patient.

4. Method according to any of claims 1 to 3, **characterised in that** step g) comprises steps of:

g1) determining the level of PDFg corresponding to the level of D-dimers in the sample $(Ddi_E)$ on a standard curve established by using blood samples with known levels of PDFg and of D-dimers,
g2) determining the level of PDFg corresponding to the level of adjusted D-dimers (R) obtained at step e) on the standard curve used in g1), and
g3) determining the level of PDFg generated by the hyperfibrinolysis $(PDFg_{(HP)})$ by subtracting the level of PDFg obtained in g2) from the level of PDFg obtained in g1).

5. Method according to claim 4, **characterised in that** step h) comprises steps of:

h1) comparing $PDFg_{(HF)}$ to a threshold, in particular a threshold of 1 $\mu$g/mL, where $PDFg_{(HF)}$ less than the threshold or negative makes it possible to exclude thrombosis in the patient, and $PDFg_{(HF)}$ greater than the threshold is indicative of a possibility of thrombosis in the patient,
h2) comparing TFF to a threshold, in particular a threshold equal to [Control Time of e1) - 1 standard deviation], even more particularly a threshold of 120 seconds for a Control Time of 135 seconds, wherein TFF less than the threshold is indicative of a patient without thrombosis but having an acute coagulation activation, and a TFF greater than the threshold is indicative of a thrombosis in the patient.

6. Method according to claim 5, **characterised in that**:

- when a thrombosis has been diagnosed at step h2), the level of D-dimers that are specific to venous thromboembolism $(Ddi_{MTEV})$ is the level of D-dimers, R, obtained at step e),
- when a thrombosis has been excluded in steps h1) and h2) but the level R obtained at step e) is greater than a threshold, preferably greater than the threshold of 0.5 $\mu$g/mL, the method further comprises a step of calculating the level of D-dimers that are specific to venous thromboembolism $(Ddi_{MTEV})$ by using the following equation:

$$Ddi_{MTEV} = 0.5 \text{ x } \frac{R}{Ddi_E}$$

wherein DdiE is the level of D-dimers in the sample.

7. Method for assaying D-dimers that are specific to venous thromboembolism in a patient blood sample, said method comprising on the one hand the assay of D-dimers of the sample in order to obtain the level of D-dimers in the sample $(Ddi_E)$ and on the other hand, the dynamic measurement of the fibrin formation in this same sample, said dynamic measurement comprising the following steps:

a) initiating the activation of the coagulation in the sample without triggering it;

b) incubating the mixture obtained in step a), and triggering, in the incubated sample, the generating of thrombin and the formation of a fibrin clot;

c) measuring the temporal variation of at least one property of the sample obtained in b), where the fibrin clot is formed;

d) establishing the formation profile of the fibrin clot analysed in c), and extracting from this profile the fibrin formation time (TFF) measured at the inflection point of the tangent of the curve of the profile, and the value of the property ($Vp_{(TA)}$) of the sample measured at the reaching time (TA) of the polymerisation plateau of the fibrin;

e') calculating the level of D-dimers resulting from degradation of the intravascular fibrin (R):

e'1) by adjusting the level of D-dimers of the sample, $Ddi_E$, according to the level of the D-dimers generated by inflammation by using $Vp_{(TA)}$ determined in d), in order to obtain the level of adjusted D-dimers for the inflammation ($Ddi_{E/I}$); and

e'2) by correcting the level of adjusted D-dimers for the inflammation ($Ddi_{E/I}$) obtained in e'1) for the low levels of D-dimers, and

classifying the sample of the patient according to the inflammation;

f) comparing the level of D-dimers resulting from the degradation of the fibrin R obtained in e), with respect to a threshold, preferably to the threshold of 0.5 $\mu$g/mL;

g') determining the level of D-dimers generated by hyperfibrinolysis ($Ddi_{(HF)}$) by using the level of D-dimers in the sample ($Ddi_E$) and the level R obtained in e') or by using the level of adjusted D-dimers according to the inflammation, $Ddi_{E/I}$, obtained in e'1) and the level R obtained in e');

h') comparing the level $Ddi_{(HF)}$ obtained in g') with respect to a threshold, and comparing the ratio TA/TTF with respect to a threshold, where TA is the reaching time of the fibrin polymerisation plateau obtained in d), and TFF is the fibrin formation time obtained in d).

8. Method according to claim 7, **characterised in that** in step e'), the level R expressed in initial fibrinogen equivalent units (FEU) is obtained:

e'1) by calculating the level of adjusted D-dimers according to the inflammation ($Ddi_{E/I}$) by the following equation:

$$Ddi_{E/I} = \frac{Ddi_E}{[Fib]_{(Vp(TA))}}$$

where $[Fib]_{(Vp(TA))}$ is the concentration in fibrinogen deduced for the value of the property $Vp_{(TA)}$ on a standard curve of equation:

$$y = a \ln(x) - b,$$

wherein:

y is the value of the property measured at the reaching time (TA) of the polymerisation plateau of the fibrin, x is the concentration in fibrinogen,

a and b are the constants of the logarithmic equation that connects the level of the fibrin plateau, and the concentration in fibrinogen,

the standard curve having been established for blood samples of which the concentration in fibrinogen has been determined and of which the value of the property $Vp_{(TA)}$ has been determined by steps a)-d).

e'2) by correcting the level $Ddi_{E/I}$ obtained in e'1) by the following equation:

$$R = Ddi_{E/I} + [0.5 - F_{Ddi-E}]$$

wherein $F_{Ddi-E}$ is a correction factor of the low levels of D-dimers (< 4 $\mu$g/mL) of which the value corresponds to the value of the correction factor for the level of D-dimers of the sample ($Ddi_E$) on the standard curve of equation:

$$y = a\,x^2 + b\,x + c$$

wherein y is the correction factor, F,
x is the level of D-dimers,
a, b and c are the constants of the polynomial equation that connects the correction factor, and the level of D-dimers,
the standard curve having been established for blood samples of which the level of D-dimers has been determined and for which the correction factor has been determined empirically so that the level $Ddi_{E/I}$ is brought to the threshold of 0.5 $\mu$g/mL FEU units (fibrinogen equivalent units).

9. Method according to claim 7 or claim 8, **characterised in that** in step e'), classifying the sample of the patient according to the inflammation comprises:

- calculating the ratio $1/[Fib]_{(Vp(TA)}$, wherein $[Fib]_{(Vp(TA)}$ is the concentration in fibrinogen determined in e1'); and
- classifying the sample of the patient in group I of patients without inflammation if the ratio $1/[Fib]_{(Vp(TA)} > 0.20$, or
- classifying the sample of the patient in group II of patients with inflammation if the ratio $1/[Fib]_{(Vp(TA)} \leq 0.20$.

10. Method according to any of claims 7 to 9, **characterised in that** in step f), a level R obtained in e'), less than the threshold, preferably less than the threshold of 0.5 $\mu$g/ml makes it possible to exclude a thrombosis in the patient, and a level R obtained in e'), greater than the threshold, preferably greater than the threshold of 0.5 $\mu$g/mL, is indicative of the possibility of a thrombosis in the patient.

11. Method according to claim 9 or claim 10, **characterised in that** in step g'), the level of D-dimers generated by hyperfibrinolysis ($Ddi_{HF}$) is calculated:

g'1) as the ratio between the level R determined in step e') and the level of D-dimers of the sample, $Ddi_E$, by using the equation:

$$Ddi_{HF} = (R/Ddi_E)_{patient} = \frac{R}{Ddi_E}$$

g'2) as the ratio between the level R determined in step e') and the level $Ddi_{E/I}$, obtained in e'1), by using the equation:

$$Ddi_{HF} = (R/Ddi_{E/I})_{patient} = \frac{R}{Ddi_{E/I}}$$

12. Method according to claim 11, **characterised in that** in step h'):

the level $(R/Ddi_E)_{Patient}$ obtained in g'1) is compared with a threshold

h'1) by determining for the level $Ddi_E$ of the sample, the value of the ratio $(R/Ddi_E)_{standard}$ on a standard curve of equation:

$$y = a\,x^{-b}$$

wherein x is the level of D-dimers,
y is the ratio between the level of D-dimers that result from the degradation of the intravascular fibrin and the level of D-dimers, $(R/Ddi_E)$, a, and b are the constants of the equation that connects the ratio $R/Ddi_E$, and the level of D-dimers,
the standard curve having been established:

- by using, if the sample of the patient has been classified in group I: blood samples classified in group I by step e') and of which the level of D-dimers is known or has been determined and the level R has been obtained by steps a)-e'2); and
- by using, if the sample of the patient has been classified in group II: blood samples classified in group II by step e') and of which the level of D-dimers is known or has been determined and the level R has been obtained by steps a)-e'2); and

h"1) by comparing the value of the level of D-dimers generated by hyperfibrinolysis $(R/Ddi_E)_{Patient}$ obtained in g'1) with the value of the ratio $(R/Ddi_E)_{standard}$ obtained in h'1), wherein:
$(R/Ddi_E)_{Patient}$ less than the ratio $(R/Ddi_E)_{standard}$ makes it possible to exclude thrombosis in the patient, and $(R/Ddi_E)_{Patient}$ greater than or equal to the ratio $(R/Ddi_E)_{standard}$ is indicative of a possibility of thrombosis in the patient;
the level $(R/Ddi_{E/I})_{patient}$ obtained in g'2) is compared with a threshold
h'2) by determining for the level $Ddi_E$ of the sample, the value of the ratio $(R/Ddi_{E/I})_{standard}$ on a standard curve of equation:

$$y = a\ x^{-b}$$

where x is the level of D-dimers,
y is the ratio between the level of D-dimers that result from the degradation of the intravascular fibrin and the level of adjusted D-dimers according to the inflammation, $(R/Ddi_{E/I})$,
a, and b are the constants of the equation that connects the ratio $R/Ddi_{E/I}$, and the level of D-dimers, the standard curve having been established:

- by using, if the sample if the patient has been classified in group I: blood samples classified in group I by step e') and of which the level of D-dimers is known or has been determined, the level $Ddi_{E/I}$ has been obtained by steps a)-e'1), and the level R has been obtained by steps a)-e'2); and
- by using, if the sample if the patient has been classified in group II: blood samples classified in group II by step e') and of which the level of D-dimers is known or has been determined, the level $Ddi_{E/I}$ has been obtained by steps a)-e'1), and the level R has been obtained by steps a)-e'2); and

h"2) by comparing the value of the level of D-dimers generated by hyperfibrinolysis $(R/Ddi_{E/I})_{patient}$ obtained in g'2) with the value of the ratio $(R/Ddi_{E/I})_{standard}$ obtained in h'2), where:
$(R/Ddi_{E/I})_{patient}$ less than the ratio $(R/Ddi_{E/I})_{standard}$ makes it possible to exclude thrombosis in the patient, and where $(R/Ddi_{E/I})_{patient}$ greater than or equal to the ratio $(R/Ddi_{E/I})_{standard}$ is indicative of a possibility of thrombosis in the patient.

13. Method according to claim 12, **characterised in that** in step h'), comparing the ratio TA/TTF with respect to a threshold comprises:

h'3) calculating the ratio TA/TFF where TA is the reaching time of the polymerisation plateau of the fibrin determined at step d) and TFF is the formation time of the fibrin clot determined at step d); and
h"3) if the sample of the patient was classified in group I:
comparing the ratio TA/TFF with respect to a first threshold, in particular to a first threshold of 1.75, where:

a ratio TA/TFF greater than the first threshold makes it possible to exclude thrombosis and to diagnose a thrombophilia or a coagulation activation in the patient classified in group I, and
a ratio TA/TFF less than or equal to the threshold is indicative of a thrombosis in the patient classified in group I;

if the sample of the patient was classified in group II:
comparing the ratio TA/TFF with respect to a first threshold, in particular to a first threshold of 1.65, where

a ratio TA/TFF greater than the first threshold makes it possible to exclude thrombosis and to diagnose a thrombophilia or a coagulation activation in the patient classified in group II, and
a ratio TA/TFF less than or equal to the threshold is indicative of a thrombosis in the patient classified in group II.

14. Method according to claim 13, **characterised in that**:

- when a thrombosis has been diagnosed in step h"3), the level of D-dimers that are specific to venous thromboembolism (Ddi$_{MTEV}$) is the level of D-dimers, R, obtained at step e'),
- when a thrombosis is excluded in steps h"1), h"2) and h"3) but the level R obtained at step e') is greater than the threshold, preferably greater than the threshold of 0.5 µg/mL, the method further comprises a step of calculating the level of D-dimers that are specific to venous thromboembolism (Ddi$_{MTEV}$) by using the following equation:

$$Ddi_{MTEV} = 0.5 \text{ x } \frac{R}{Ddi_E}$$

wherein DdiE is the level of D-dimers in the sample.

15. Method according to any one of claims 1 to 14, **characterised in that** the blood sample has a volume comprised between 1 µL and 300 µL, preferably comprised between 50 µL and 200 µL.

16. Method according to any one of claims 1 to 15, **characterised in that** the blood sample is not diluted.

17. Method according to any one of claims 1 to 16, **characterised in that** the assay of D-dimers of the sample is carried out according to an immuno-turbidimetric or immuno-enzymatic method.

18. Method according to any one of claims 1 to 17, **characterised in that** step a) is carried out by mixing the blood sample of the patient with tissue factor and optionally with phospholipids, preferably by mixing the blood sample of the patient with tissue factor and with phospholipids.

19. Method according to claim 18, **characterised in that** the tissue factor of step a) is present at a concentration comprised between 0.5 and 5 pM, preferably 2 pM.

20. Method according to claim 18 or claim 19, **characterised in that** the mixture of step a) comprises calcium ions.

21. Method according to any one of claims 1 to 20, **characterised in that** step b) comprises incubating the mixture obtained at step a) for a time comprised between 20 seconds and 400 seconds, preferably comprised between 60 seconds and 300 seconds, at a temperature between 30°C and 40°C.

22. Method according to any one of claims 1 to 21, **characterised in that**, in step b), triggering the generation of thrombin and the formation of a fibrin clot is carried out by adding calcium ions to the incubated sample.

23. Method according to any one of claims 1 to 22, **characterised in that** the patient blood sample is a sample of plasma.

24. Method according to claim 23, **characterised in that** the sample of plasma is a sample of platelet poor plasma.

25. Method according to any one of claims 23 to 24, **characterised in that**, in step c), measuring the temporal variation of at least one property of the sample obtained in b) is carried out by measuring the temporal variation in the optical density (DDO) at a wavelength comprised between 350 and 800 nm, preferably at a wavelength of 540 nm.

26. Method according to claim 25, **characterised in that** the measurement of the optical density of step c) is carried out at the same wavelength as that used for the assay of D-dimers, 540 nm.

27. Method according to any of claims 1 to 22, **characterised in that** the patient blood sample is a sample of whole blood.

28. Methods according to claim 27, **characterised in that** the sample of whole blood is a sample of citrated whole blood.

29. Method according to claim 27 or claim 28, **characterised in that** measuring the temporal variation of at least one property of the sample obtained in b) is carried out by thromboelastography, rheometry or by image analysis.

30. Method according to any one of claims 1 to 29, **characterised in that** at least steps c) and d) are carried out on a diagnosis automaton or on a delocalised biology analyser, preferably on a coagulation analyser.

31. *In vitro* method for diagnosing venous thromboembolism (MTEV) in a patient, comprising steps of:

- carrying out an assay of D-dimers that are specific to MTEV in a blood sample of the patient by using a method according to any of claims 3, 5, 9, 12, 13, and 14; and
- providing a diagnosis concerning the patient.

32. *In vitro* method *in vitro* for diagnosing according to claim 31, **characterised in that** the diagnostic concerning the patient is (i) exclusion of thrombosis, (ii) acute coagulation activation, or (iii) thrombosis.

33. *In vitro* method for diagnosing according to claim 31, **characterised in that** if the patient is an elderly person, a patient afflicted with cancer, a patient afflicted with an infection or a patient afflicted with thrombophilia, the assay of D-dimers that are specific to MTEV is carried out by using a method according to any of claims 12, 13 and 14.

34. *In vitro* method for diagnosing according to claim 32, **characterised in that** the diagnostic concerning the patient is (i) exclusion of thrombosis, (ii) acute coagulation activation, (iii) thrombophilia, or (iv) thrombosis.

35. *In vitro* method for diagnosing according to claim 34, **characterised in that** the diagnostic concerning the patient is a low-severity thrombosis or thrombosis in case of pulmonary infarction.

36. Method according to any of claims 6, 14 and 15-30, **characterised in that** a high level of D-dimers that are specific to venous thromboembolism, $Ddi_{MTEV}$, is representative of the extent of the pulmonary embolism or deep vein thrombosis.

**Figure 1**

**Figure 2**

| DOSAGES | | |
|---|---|---|

| Echantillon | Plasma | |
|---|---|---|
| Réactifs | Latex Ddimères | |
| Mesure | Variation de DO à 540 nm | |

**1) Dosage des Ddimères**
- DDO
- seuil 0.5 µg/ml

| Echantillon | Plasma | Sang total |
|---|---|---|
| Réactifs | FT Phospholipides Calcium | |
| Mesure | Variation de DO à 540 nm pendant 10 min | Variation des propriétés physiques par analyse rhéologique ou d'images pendant 5 min |

**2) Mesure de la formation de fibrine**

1) Initiation de la génération de thrombine
2) Formation du caillot
3) Profil de formation du caillot :
- Temps de formation du caillot (tangente)
- DDO ou amplitude maximale du plateau

**Ajustement des Ddimères en fonction de :**
- l'hypercoagulation
- l'inflammation
Expression par unité de fibrinogène
- µg/ml
- seuil 0.5 µg/ml

**Figure 3**

**INTERPRETATION DE LA METHODE**

Ddimères ajustés pour l'hyper coagulation et l'inflammation 'Ddi ≥ 0.50 µg /ml

Calcul des PDFg [Ddi] - PDFg [Ddi ajustés] générés par l'hyperfibrinolyse

Présence et différence ≥ 1 µg/ml = thrombose

Présence et différence < 1 µg/ml = absence de thrombose

+ Temps de formation de la Fibrine raccourci <T Témoin-16

**Probabilité d'EP et/ou de TVP**

Différenciation de la thrombose des états d'activation de la coagulation (cancer, grossesse, ..)

Confirmation ou infirmation d'une EP et/ou d'une TVP ou orientation vers un état d'hyper-coagulation

**Figure 4**

**A**

**B**

**Figure 5**

**A**

**B**

**Figure 6**

**A**

T atteinte du plateau = f (T formation de fibrine)
n=219 sans cancer

$y = 1.1193x + 67.653$
$R^2 = 0.838$

**B**

T atteinte du plateau = f (T formation de fibrine)
n=34 cancers

$y = 1.1188x + 63.168$
$R^2 = 0.9451$

**Figure 7**

**A**

Ddi/T formation de fibrine = f (Ddi)
n=87 patients Ddi+ sans cancer

y = 0.0572x+0.0196
R² = 0.9701

**B**

Ddi/T formation de fibrine = f (Ddi)
n=28 cancers Ddi+

y = 0.0526x+0.0287
R² = 0.9216

**Figure 8**

**A**

**B**

**C**

Figure 9

**A**

**B**

**C**

**Figure 10**

**A**

**B**

**Figure 11**

**A**

Ddi et Ddi ajustés FAUX POSITIFS
**Patients sans thrombose (n=215)**

**B**

Ddi et Ddi ajustés POSITIFS
**Patients avec thrombose (n=21)**

**Figure 12**

**A**

Ddi et Ddi ajustés FAUX POSITIFS
Patients sans thrombose (n=199)

**B**

Ddi et Ddi ajustés POSITIFS
Patients avec thrombose (n=15)

**Figure 13**

**A**

**B**

**Figure 14**

**A**

**B**

Figure 15

## RÉFÉRENCES CITÉES DANS LA DESCRIPTION

**Documents brevets cités dans la description**

- US 201227 A
- WO 2011022054 A
- US 2004029286 A

**Littérature non-brevet citée dans la description**

- **LOPEZ.** *Thromb. Res.,* 2009, vol. 123 (4), 30-34
- **WELLS.** *J. Thromb. Haemost.,* 2007, vol. 5 (1), 41-50
- **CERIANI.** *J. Thromb. Haemost.,* 2010, vol. 8 (5), 957-970
- **KLOK.** *Arch. Intern. Med.,* 2008, vol. 168 (19), 2131-2136
- **LE GAL.** *Ann. Intern. Med.,* 2006, vol. 144 (3), 165-171
- **ADAM.** *Blood,* 2009, vol. 113 (13), 2878-2887
- **MIRSHAHI.** *PLoS ONE,* 2014, vol. 9 (3), e92379
- **DIRIX.** *Br. J. Cancer,* 2002, vol. 86 (3), 389-395
- **DEMPFLE.** FACT study group. *Thromb. Haemost.,* 2001, vol. 85 (4), 671-678
- **DE GROOT.** *Thromb. Haemost.,* 1999, vol. 82 (6), 1588-1592
- **RIGHINI et al.** *JAMA,* 2014, vol. 311, 1117-1124
- **KOLOK et al.** *Arch. Intern. Med.,* 2008, vol. 168, 21-31
- **WELLS et al.** *NEJM,* 2003, vol. 349, 1227-1235